(19) Europäisches Patentamt
European Patent Office
Office européen des brevets

(11) **EP 2 890 984 B1**

(12) **EUROPEAN PATENT SPECIFICATION**

(45) Date of publication and mention
of the grant of the patent:
**03.10.2018 Bulletin 2018/40**

(51) Int Cl.:
*B01J 19/00* (2006.01)    *G01N 33/543* (2006.01)
*G01N 33/68* (2006.01)    *B82Y 15/00* (2011.01)
*B82Y 5/00* (2011.01)    *B82Y 30/00* (2011.01)

(21) Application number: **13833992.4**

(22) Date of filing: **29.08.2013**

(86) International application number:
**PCT/US2013/057373**

(87) International publication number:
**WO 2014/036312 (06.03.2014 Gazette 2014/10)**

(54) **IMMUNOSIGNATURING: A PATH TO EARLY DIAGNOSIS AND HEALTH MONITORING**

IMMUNSIGNIERUNG: WEG ZUR FRÜHDIAGNOSE UND GESUNDHEITSÜBERWACHUNG

IMMUNO-SIGNATURE : UNE VOIE VERS LE DIAGNOSTIC PRÉCOCE ET LA SURVEILLANCE DE LA SANTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2012 US 201261694598 P**

(43) Date of publication of application:
**08.07.2015 Bulletin 2015/28**

(73) Proprietor: **Arizona Board of Regents, a Body Corporate of the**
**State of Arizona, acting for and on behalf of Arizona State University**
**Scottsdale, AZ 85257-9908 (US)**

(72) Inventors:
• **JOHNSTON, Stephen, Albert**
**Tempe, AZ 85284 (US)**
• **STAFFORD, Phillip**
**Phoenix, AZ 85044 (US)**
• **WOODBURY, Neal**
**Tempe, AZ 85282 (US)**

(74) Representative: **Avidity IP**
**Broers Building**
**Hauser Forum**
**21 JJ Thomson Avenue**
**Cambridge CB3 0FA (GB)**

(56) References cited:
**WO-A1-2011/109440    US-A1- 2007 020 678**
**US-A1- 2012 190 574    US-A1- 2012 190 574**

**US-B1- 6 346 423**

• **B. A. CHASE ET AL: "Evaluation of Biological Sample Preparation for Immunosignature-Based Diagnostics", CLINICAL AND VACCINE IMMUNOLOGY, vol. 19, no. 3, 11 January 2012 (2012-01-11), pages 352-358, XP55261902, US ISSN: 1556-6811, DOI: 10.1128/CVI.05667-11**
• **LEGUTKI J B ET AL: "A general method for characterization of humoral immunity induced by a vaccine or infection", VACCINE, ELSEVIER LTD, GB, vol. 28, no. 28, 17 June 2010 (2010-06-17) , pages 4529-4537, XP027078073, ISSN: 0264-410X, DOI: 10.1016/J.VACCINE.2010.04.061 [retrieved on 2010-06-04]**
• **P. STAFFORD ET AL: "Physical Characterization of the "Immunosignaturing Effect"", MOLECULAR & CELLULAR PROTEOMICS, vol. 11, no. 4, 18 January 2012 (2012-01-18), XP55261864, ISSN: 1535-9476, DOI: 10.1074/mcp.M111.011593**
• **ALEXA K. HUGHES ET AL: "Immunosignaturing Can Detect Products from Molecular Markers in Brain Cancer", PLOS ONE, vol. 7, no. 7, 16 July 2012 (2012-07-16), page e40201, XP55261866, DOI: 10.1371/journal.pone.0040201**

EP 2 890 984 B1

- KAITLIN KROENING ET AL: "Autoreactive antibodies raised by self derived de novo peptides can identify unrelated antigens on protein microarrays. Are autoantibodies really autoantibodies?", EXPERIMENTAL AND MOLECULAR PATHOLOGY, vol. 92, no. 3, 8 March 2012 (2012-03-08), pages 304-311, XP028423893, ISSN: 0014-4800, DOI: 10.1016/J.YEXMP.2012.03.002 [retrieved on 2012-03-08]
- LUCAS RESTREPO ET AL: "Application of immunosignatures to the assessment of Alzheimer's disease", ANNALS OF NEUROLOGY., vol. 70, no. 2, 5 August 2011 (2011-08-05) , pages 286-295, XP55261912, BOSTON, US ISSN: 0364-5134, DOI: 10.1002/ana.22405
- P. STAFFORD ET AL: "Immunosignature system for diagnosis of cancer", PROCEEDINGS OF THE NATIONAL ACADEMY OF SCIENCES, vol. 111, no. 30, 14 July 2014 (2014-07-14), pages E3072-E3080, XP55261857, US ISSN: 0027-8424, DOI: 10.1073/pnas.1409432111
- JOSEPH BARTEN LEGUTKI ET AL: "Scalable high-density peptide arrays for comprehensive health monitoring", NATURE COMMUNICATIONS, vol. 5, 3 September 2014 (2014-09-03), page 4785, XP55263550, DOI: 10.1038/ncomms5785
- NAVALKAR KRUPA ARUN ET AL: "Peptide based diagnostics: Are random-sequence peptides more useful than tiling proteome sequences?", JOURNAL OF IMMUNOLOGICAL METHODS, vol. 417, 11 December 2014 (2014-12-11), pages 10-21, XP029199029, ISSN: 0022-1759, DOI: 10.1016/J.JIM.2014.12.002

**Description**

**BACKGROUND**

Monitoring one's health is a great challenge. Early detection of a condition can have a significant impact in the outcome of a disease, and yet, for most conditions, no single test exists that can detect disease before the appearance of major symptoms. Numerous groups have attempted to develop assays that can diagnose specific conditions; however, such assays are limited to a specific disease or diagnosis. Moreover, monitoring health over a period of time is cost and time-prohibitive for currently available diagnostic assays.

B.A. Chase et al, clinical and Vaccine Immunology, 19, 352-358 (2012) discloses assays performed on dried blood from a patient. US 2012/190574 discloses methods of analyzing a sample using an array of different compounds.

**SUMMARY OF THE INVENTION**

The present invention provides a method, performed on a dried blood sample obtained from a patient said method comprising:

a) reconstituting the dried blood sample with a solution; contacting the reconstituted sample to a peptide array, wherein the array comprises a plurality of in-situ synthesized peptides immobilized to pre-determined different locations on a solid support, wherein the in-situ synthesis of the peptides comprises the steps of:
b) contacting the reconstituted sample to a peptide array, wherein the array comprises a plurality of in-situ synthesized peptides immobilized to pre-determined different locations on a solid support, wherein the in-situ synthesis of the peptides comprises the steps of:

a. adding a first monomer to a pre-determined fraction of locations on the solid support;
b. adding a second monomer to a pre-determined fraction of locations on the solid support, wherein the pre-determined fraction of locations for the second monomer includes locations containing the first monomer and locations with no monomer;
c. adding a third monomer to a pre-determined fraction of locations on the solid support, wherein the pre-determined fraction of locations for the second monomer includes locations containing the first and second monomer, locations containing the second monomer and locations containing no monomer; and
d. repeating steps a-c with a defined set of monomers until the monomers form at least one peptide of a desired average length and the sum of the fractions total is at least 100%; wherein the plurality of different peptides are capable of off-target binding of at least one antibody in the reconstituted dried blood sample;

c) measuring the off-target binding of the at least one antibody to the plurality of different peptides in the peptide array to form an immunosignature; and
d) identifying a state of health based on the immunosignature, wherein the immunosignature measures changes in binding of the antibody from the reconstituted dried blood sample to the peptide array as compared to a control,

Disclosed herein are methods, arrays, and kits for monitoring the health of a subject. In cases disclosed herein, the invention provides a rapid, robust and reproducible method of health monitoring, allowing the health of individuals to be monitored over a period of time. In some cases, the method comprising: a) contacting a complex biological sample to a peptide array, wherein the peptide array comprises different peptides capable of off-target binding of at least one antibody in the biological sample; b) measuring the off-target binding of the antibody to a plurality of different peptides in the peptide array to form an immunosignature; and c) associating the immunosignature with a state of health.

Disclosed herein is a method of providing a treatment, the method comprising: a) receiving a complex biological sample from a subject; b) contacting the complex biological sample to a peptide array, wherein the peptide array comprises different peptides capable of off-target binding of at least one antibody in the biological sample; c) measuring the off-target binding of the antibody to a plurality of the different peptides to form an immunosignature; d) associating the immunosignature with a condition; and e) providing the treatment for the condition.

Disclosed herein is a method of preventing a condition, the method comprising: a) providing a complex biological sample from a subject; b) contacting the complex biological sample to a peptide array, wherein the peptide array comprises different peptides capable of off-target binding of at least one antibody in the complex biological sample; c) measuring an off-target binding of the complex biological sample to a plurality of the different peptides to form an immunosignature; d) associating the immunosignature with a condition; and e) receiving a treatment for the condition.

Disclosed herein is a method of diagnosis, the method comprising: a) receiving a complex biological sample from a subject; b) contacting the complex biological sample to a peptide array, wherein the peptide array comprises different peptides capable of off-target binding of at least one antibody in the biological sample; c) measuring the off-target binding of the antibody to a plurality of different peptides in the peptide array to form an immunosignature; and d) diagnosing a condition based on the immunosignature.

Disclosed herein is a system to receive, log, and dilute a biological sample from a subject. In some cases, the system to receive, log and dilute a biological system from a subject is fully automated.

In some cases, an immunosignaturing system comprises an automated device consisting of the following components: 1) an automated system to receive, log, and dilute a biological sample from a subject; 2) a compartment for an automated immunosignaturing assay, the immunosignaturing assay comprising: a) an application of a diluted sample to a peptide array, b) an incubation for a specific time, c) a wash and removal of unbound sample, d) application of a secondary antibody solution for a specific time, e) a removal of the secondary antibody, and f) a drying and scanning of the array to determine a fluorescence of each spot; and 3) detecting the fluorescence with a detector.

Methods and devices are described herein to generate novel arrays which may be used in conjunction with the immunosignature assays described herein. In some cases, the arrays are manufactured to reduce the number of patterning steps necessary to generate heteropolymers on the arrays. In other cases, the methods and devices disclosed herein utilize novel patterning algorithms to add multiple monomers simultaneously. In some cases, the algorithms disclosed herein can significantly reduce the number patterning steps required for synthesizing large arrays, leading to lower costs and shorter manufacturing time.

In some cases, the methods and devices disclosed herein provide for an array comprising a plurality of in-situ synthesized polymers of variable lengths immobilized to different locations on a solid support, wherein the in-situ synthesis of polymers comprises the steps of: adding a first monomer to a pre-determined fraction of locations on the solid support; adding a second monomer to a pre-determined fraction of locations on the solid support, wherein the pre-determined fraction of locations for the second monomer includes locations containing the first monomer and locations with no monomer; adding a third monomer to a pre-determined fraction of locations on the solid support, wherein the pre-determined fraction of locations for the second monomer includes locations containing the first and second monomer, locations containing the second monomer and locations containing no monomer; and repeating steps a-c with a defined set of monomers until the polymers reach a desired average length and the sum of the fractions total at least 100%.

In other cases, the methods and devices disclosed herein also provide a method of fabricating an array comprising a plurality of in-situ synthesized polymers of variable lengths immobilized to different locations on a solid support, comprising the steps of: providing a substrate as a solid support where the polymers to be synthesized; adding a first monomer to a pre-determined fraction of locations on the solid support; adding a second monomer to a pre-determined fraction of locations on the solid support, wherein the pre-determined fraction of locations for the second monomer includes locations containing the first monomer and locations with no monomer; adding a third monomer to a pre-determined fraction of locations on the solid support, wherein the pre-determined fraction of locations for the second monomer includes locations containing the first and second monomer, locations containing the second monomer and locations containing no monomer; and repeating steps b-d with a defined set of monomers until the polymers reach a desired average length and the sum of the fractions total at least 100%.

In yet other cases, the methods and devices disclosed herein provide a method of using the arrays described herein to monitor the health status of a subject, comprising the steps of: collecting a biological sample from the subject; hybridizing the biological sample with the array; determining the components of the sample hybridizing to the array; evaluating the degree of hybridization; and determining the health status of the subject. The disclosed arrays can be used in the generation of immunosignature as described herein, but may also be used in other diagnostic and therapeutic assays utilizing microchip arrays for determining binding activity of targets in a complex biological sample.

Disclosed herein is a kit which can comprise a finger pricking device to draw a small quantity of blood from a subject and a receiving surface for the collection of the blood sample. In some cases, the kit comprises written instructions for a use thereof.

## BRIEF DESCRIPTION OF THE FIGURES

FIGURE 1 is a visual representation of the relative inter- and intra-group differences in Trial #1. The values for each of the 120 peptides and 120 patient samples are plotted with blue indicating low binding and red indicating high binding. Hierarchical clustering using Euclidean distance as the measure of similarity was used to cluster the peptides (Y axis) and patients (X axis). The hierarchy to the far left is based on this clustering.

FIGURE 2 illustrates a heatmap of samples from Trial #1. Panel A illustrates the heatmap of the training dataset using the 120 selected features. Panel B illustrates the unblended test data clustered using the same 120 peptides.

FIGURE 3 illustrates a heatmap of samples from Trial #2. 1516 samples (X axis) are shown with the values for each of the 255 predictor peptides (Y axis). Each disease is listed with the total number of patients indicated in parenthesis.

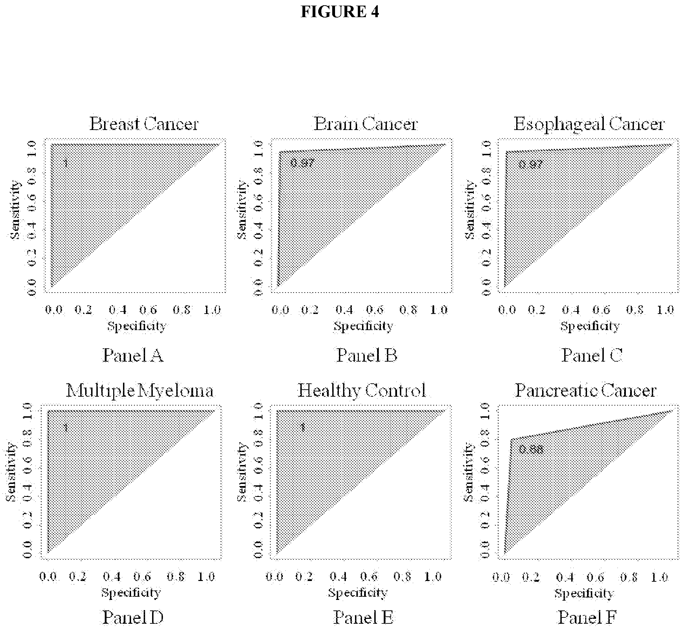

**FIGURE 4** is a graphical representation of Receiver Operator Characteristic (ROC) Curves for Trial #1. For each disease cohort of the test data from Trial #1, the sensitivity and specificity were calculated. Separate ROC curves were drawn and the Area under Curve (AUC) values calculated for each disease for each classification algorithm. The AUC for SVM is show in gray. Panel A is a graphical representation of a specificity/sensitivity AUC for SVM graph for Breast Cancer. Panel B is a graphical representation of a specificity/sensitivity AUC for SVM graph for Brain Cancer. Panel C is a graphical representation of a specificity/sensitivity AUC for SVM graph for Esophageal Cancer. Panel D is a graphical representation of a specificity/sensitivity AUC for SVM graph for Multiple Myeloma. Panel E is a graphical representation of a specificity/sensitivity AUC for SVM graph for Healthy controls. Panel F is a graphical representation of a specificity/sensitivity AUC for SVM graph for Pancreatic Cancer.

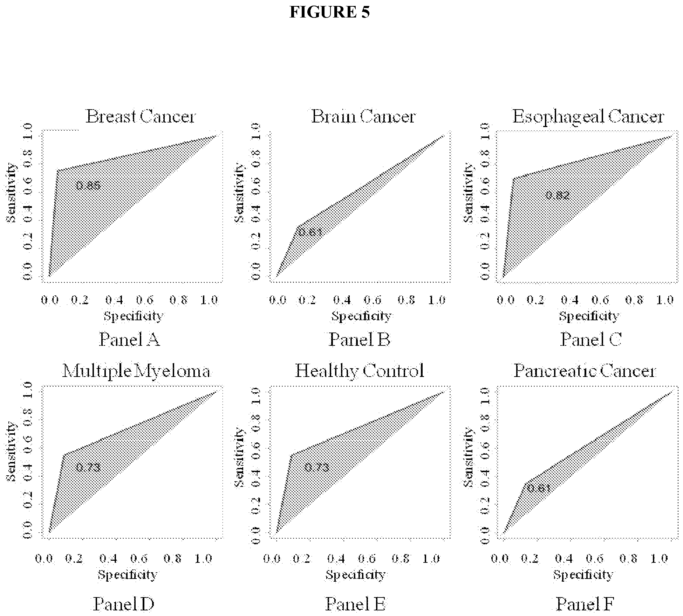

**FIGURE 5** is a graphical representation of Receiver Operator Characteristic (ROC) Curves for Trial #1. The Area Under Curve (AUC) for PCA is shown in gray. Panel A is a graphical representation of a specificity/sensitivity AUC for PCA graph for Breast Cancer. Panel B is a graphical representation of a specificity/sensitivity AUC for PCA graph for Brain Cancer. Panel C is a graphical representation of a specificity/sensitivity AUC for PCA graph for Esophageal Cancer. Panel D is a graphical representation of a specificity/sensitivity AUC for PCA graph for Multiple Myeloma. Panel E is a graphical representation of a specificity/sensitivity AUC for PCA graph for Healthy controls. Panel F is a graphical representation of a specificity/sensitivity AUC for PCA graph for Pancreatic Cancer.

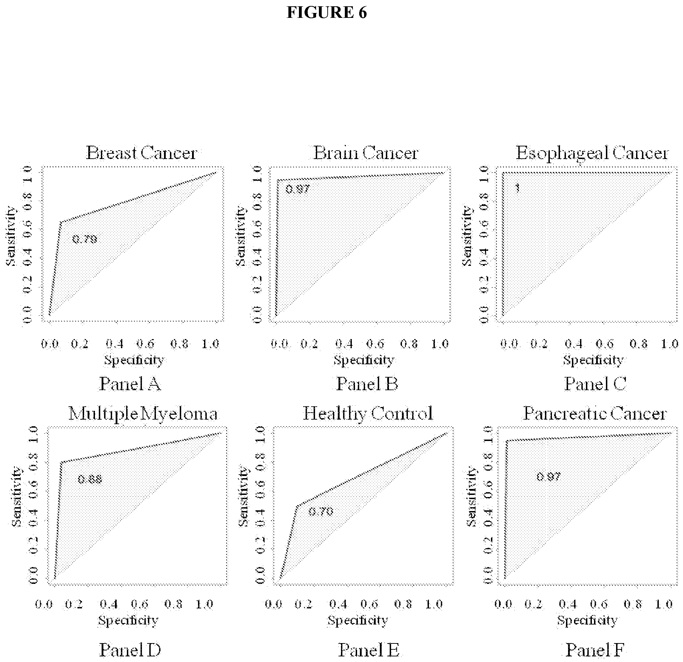

**FIGURE 6** is a graphical representation of Receiver Operator Characteristic (ROC) Curves for Trial #1. The Area Under Curve (AUC) for NB is shown in gray. Panel A is a graphical representation of a specificity/sensitivity AUC for NB graph for Breast Cancer. Panel B is a graphical representation of a specificity/sensitivity AUC for NB graph for Brain Cancer. Panel C is a graphical representation of a specificity/sensitivity AUC for NB graph for Esophageal Cancer. Panel D is a graphical representation of a specificity/sensitivity AUC for NB graph for Multiple Myeloma. Panel E is a graphical representation of a specificity/sensitivity AUC for NB graph for Healthy controls. Panel F is a graphical representation of a specificity/sensitivity AUC for NB graph for Pancreatic Cancer.

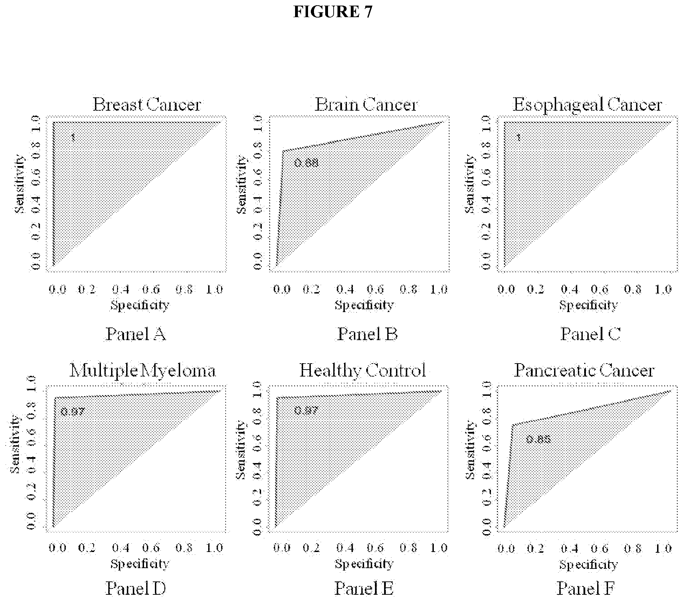

**FIGURE 7** is a graphical representation of Receiver Operator Characteristic (ROC) Curves for Trial #1. The Area Under Curve (AUC) for LDA is shown in gray. Panel A is a graphical representation of a specificity/sensitivity AUC for LDA graph for Breast Cancer. Panel B is a graphical representation of a specificity/sensitivity AUC for LDA graph for Brain Cancer. Panel C is a graphical representation of a specificity/sensitivity AUC for LDA graph for Esophageal Cancer. Panel D is a graphical representation of a specificity/sensitivity AUC for LDA graph for Multiple Myeloma. Panel E is a graphical representation of a specificity/sensitivity AUC for LDA graph for Healthy controls. Panel F is a graphical representation of a specificity/sensitivity AUC for LDA graph for Pancreatic Cancer.

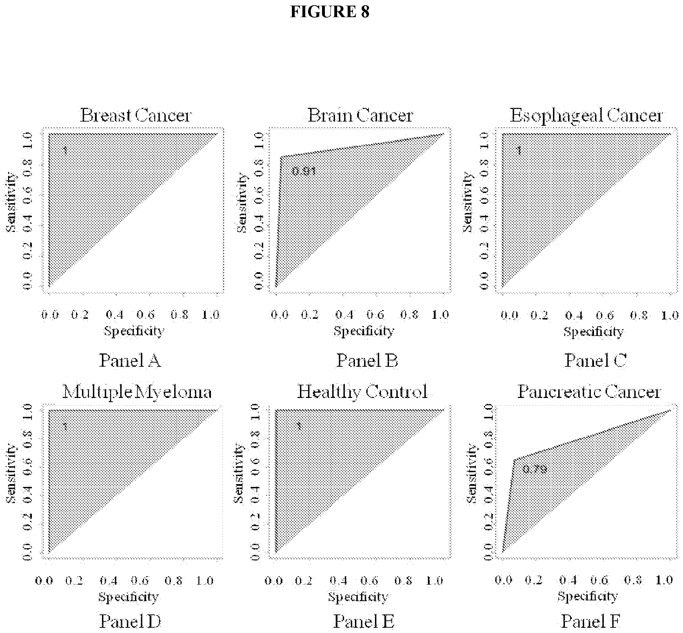

**FIGURE 8** is a graphical representation of Receiver Operator Characteristic (ROC) Curves for Trial #1. The Area Under Curve (AUC) for k-NN is shown in gray. Panel A is a graphical representation of a specificity/sensitivity AUC for k-NN graph for Breast Cancer. Panel B is a graphical representation of a specificity/sensitivity AUC for k-NN graph for Brain Cancer. Panel C is a graphical representation of a specificity/sensitivity AUC for k-NN graph for Esophageal Cancer. Panel D is a graphical representation of a specificity/sensitivity AUC for k-NN graph for Multiple Myeloma. Panel E is a graphical representation of a specificity/sensitivity AUC for k-NN graph for Healthy controls. Panel F is a graphical representation of a specificity/sensitivity AUC for k-NN graph for Pancreatic Cancer.

**FIGURE 9** is a graphical representation of four classifiers. Panel A is a graphical representation of PCA, the first two principal components are plotted. Panel B is a graphical representation of LDA, the X and Y axes depict the top two linear discriminants. Panel C is a graphical representation of NB, the predictor variable are plotted. Panel D is a graphical representation of k-NN, the groupwise distances are plotted.

**FIGURE 10** is a linegraph for 3 of the 255 classifier peptides from Trial #2. This intensity profile shows the individuals on the X axis, with the diseases separated by spaces, and the logic intensity for each peptide on the Y axis. Panel A illustrates a linegraph for a peptide high for disease 6 and 9 but low for all others. Panel B illustrates a linegraph for a peptide high for disease 11. Panel C illustrates a peptide high for disease 1 and part of disease 9.

**FIGURE 11** is a block diagram illustrating a first example architecture of a computer system that can be used in connection with example cases of the present invention.

**FIGURE 12** is a diagram illustrating a computer network that can be used in connection with example cases of the present invention.

**FIGURE 13** is a block diagram illustrating a second example architecture of a computer system that can be used in connection with example cases of the present invention.

**FIGURE 14** illustrates exemplary arrays of the invention with distinct peptide densities.

**FIGURE 15** is a heatmap illustrating an Immunosignature profile of multiple subjects over a period of time after receiving the flu vaccine.

**FIGURE 16** is a heatmap illustrating an Immunosignaturing binding pattern. Panel A illustrates an immunosignature of different biological samples from the same subject over the course of 1 day. Panel B illustrates a close up of a portion of Panel A.

**FIGURE 17** is a heatmap illustrating an Immunosignaturing binding pattern of 1 subject monitored over several months.

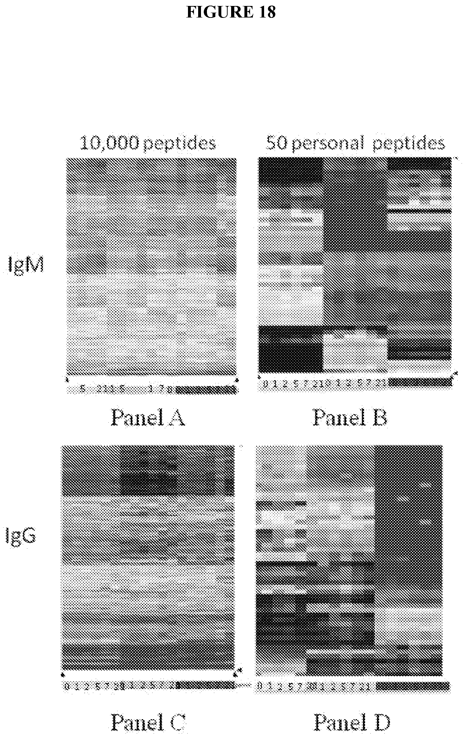

FIGURE 18 is a heatmap illustrating an Immunosignaturing binding pattern of 3 subjects over a time course of 21 days. Panel A illustrates the clustering of a peptide microarray with about 10,000 peptides when the binding of an IgM immunoglobulin is detected. Panel B illustrates the clustering of a peptide microarray with 50 personal peptides when the binding of an IgM immunoglobulin is detected. Panel C illustrates the clustering of a peptide microarray with about 10,000 peptides when the binding of an IgG immunoglobulin is detected. Panel D illustrates the clustering of a peptide microarray with 50 personal peptides when the binding of an IgG immunoglobulin is detected.

FIGURE 19 is a heatmap illustrating a 30 day health monitoring analyses of two subjects with Immunosignaturing binding pattern analysis.

FIGURE 20 is a heatmap illustrating an Immunosignaturing binding pattern of a subject who received a flu vaccine on day 17 of a 30 day time-course. The Immunosignaturing binding profile of the subject to 22 select peptide sequences is shown over distinct time-frames.

FIGURE 21 is a heatmap illustrating a diagnosis of the subject characterized in FIGURE 20 with bronchitis on 3/05/2013.

FIGURE 22 is a heatmap illustrating a post-symptom diagnosis of the subject characterized in FIGURE 20 with influenza on 12/11/2011.

FIGURE 23 is a heatmap illustrating an Immunosignaturing binding pattern of a subject receiving a treatment with a hepatitis vaccine, and a first booster treatment 3 months thereafter.

FIGURE 24 illustrates a summary of a classification of multiple infectious diseases. Panel A is a heatmap illustrating a clustered Immunosignaturing binding profile of Dengue, West Nile Virus (WNV), Syphilis, Hepatitis B Virus (HBV), Normal Blood, Valley Fever, and Hepatitis C Virus. Panel B is a graphical representation of a PCA classification.

FIGURE 25 is a diagram of components of an Immunosignaturing system of the invention.

FIGURE 26: Panel A illustrates a phage display library. Panel B illustrates a peptide microarray.

FIGURE 27 shows the average length of peptides synthesized as a function of the number of patterning steps. The X axis is the number of patterning cycles, and the Y axis is the average peptide length. Using an arbitrary number of patterning cycles, the patterning algorithms disclosed herein reduces patterning steps by almost a factor of two.

FIGURE 28 shows the results generated by applying all 20 amino acids as monomers using the standard layer by layer approach versus the novel patterning algorithms.

FIGURE 29 shows the results in an immunosignaturing case, where it takes less than 60 steps to achieve an average of 12 residues in length.

FIGURE 30 shows the distribution resulting from 70 steps of the optimized algorithm using 16 different amino acids.

FIGURE 31 shows a distribution of the lengths of the peptides selected after the peptide array generation.

FIGURE 32 are graphs showing the distributions of the possible sequences that are 3, 4 or 5 amino acids long.

FIGURE 33 shows the amino acid composition as a function of position in the peptide for a select peptide library.

## DETAILED DESCRIPTION

"Health" is a complex state that represents the continuously changing outcome of nearly all human activities and interactions. This makes it difficult to define health status quantitatively. Thousands of biochemical and physical attributes must be systematically measured. A great challenge in health monitoring is the complexity of a subject's response to various stimuli. Most living beings are exposed to a number of different stimuli every day, however some living creatures possess a system of biological structures and processes capable of responding to such stimuli, and protecting against the initiation or formation of disease. To function properly, such systems must detect a wide variety of stimuli, such as the presence of a virus or a parasitic worm, and initiate a response in the body against these substances, abnormal cells and/or tissues.

A corollary challenge in health monitoring is the complexity of a subject's response to complex stimuli. A physiological response produced by, for example, diseased cells within one's own body, can be different than a physiological response to an infection. Yet, the ability to detect, process, recognize, and act upon the early signs of, for example, an infection or a cancer, can have a significant impact in the health of a subject. If cancer is diagnosed before tumor cells have time to propagate, suppress the immune system, form metastatic colonies, and inflict tissue damage, then one can expect to respond more favorably to therapies.

Similarly, if the presence of a pathogen in a subject is detected soon after infection, antimicrobials can be administered before host inflammation prevents access of the invader, and before the pathogen load becomes immunologically overwhelming. If an autoimmune disease such as lupus is detected early, while auto-antibody levels are low, treatments to attenuate immunological flares can be far more effective. Fortunately, the immune system continuously monitors the state of health of a subject. However, robust, reliable, and effective methods for health monitoring and early detection remain an unmet need.

Immunosignaturing is a merger of microarray and phage technologies that displays the complexity of the humoral immune response and converts it into a machine-readable, quantitative format. Immunosignaturing detects even tiny perturbations in health status early and accurately. These comprehensive measurements of antibody repertoires provide the means for rapid, inexpensive and early diagnosis of any diseased state; ultimately, the continuous monitoring of immunosignatures may provide the means to detect dangerous disease states presymptomatically.

The invention disclosed herein thus provides sensitive, robust, effective, and reliable methods for health monitoring, diagnosis, treatment, and preventive health care. The cases disclosed herein address the lack of correlate and surrogate markers to a plurality of different conditions and health states by providing a large scale platform for the association of a humoral state of a subject with a condition.

Any component of a physiological system, whether foreign or self, can serve as a positive or negative marker of a condition, or a state of health. The immune system is a physiological system of biological structures and processes within an organism designed to detect a wide variety of markers, including foreign and self agents. An immune system can produce various antibodies which can be present in a peripheral blood sample of an individual and which can be endogenously amplified to high concentrations. Antibodies can be abundant, can have high target affinities, and can display a vast diversity of epitopes and structural flexibilities.

Components of the immune system, such as antibodies, can be very robust, and can act as suitable markers of the health state of a subject. Antibodies in blood, plasma, and/or serum can retain their integrity when subjected to heating, drying, and/or exposure to a wide range of pH values. Antibodies in blood, plasma, and/or serum can retain their integrity when subjected to long term storage either dry, frozen, or desiccated. Antibodies can retain partial and/or full integrity when, for example, the antibodies are kept on a dry filter paper and mailed. Such properties can render most blood, plasma, and/or serum samples potential sources of biological markers for use in a method of monitoring, diagnosing, preventing, and treating a condition.

A peptide array described herein can be structured to detect with high sensitivity a pattern of binding of a small quantity of a biological sample to a plurality of peptides in the array. Described herein is a method of detecting, processing, analyzing, and correlating the pattern of binding of the biological sample to the plurality of peptides with a condition. In some cases, the invention produces an "Immunosignature," which is associated with a state of health of a subject.

Immunosignaturing detects and partitions an antibody response into a coherent set of signals that can be mathematically interpreted. A coherent set of signals from an Immunosignature obtained with arrays and methods of the invention can provide a robust and comprehensive method for the diagnosis of various conditions, including cancer, inflammation, infection and other physiological conditions. Immunosignaturing is distinct from and an alternative to traditional, individual protein or genetic biomarkers for the diagnosis of various conditions. A coherent set of signals from an Immunosignature obtained with arrays and methods of the invention can be used as an effective method of preventive care, health monitoring, diagnosis, and as a method of treatment.

Multiplexed Detection of Antibody Biomarkers.

Diagnostic approaches designed to detect host-produced antibodies, rather than other less abundant biomarkers, are far more likely to be sufficiently sensitive to detect rare events. A plentiful supply of high-affinity, high-specificity antibodies do not need to be created since a tremendously diverse source of these markers already exists in circulating blood. In multiplexed arrays designed to detect antibodies, panels of protein or peptides are attached to a solid support and then exposed to blood.

Protein arrays are emerging as a high-capacity method capable of simultaneously detecting large numbers of parameters in a single experiment. Protein targets provide a source of conformational epitopes for antibody binding, though linear epitopes are not always exposed. Invitrogen produces one of the more comprehensive protein microarray containing ~9000 different baculovirus-produced human proteins arrayed onto a single slide. Large-scale potential for these protein arrays is dampened by high costs per slide, lack of scalability, and inconsistencies of recombinant protein production, purification, and stability. Using in vitro synthesized proteins has improved the throughput and success of protein production but inconsistencies in quantities arrayed, stability, post-translational modifications, and biases against membrane (surface), multimeric, and large proteins remain problematic. Both approaches are limited to detecting autoantibodies unless one specifically synthesizes known mutant or pathogen-derived candidate proteins. Biochemical fractionation of diseased cells enables antibodies against modified and mutated antigens to be queried but this is a substantially more complicated procedure (Hanash, S. (2003) Disease proteomics. Nature 422, 226-232).

In contrast to proteins, peptides can be synthesized chemically so that highly reproducible and pure products are available in large quantities, with long shelf lives. Attachment of biologically relevant modifications or detection molecules is simple, and non-natural designs are also possible (Reddy, M.M., et al. Identification of Candidate IgG Biomarkers for Alzheimer's Disease via Combinatorial Library Screening. Cell 144, 132-142).

Peptides are displayed in solution similarly, even when bound to a solid support; therefore, antibody interactions are screened against highly consistent structures regardless of batch to batch production differences. Peptide microarrays have been available far longer than protein microarrays (Panicker, R.C., et al. (2004) Recent advances in peptide-based microarray technologies. Comb Chem High Throughput Screen 7, 547-556), and have been used for a variety of applications. Enzymes (Fu, J., et al. (2010) Exploring peptide space for enzyme modulators. J Am Chem Soc 132, 6419-6424; and Fu, J., et al. (2011) Peptide-modified surfaces for enzyme immobilization. PLoS One 6, e18692), proteins (Diehnelt, C.W., et al. Discovery of high-affinity protein binding ligands-backwards. PLoS One 5, e10728; Greving, M.P., et al. High-throughput screening in two dimensions: binding intensity and off-rate on a peptide microarray. Anal Biochem 402, 93-95; Greving, M.P., et al. Thermodynamic additivity of sequence variations: an algorithm for creating high affinity peptides without large libraries or structural information. PLoS One 5, e15432; Gupta, N., et al. Engineering a synthetic ligand for tumor necrosis factor-alpha. Bioconjug Chem 22, 1473-1478), DNA and small molecules (Boltz, K.W., et al. (2009) Peptide microarrays for carbohydrate recognition. Analyst 134, 650-652; Foong, Y.M., et al. (2012) Current advances in peptide and small molecule microarray technologies. Curr Opin Chem Biol 16, 234-242; Morales Betanzos, C., et al. (2009) Bacterial glycoprofiling by using random sequence peptide microarrays. Chembiochem 10, 877-888), whole cells (Falsey, J.R., et al. (2001) Peptide and small molecule microarray for high throughput cell adhesion and functional assays. Bioconjug Chem 12, 346-353), and antibodies (Cerecedo, I., et al. (2008) Mapping of the IgE and IgG4 sequential epitopes of milk allergens with a peptide microarray-based immunoassay. J Allergy Clin Immunol 122, 589-594; Cretich, M., et al. (2009) Epitope mapping of human chromogranin A by peptide microarrays. Methods Mol Biol 570, 221-232; Lin, J., et al. (2009) Development of a novel peptide microarray for large-scale epitope mapping of food allergens. J Allergy Clin Immunol 124, 315-322, 322 e311-313; Lorenz, P., et al. (2009) Probing the epitope signatures of IgG antibodies in human serum from patients with autoimmune disease. Methods Mol Biol 524, 247-258; Perez-Gordo, M., et al. (2012) Epitope mapping of Atlantic salmon major allergen by peptide microarray immunoassay. Int. Arch Allergy Immunol 157, 31-40; and Shreffler, W.G., et al. (2005) IgE and IgG4 epitope mapping by microarray immunoassay reveals the diversity of immune response to the peanut allergen, Ara h 2. J Allergy Clin Immunol 116, 893-899 are just a subset of the biomolecules that can be assayed for binding to peptides. A classic example is epitope mapping: peptides that span an antigen can be tiled to efficiently decipher the epitope of a monoclonal antibody. A high-specificity antibody will recognize and bind its epitope sequence with little or no measurable binding to other antigen-derived peptides, usually. With this method, different monoclonals raised against the same antigen can be distinguished and characterized.

<u>Relevance of Cross-Reactivity.</u>

The immune system has evolved to elicit and amplify antibodies that ignore self proteins and bind non-self targets with significant strength. The immune system has evolved to elicit and amplify antibodies that ignore self proteins and bind non-self targets with significant strength. These conflicting pressures become clear at the molecular level. A typical antibody recognizes an epitope of ~15 amino acids of which ~5 dominate the binding energy. A change in any of these 5 residues will greatly affect binding strength.

Sequence changes in other epitope positions will alter the spatial conformation of the binding region and modestly affect overall strength. Therefore if binding strength is to be maximized, conditions must be adjusted to permit both high and low affinity residues to interact. This implies a reduced stringency and consequently, allows variants of the epitope sequence to bind an antibody. Further contributing to the potential for cross-reactivity, antibodies have a 50 amino acid variable region that contains many overlapping paratopes (the epitope-recognizing portions of the antibody) (Mohan, S., et al. (2009) Association energetics of cross-reactive and specific antibodies. Biochemistry 48, 1390-1398; Thorpe, I.F., and Brooks, C.L., 3rd (2007) Molecular evolution of affinity and flexibility in the immune system. Proceedings of the National Academy of Sciences of the United States of America 104, 8821-8826; and Zhou, Z.H., et al. (2007) Properties and function of polyreactive antibodies and polyreactive antigen-binding B cells. J Autoimmun. 29, 219-228. Epub 2007 Sep 2020).

Each of these paratopes is comprised of ~15 amino acids such that paratopes and epitopes are similarly-sized stretches that define complementary regions of shape and charge. A paratope can bind more than one epitope, and a single epitope can bind to more than one paratope, each pair displaying unique binding properties. Since a single antibody carries multiple paratopes, an antibody has a distinct yet potentially diverse set of epitopes that it can bind, with varying strengths. This cross-reactivity and complex interplay of specificity and affinity are hallmarks of a sophisticated immune system that orchestrates a direct attack against an immediate threat and indirect attacks against possible exposure to variants in the future.

In vitro, antibodies specific to a particular linear epitope have been shown not only to bind sequence-related peptides but also unrelated ones (Folgori, A., et al. (1994) A general strategy to identify mimotopes of pathological antigens using only random peptide libraries and human sera. Embo J 13, 2236-2243). These sequence-unrelated peptides, typically showing conformational relatedness, are known as mimotopes and were originally described in early phage display studies (Folgori, A., et al. (1994) A general strategy to identify mimotopes of pathological antigens using only random peptide libraries and human sera. Embo J 13, 2236-2243; Christian, R.B., et al. (1992) Simplified methods for construction, assessment and rapid screening of peptide libraries in bacteriophage. Journal of Molecular Biology 227, 711-718; Liu, R., et al. (2003) Combinatorial peptide library methods for immunobiology research. Experimental Hematology 31, 11-30; Wang, Y., et al. (1995) Detection of Mammary Tumor Virus ENV Gene-like Sequences in Human Breast Cancer. Cancer Research 55, 5173-5179.

Phage-based systems provide the opportunity to build and screen libraries of much larger ligand diversity than possible with most other systems. For example, large random sequence libraries displaying peptides were panned against a particular monoclonal antibody. Iterative rounds of selection often led to the identification of the cognate epitope, but several unrelated peptide sequences as well. The fact that random peptide diversity is many orders of magnitude greater than biological sequence diversity means that the peptides will not correspond to any biological peptide. All binding reactions rely on non-cognate, cross reactivity, an inherent property of antibodies. This implies that a ligand for any category of antibody could be identified: autoantigen, modified antigen, mutated epitope, or non-peptidic mimotope. Despite these advantages to screening in random sequence space, phage display techniques are limited by the repeated rounds of panning with phage and bacterial cultures, a binary selection process, and lack of scalability (Derda, R., et al. (2011) Diversity of phage-displayed libraries of peptides during panning and amplification. Molecules 16, 1776-1803; Szardenings, M. (2003) Phage display of random peptide libraries: applications, limits, and potential. J Recept Signal Transduct Res 23, 307-34953, 54). To date, random phage libraries have not yielded an antibody biomarker.

Immunosignaturing.

Immunosignaturing is a synthesis of the technologies described above. First, rather than display peptides biologically on a phage, linking synthetic and longer peptides onto a glass slide in addressable ordered arrays is a far more systematic method. Although phage libraries can exceed $10^{10}$ individual clones, microarrays have increased from a few thousand to millions of spots per slide. The cost, reliability, precision, and assay speed imbue microarrays with significant advantages. Microarrays have proven themselves invaluable for genomics and proteomics due to their low cost and scalability and commercial array chambers and scanners have existed for years.

Second, using antibodies as biomarkers of disease takes advantage of a stable and easily accessible molecule and the immune system's convenient properties of diversity, surveillance, and biological amplification. The complexity of a mammalian immune system is staggering (Janeway, C., and Travers, J. (1997) Immunobiology: The Immune System in Health and Disease. Current Biology Limited) and therefore so is the information content. As immunologists explore the immunome there is growing consensus that the antibody repertoire, capable of >$10^{10}$ different molecular species (Nobrega, A., et al. (1998) Functional diversity and clonal frequencies of reactivity in the available antibody repertoire. European Journal of Immunology 28, 1204-1215), is a dynamic database of past, current, and even prodromic perturbations to an individual's health status.

Third, use of random sequence peptides enables the diversity of the antibody repertoire to be matched by an unbiased, comprehensive library of ligands to screen. Random-sequence peptides can be used in phage display libraries, but they carry biases and are not in an unordered, poorly controlled format. Since random peptide sequences have no constraints and no intentional homology to biological space, the microarrays contain sparse but very broad coverage of sequence space. Normal, mutated, post-translationally modified, and mimetic epitopes corresponding to any disease or organism can be screened on the same microarray. Recent publications in the field have used 10,000 unique random-sequence 20-mer peptides to characterize a multitude of disease states 1, 10, (Brown, J.R., et al. (2011) Statistical methods for analyzing immunosignatures. BMC Bioinformatics 12,349; Hughes, A.K., et al. (2012) Immunosignaturing can detect products from molecular markers in brain cancer. PLoS One 7, e40201; Kroening, K., et al. (2012) Autoreactive antibodies raised by self derived de novo peptides can identify unrelated antigens on protein microarrays. Are autoantibodies really autoantibodies? Exp Mol Pathol 92, 304-311; Kukreja, M., et al. (2012) Comparative study of classification algorithms for immunosignaturing data. BMC Bioinformatics 13, 139; Kukreja, M., et al. (2012) Immunosignaturing Microarrays Distinguish Antibody Profiles of Related Pancreatic Diseases. Journal of Proteomics and Bioinformatics; and Legutki, J.B., et al. (2010) A general method for characterization of humoral immunity induced by a vaccine or infection. Vaccine 28, 4529-4537).

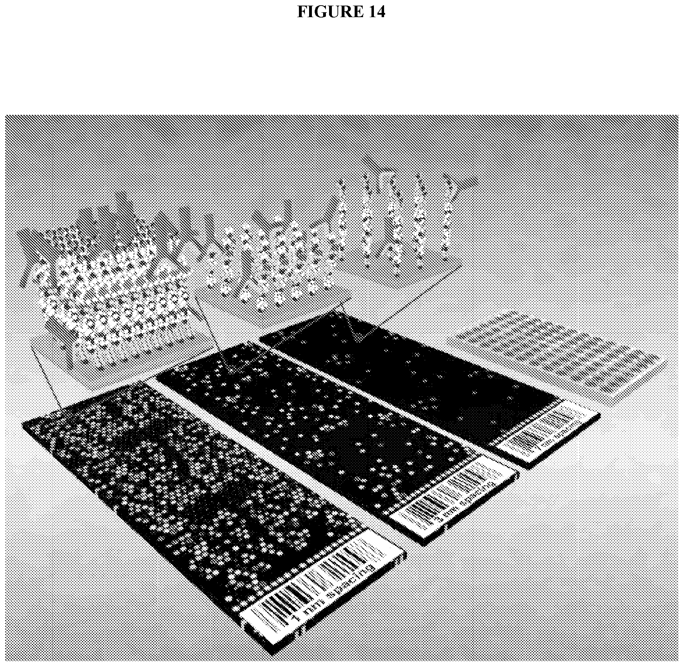

There are several notable differences in the results obtained from phage display versus immunosignaturing microarrays. Immunosignaturing queries all of the peptides on the array and produces binding values for each. Phage display yields sequences that survive restrictive selection, and typically identifies only consensus sequences. Processing immunosignaturing microarrays takes hours rather than weeks. **FIGURE 14** displays the distinction between these technologies. Technically an 'immunosignature' refers to the statistically significant pattern of peptides, each with specific binding values that can robustly classify one state of disease from others.

This integration of technologies may represent progress toward the goal of a universally applicable early diagnostic platform. The key issues remaining to be addressed are whether or not: i) the immune system elicits consistent disease-specific humoral responses to both infectious and chronic diseases, ii) antibodies respond sufficiently early in the etiology of disease to be clinically useful and iii) the assay is sufficiently sensitive, informative, inexpensive, and scalable to screen large numbers of patient samples for confident determinations. If these points can be satisfied, then the immunosignature of any immune-related disease can be discovered. These defined patterns of reactivity can then be used to diagnose disease early and comprehensively. If these tests can be made widely accessible to the population, immunosignaturing could form the basis for a long-term health monitoring system with important implications at individual but also epidemiological levels. We present several features of the platform that are promising in this regard.

Immunosignaturing is a synthesis of the technologies described above. First, rather than display peptides biologically on a phage, linking synthetic and longer peptides onto a glass slide in addressable ordered arrays is a far more systematic method. Although phage libraries can exceed $10^{10}$ individual clones, microarrays have increased from a few thousand to millions of spots per slide. The cost, reliability, precision, and assay speed imbue microarrays with significant advantages. Microarrays have proven themselves invaluable for genomics and proteomics due to their low cost and scalability and commercial array chambers and scanners have existed for years. Second, using antibodies as biomarkers of disease takes advantage of a stable and easily accessible molecule and the immune system's convenient properties of diversity, surveillance, and biological amplification.

The complexity of a mammalian immune system is staggering and therefore so is the information content. As immunologists explore the immunome there is growing consensus that the antibody repertoire, capable of >$10^{10}$ different molecular species, is a dynamic database of past, current, and even prodromic perturbations to an individual's health status. Third, use of random sequence peptides enables the diversity of the antibody repertoire to be matched by an unbiased, comprehensive library of ligands to screen. Random-sequence peptides can be used in phage display libraries, but they carry biases and are not in an unordered, poorly controlled format. Since random peptide sequences have no constraints and no intentional homology to biological space, the microarrays contain sparse but very broad coverage of sequence space.

Normal, mutated, post-translationally modified, and mimetic epitopes corresponding to any disease or organism can be screened on the same microarray. Publications in the field have used 10,000 unique random-sequence 20-mer peptides to characterize a multitude of disease states. There are several notable differences in the results obtained from phage display versus immunosignaturing microarrays. Immunosignaturing queries all of the peptides on the array and produces binding values for each. Phage display yields sequences that survive restrictive selection, and typically identifies only consensus sequences.

Processing immunosignaturing microarrays can take hours rather than weeks. An 'immunosignature' refers to the statistically significant pattern of peptides, each with specific binding values that can robustly classify one state of disease from others. Accordingly, one aspect of the cases disclosed herein is the relatively quick processing time for querying an immunosignature array with a complex biological sample, wherein the querying and processing time can take up to 10 minutes, up to 20 minutes, up to 30 minutes, up to 45 minutes, up to 60 minutes, up to 90 minutes, up to 2 hours, up to 3 hours, up to 4 hours or up to 5 hours. Alternatively, the querying and processing time can take not more than 10 minutes, not more than 20 minutes, not more than 30 minutes, not more than 45 minutes, not more than 60 minutes, not more than 90 minutes, not more than 2 hours, not more than 3 hours, not more than 4 hours or not more than 5 hours.

This integration of technologies may represent progress toward the goal of a universally applicable early diagnostic platform. The key issues remaining to be addressed are whether or not: i) the immune system elicits consistent disease-specific humoral responses to both infectious and chronic diseases, ii) antibodies respond sufficiently early to in the etiology of disease to be clinically useful and iii) the assay is sufficiently sensitive, informative, inexpensive, and scalable to screen large numbers of patient samples for confident determinations. If these points can be satisfied, then the immunosignature of any immune-related disease can be discovered.

These defined patterns of reactivity can then be used to diagnose disease early and comprehensively. If these tests can be made widely accessible to the population, immunosignaturing could form the basis for a long-term health monitoring system with important implications at individual but also epidemiological levels. We present several features of the platform that are promising in this regard.

Unique Features of Immunosignaturing.

In addition to the affinity of an antibody's paratope for the ligand, binding strength can be influenced by the concentration of the antibody species in serum. Unlike phage display, immunosignaturing can quantitatively measure the product of these parameters, and can do so with a very large dynamic range (Legutki, J.B., et al. (2010) A general method for characterization of humoral immunity induced by a vaccine or infection. Vaccine 28, 4529-4537; Stafford, P., and Johnston, S. (2011) Microarray technology displays the complexities of the humoral immune response. Expert Rev Mol Diagn 11, 5-8; Halperin, R.F., et al. (2011) Exploring Antibody Recognition of Sequence Space through Random-Sequence Peptide Microarrays. Molecular & Cellular Proteomics 10).

Scientists used this capability to examine the binding of high affinity monoclonal antibodies to the immunosignaturing microarrays. They found that a single monoclonal recognized hundreds of random sequences, and the varying strengths of these unique binding reactions could be measured and compared (Halperin, R.F., et al. (2011) Exploring Antibody Recognition of Sequence Space through Random-Sequence Peptide Microarrays. Molecular & Cellular Proteomics 10). Curiously, many of these off-target mimotope interactions had higher binding than the cognate epitope. Although the corresponding solution-phase binding of these interactions is low, the way the immunosignaturing microarray is constructed enhances these interactions. This immunological phenomenon of off-target antibody binding to the immunosignaturing microarray is central to the technology.

Another important observation is the greater sensitivity of immunosignaturing for the detection of low affinity interactions than either phage display or ELISA-based assays (Stafford, P., and Johnston, S. (2011) Microarray technology displays the complexities of the humoral immune response. Expert Rev Mol Diagn 11). The high sensitivity is a consequence of the high density of peptides on the slide surface and has been called the "immunosignaturing effect". This has been established by printing and testing different spatial arrangements of peptides on the functionalized glass surface. If arrays are printed such that peptides are spaced about 9 to about 12 nm apart, cognate epitopes compete for antibodies more favorably than the off-target random peptides (with the exception of very strong mimotopes).

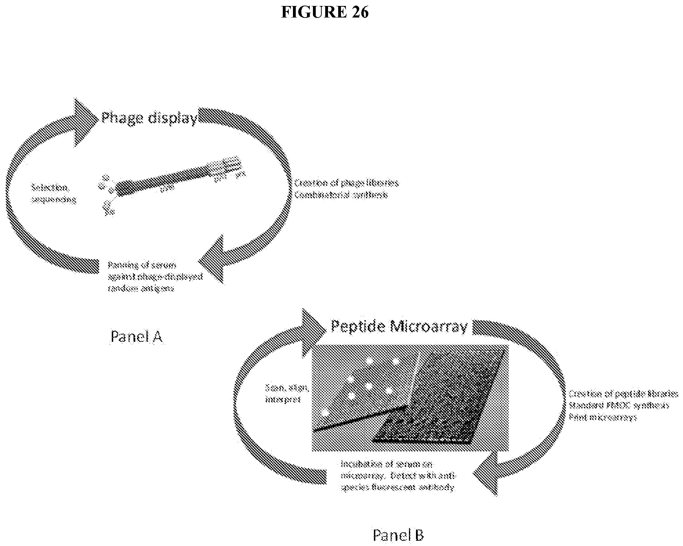

We commonly space peptides 1-2 nm apart on average but observe the off-target binding with peptides spaced 3-4 nm apart. If the peptides are spaced from about 1 to about 1.5 nm apart, then an increase in off-target binding is observed. Tightly packed peptides appear to trap antibodies through avidity and rapid rebinding. This concept has been shown to be extremely reproducible, and is illustrated in **FIGURE 26** (Stafford, P., et al. (2012) Physical characterization of the "immunosignaturing effect". Mol Cell Proteomics 11, M111 011593; Chase, B.A., et al. (2012) Evaluation of biological sample preparation for immunosignature-based diagnostics. Clin Vaccine Immunol 19, 352-358; Hughes, A.K., et al. (2012) Immunosignaturing can detect products from molecular markers in brain cancer. PLoS One 7, e40201; Restrepo, L., et al. (2011) Application of immunosignatures to the assessment of Alzheimer's disease. Annals of Neurology 70, 286-295). While the sequences of the peptides are entirely random, their off-target captures of antibody are clearly not; rather, the patterns of sera binding to the array are remarkably coherent. An early concern relative to this technology was that the large diversity of antibody species in any serum sample might lead to overlapping binding competitions resulting in a flat, uninformative field of intensities. The data have not borne this out. In fact even a purified monoclonal antibody diluted into serum retains its distinct reactivity pattern with little to no loss of binding (Uhlen, M., and Hober, S. (2009) Generation and validation of affinity reagents on a proteome-wide level. J Mol Recognit 22, 57-64).

Classical statistical models used to explain conventional nucleic acid microarrays (Draghici, S. (2012) Statistics and Data Analysis for Microarrays Using R and Bioconductor. Chapman & Hall/CRC) do not have the flexibility to address the new complexities presented by immunosignature arrays. Rather than a one-to-one binding model that describes RNA or DNA binding to complimentary probes on a microarray, the immunosignaturing peptides may bind to more than one antibody, and many different antibodies can bind to the same peptide. Three different reports compared methods for image analysis (Yang, Y., et al. (2011) Segmentation and intensity estimation for microarray images with saturated pixels. BMC Bioinformatics 12, 462), factor analysis and mixture models (Brown, J.R., et al. (2011) Statistical methods for analyzing immunosignatures. BMC Bioinformatics 12, 349), and classification (Kukreja, M., et al. (2012) Comparative study of classification algorithms for immunosignaturing data. BMC Bioinformatics 13, 139) specifically for immunosignaturing. There are a number of fundamental properties of the immunosignaturing microarray that enable discriminating diseases.

First, control sera from healthy volunteers display a rather broad distribution of baseline binding reactivity. This imposes a requirement that a large-scale study using the technology must sample sera from a large number of non-diseased individuals to accommodate the population variability. Second, signatures from sera of persons with a given disease are extremely consistent, unlike that of the non-disease sera. This observation implies that the immune system is constantly probing and reacting to local environments causing broad differences in signatures. However, once directed toward an antigen, antibodies tend to form a narrow and well-defined signature with little individual variability.

Even so, the technology is able to discern sub-types of disease (Hughes, A.K., et al. (2012) Immunosignaturing can detect products from molecular markers in brain cancer. PLoS One 7, e40201) while still providing a distinction between controls and affected. The analysis of common relationships and covariances between pluralities of peptides provides tremendous discerning power that is not possible at the single epitope level. Immunologically, the antibody: peptide binding patterns are not created by a non-specific danger signal or the activities of natural antibodies: they are created by a recognizable stimulus. Antibody adsorption experiments demonstrated that the peptides from an influenza infection bind mostly virus-specific antibodies and the signature of Alzheimer's Disease binds many anti-A$\beta$ antibodies (Legutki, J.B., et al. (2010) A general method for characterization of humoral immunity induced by a vaccine or infection. Vaccine 28, 4529-4537; Restrepo, L., et al. (2011) Application of immunosignatures to the assessment of Alzheimer's disease. Annals of Neurology 70, 286-295).

The disease determinations by immunosignaturing have correlated well with the results obtained using current diagnostic tests (Hughes, A.K., et al. (2012) Immunosignaturing can detect products from molecular markers in brain cancer. PLoS One 7, e40201; Kukreja, M., et al. (2012) Immunosignaturing Microarrays Distinguish Antibody Profiles of Related Pancreatic Diseases. Journal of Proteomics and Bioinformatics; Legutki, J.B., et al. (2010) A general method for characterization of humoral immunity induced by a vaccine or infection. Vaccine 28, 4529-4537). Immunosignatures carry historical health information not accessible with traditional diagnostics; namely, both immediate and memory responses can be detected (Legutki, J.B., et al. (2010) A general method for characterization of humoral immunity induced by a vaccine or infection. Vaccine 28, 4529-4537). To date the approach has been applied to more than 33 different diseases and sequelae including viral, bacterial, fungal and parasitic infections, cancers, diabetes, autoimmune disease, transplant patients and many chronic diseases in mouse, rat, dog, pig, and human hosts. A highly reproducible pattern of peptide binding patterns can be established that correlates with pathology.

These binding profiles correctly classify blinded sera samples obtained from patients and healthy volunteers and outperform classic immunological tests in sensitivity and accuracy. In a large-scale study, immunosignatures were able to diagnose Valley Fever patients with very high accuracy, including the correct diagnosis of patients that were initially negative by standard ELISA tests. Analyses of patient immunosignatures were able to distinguish among and within cancers (Brown, J.R., et al. (2011) Statistical methods for analyzing immunosignatures. BMC Bioinformatics 12,349; Hughes, A.K., et al. (2012) Immunosignaturing can detect products from molecular markers in brain cancer. PLoS One 7, e40201; Kukreja, M., et al. (2012) Immunosignaturing Microarrays Distinguish Antibody Profiles of Related Pancreatic Diseases. Journal of Proteomics and Bioinformatics; and Yang, Y., et al. (2011) Segmentation and intensity estimation for microarray images with saturated pixels. BMC Bioinformatics 12, 462) even to the point of accurately diagnosing cancer types that will and will not respond to drug treatment (Hughes, A.K., et al. Immunosignaturing can detect products from molecular markers in brain cancer. PLoS One 7, e40201).

One of the most unique features of the immunosignaturing technology may turn out to be measurement of decreases in particular peptide:antibody reactivity, a class of interactions previously not measurable. Namely, while sera from diseased individuals produce high signals relative to normal sera, there are also peptides that consistently show reduced binding relative to healthy persons. (Kukreja, M., et al. (2012) Immunosignaturing Microarrays Distinguish Antibody Profiles of Related Pancreatic Diseases. Journal of Proteomics and Bioinformatics; and Legutki, J.B., et al. (2010), A general method for characterization of humoral immunity induced by a vaccine or infection. Vaccine 28, 4529-4537). The role of these "down" peptides in an immune response is intriguing. Although at its simplest level, these "down" peptides enhance disease classification, there may be some underlying immunological phenomenon that would not otherwise be seen.

Binding of Molecules to an Array.

According to the National Cancer Institute, there are approximately 150 classes of cancer and, depending on how one defines them, hundreds of distinct subtypes. Antibodies are often raised against antigens expressed by tumor cells, and are subsequently amplified during B-cell maturation. Antibodies are also raised during a response to a vaccine or infection. Antibodies can also be raised during the daily exposure of a subject to various pathogenic, as well as non-pathogenic stimuli.

The process of antibody amplification in a subject's body can generate an ample supply of subject specific markers associated with a condition. Antibody amplification can provide ample numbers of antibodies which are associated with a specific health state of a subject and/or a condition. The presence of a sufficient number of antibodies in a sample can reduce a requirement for artificial biomarker amplification in a method of health monitoring. The presence of a sufficient number of antibodies in a sample can allow a small quantity of sample to be successfully applied in, for example, a method of health monitoring.

The methods and arrays of the invention allow for health monitoring, diagnosis, treatment, and prevention with small quantitites of biological samples from a subject. In some cases, the biological samples can be used in a method of the invention without further processing and in small quantities. In some cases, the biological samples comprise, blood, serum, saliva, sweat, cells, tissues, or any bodily fluid. In some cases, about 0.5 nl, about 1 nl, about 2 nl, about 3 nl, about 4 nl, about 5 nl, about 6 nl, about 7 nl, about 8 nl, about 9 nl, about 10 nl, about 11 nl, about 12 nl, about 13 nl, about 14 nl, about 15 nl, about 16 nl, about 17 nl, about 18 nl, about 19 nl, about 20 nl, about 21 nl, about 22 nl, about 23 nl, about 24 nl, about 25 nl, about 26 nl, about 27 nl, about 28 nl, about 29 nl, about 30 nl, about 31 nl, about 32 nl, about 33 nl, about 34 nl, about 35 nl, about 36 nl, about 37 nl, about 38 nl, about 39 nl, about 40 nl, about 41 nl, about 42 nl, about 43 nl, about 44 nl, about 45 nl, about 46 nl, about 47 nl, about 48 nl, about 49 nl, or about 50 nl, about 51 nl, about 52 nl, about 53 nl, about 54 nl, about 55 nl, about 56 nl, about 57 nl, about 58 nl, about 59 nl, about 60 nl, about 61 nl, about 62 nl, about 63 nl, about 64 nl, about 65 nl, about 66 nl, about 67 nl, about 68 nl, about 69 nl, about 70 nl, about 71 nl, about 72 nl, about 73 nl, about 74 nl, about 75 nl, about 76 nl, about 77 nl, about 78 nl, about 79 nl, about 80 nl, about 81 nl, about 82 nl, about 83 nl, about 84 nl, about 85 nl, about 86 nl, about 87 nl, about 88 nl, about 89 nl, about 90 nl, about 91 nl, about 92 nl, about 93 nl, about 94 nl, about 95 nl, about 96 nl, about 97 nl, about 98 nl, about 99 nl, about 0.1, about 0.2 µl, about 0.3 µl, about 0.4 µl, about 0.5 µl, about 0.6 µl. about 0.7 µl, about 0.8 µl, about 0.9 µl, about 1 µl, about 2 µl, about 3 µl, about 4 µl, about 5 µl, about 6 µl, about 7 µl, about 8 µl, about 9 µl, about 10 µl, about 11 µl, about 12 µl, about 13 µl, about 14 µl, about 15 µl, about 16 µl, about 17 µl, about 18 µl, about 19 µl, about 20 µl, about 21 µl, about 22 µl, about 23 µl, about 24 µl, about 25 µl, about 26 µl, about 27 µl, about 28 µl, about 29

μl, about 30 μl, about 31 μl, about 32 μl, about 33 μl, about 34 μl, about 35μl, about 36 μl, about 37 μl, about 38 μl, about 39 μl, about 40 μl, about 41 μl, about 42 μl, about 43 μl, about 44 μl, about 45 μl, about 46 μl, about 47 μl, about 48 μl, about 49 μl, or about 50 μl of biological samples are required for analysis by an array and method of the invention.

The methods and arrays of the invention allow for health monitoring, diagnosis, treatment, and prevention with small quantities of biological samples from a subject. In some cases, the methods of the invention require no more than about 0.5 nl to about 50 nl, no more than about 1 nl to about 100 nl, no more than about 1 nl to about 150 nl, no more than about 1 nl to about 200 nl, no more than about 1 nl to about 250 nl, no more than about 1 nl to about 300 nl, no more than about 1 nl to about 350 nl, no more than about 1 nl to about 400 nl, no more than about 1 to about 450 nl, no more than about 5 nl to about 500 nl, no more than about 5 nl to about 550 nl, no more than about 5 nl to about 600 nl, no more than about 5 nl to about 650 nl, no more than about 5 nl to about 700 nl, no more than about 5 nl to about 750 nl, no more than about 5 nl to about 800 nl, no more than about 5 nl to about 850 nl, no more than about 5 nl to about 900 nl, no more than about 5 nl to about 950 nl, no more than about 5 nl to about 1 μl, no more than about 0.5 μl to about 1 μl, no more than about 0.5 μl to about 5 μl, no more than about 1 μl to about 10 μl, no more than about 1 μl to about 20 μl, no more than about 1 μl to about 30 μl, no more than about 1 μl to about 40 μl, or no more than about 1 μl to about 50 μl.

In some cases, the methods described herein require at least 0.5 nl to about 50 nl, at least about 1 nl to about 100 nl, at least about 1 nl to about 150 nl, at least about 1 nl to about 200 nl, at least about 1 nl to about 250 nl, at least about 1 nl to about 300 nl, at least about 1 nl to about 350 nl, at least about 1 nl to about 400 nl, at least about 1 to about 450 nl, at least about 5 nl to about 500 nl, at least about 5 nl to about 550 nl, at least about 5 nl to about 600 nl, at least about 5 nl to about 650 nl, at least about 5 nl to about 700 nl, at least about 5 nl to about 750 nl, at least about 5 nl to about 800 nl, at least about 5 nl to about 850 nl, at least about 5 nl to about 900 nl, at least about 5 nl to about 950 nl, at least about 5 nl to about 1 μl, at least about 0.5 μl to about 1 μl, at least about 0.5 μl to about 5 μl, at least about 1 μl to about 10 μl, at least about 1 μl to about 20 μl, at least about 1 μl to about 30 μl, at least about 1 μl to about 40 μl, at least about 1 μl to about 50 μl, or at least 50 μl

The methods and arrays described herein provide sensitive methods for health monitoring, diagnosis, treatment, and prevention of conditions with small quantities of biological samples from a subject. In some cases, biological samples from a subject are too concentrated and require a dilution prior to being contacted with an array of the invention. A plurality of dilutions can be applied to a biological sample prior to contacting the sample with an array of the invention. A dilution can be a serial dilution, which can result in a geometric progression of the concentration in a logarithmic fashion. For example, a ten-fold serial dilution can be 1 M, 0.01 M, 0.001 M, and a geometric progression thereof. A dilution can be, for example, a one-fold dilution, a two-fold dilution, a three-fold dilution, a four-fold dilution, a five-fold dilution, a six-fold dilution, a seven-fold dilution, an eight-fold dilution, a nine-fold dilution, a ten-fold dilution, a sixteen-fold dilution, a twenty-five-fold dilution, a thirty-two-fold dilution, a sixty-four-fold dilution, and/or a one-hundred-and-twenty-five-fold dilution.

The binding of a molecule to an array described herein creates a pattern of binding that can be associated with a condition. The affinity of binding of a molecule to a peptide in the array can be mathematically associated with a condition. The off-target binding pattern of an antibody to a plurality of different peptides of the invention can be mathematically associated with a condition. The avidity of binding of a molecule to a plurality of different peptides of the invention can be mathematically associated with a condition. The off-target binding and avidity can comprise the interaction of a molecule in a biological sample with multiple, non-identical peptides in a peptide array. An avidity of binding of a molecule with multiple, non-identical peptides in a peptide array can determine an association constant of the molecule to the peptide array. In some cases, the concentration of an antibody in a sample contributes to an avidity of binding to a peptide array, for example, by trapping a critical number or antibodies in the array and allowing for rapid rebinding of an antibody to an array.

The avidity of binding of biological molecules to an array can be determined by a combination of multiple bond interactions. A cross-reactivity of an antibody to multiple peptides in a peptide array can contribute to an avidity of binding. In some cases, an antibody can recognize an epitope of about 3 amino acids, about 4 amino acids, about 5 amino acids, about 6 amino acids, about 7 amino acids, about 8 amino acids, about 9 amino acids, about 10 amino acids, about 11 amino acids, about 12 amino acids, about 13 amino acids, about 14 amino acids, about 15 amino acids, about 16 amino acids, or about 17 amino acids. In some cases, a sequence of about 5 amino acids dominates a binding energy of an antibody to a peptide.

An off-target binding, and/or avidity, of a molecule to an array described herein can, for example, effectively compress binding affinities that span femtomolar (fM) to micromolar (μM) dissociation constants into a range that can be quantitatively measured using only 3 logs of dynamic range. A molecule can bind to a plurality of peptides in the array with association constants of $10^3$ M$^{-1}$ or higher. A molecule can bind to a plurality of peptides in the array with association constants ranging from $10^3$ to $10^6$ M$^{-1}$, $2 \times 10^3$ M$^{-1}$ to $10^6$ M$^{-1}$, and/or association constants ranging from $10^4$ M$^{-1}$ to $10^6$ M$^{-1}$. A molecule can bind to a plurality of peptides in the array with a dissociation constant of about 1 fM, about 2 fM, about 3 fM, about 4 fM, about 5 fM, about 6 fM, about 7 fM, about 8 fM, about 9 fM, about 10 fM, about 20 fM, about 30 fM, about 40 fM, about 50 fM, about 60 fM, about 70 fM, about 80 fM, about 90 fM, about 100 fM, about 200 fM, about 300 fM, about 400 fM, about 500 fM, about 600 fM, about 700 fM, about 800 fM, about 900 fM, about 1 picomolar (pM), about 2 pM, about 3 pM, about 4 pM, about 5 pM, about 6 pM, about 7 pM, about 8 pM, about 9 pM, about 10 pM, about 20 pM, about 30 pM, about 40 pM, about 50 pM, about 60 pM, about 7 pM, about 80 pM, about 90 pM, about 100 pM, about 200 pM, about 300 pM, about 400 pM, about 500 pM, about 600 pM, about 700 pM, about 800 pM, about 900 pM, about 1 nanomolar (nM), about 2 nM, about 3 nM, about 4 nM, about 5 nM, about 6 nM, about 7 nM, about 8 nM, about 9 nM, about 10 nM, about 20 nM, about 30 nM, about 40 nM, about 50 nm, about 60 nM, about 70 nM, about 80 nM, about 90 nM, about 100 nM, about 200 nM, about 300 nM, about 400 nM, about 500 nM, about 600 nM, about 700 nM, about 800 nM, about 900 nM, about 1 μM, about 2 μM, about 3 μM, about 4 μM, about 5 μM, about 6 μM, about 7 μM, about 8 μM, about 9 μM, about 10 μM, about 20 μM, about 30 μM, about 40 μM, about 50 μM, about 60 μM, about 70 μM, about 80 μM, about 90 μM, or about 100 μM.

A molecule can bind to a plurality of peptides in the array with a dissociation constant of at least 1 fM, at least 2 fM, at least 3 fM, at least 4 fM, at least 5 fM, at least 6 fM, at least 7 fM, at least 8 fM, at least 9 fM, at least 10 fM, at least 20 fM, at least 30 fM, at least 40 fM, at least 50 fM, at least 60 fM, at least 70 fM, at least 80 fM, at least 90 fM, at least 100 fM, at least 200 fM, at least 300 fM, at least 400 fM, at least 500 fM, at least 600 fM, at least 700 fM, at least 800 fM, at least 900 fM, at least 1 picomolar (pM), at least 2 pM, at least 3 pM, at least 4 pM, at least 5 pM, at least 6 pM, at least 7 pM, at least 8 pM, at least 9 pM, at least 10 pM, at least 20 pM, at least 30 pM, at least 40 pM, at least 50 pM, at least 60 pM, at least 7 pM, at least 80 pM, at least 90 pM, at least 100 pM, at least 200 pM, at least 300 pM, at least 400 pM, at least 500 pM, at least 600 pM, at least 700 pM, at least 800 pM, at least 900 pM, at least 1 nanomolar (nM), at least 2 nM, at least 3 nM, at least 4 nM, at least 5 nM, at least 6 nM, at least 7 nM, at least 8 nM, at least 9 nM, at least 10 nM, at least 20 nM, at least 30 nM, at least 40 nM, at least 50 nm, at least 60 nM, at least 70 nM, at least 80 nM, at least 90 nM, at least 100 nM, at least 200 nM, at least 300 nM, at least 400 nM, at least 500 nM, at least 600 nM, at least 700 nM, at least 800 nM, at least 900 nM, at least 1 μM, at least 2 μM, at least 3 μM, at least 4 μM, at least 5 μM, at least 6 μM, at least 7 μM, at least 8 μM, at least 9 μM, at least 10 μM, at least 20 μM, at least 30 μM, at least 40 μM, at least 50 μM, at least 60 μM, at least 70 μM, at least 80 μM, at least 90 μM, or about 100 μM.

A dynamic range of binding of an antibody from a biological sample to a peptide microarray can be described as the ratio between the largest and smallest value of a detected signal of binding. A signal of binding can be, for example, a fluorescent signal detected with a secondary antibody. Traditional assays are limited by pre-determined and narrow dynamic ranges of binding. The methods and arrays described herein can detected a broad dynamic range of antibody binding to the peptides in the array of the invention. In some cases, a broad dynamic range of antibody binding can be detected on a logarithmic scale. In some cases, the methods and arrays of the invention allow the detection of a pattern of binding of a plurality of antibodies to an array using up to 2 logs of dynamic range, up to 3 logs of dynamic range, up to 4 logs of dynamic range or up to 5 logs of dynamic range.

The composition of molecules in an array can determine an avidity of binding of a molecule to an array. A plurality of different molecules can be present in an array used in the prevention, treatment, diagnosis or monitoring of a health condition. Non-limiting examples of biomolecules include amino acids, peptides, peptide-mimetics, proteins, recombinant proteins antibodies (monoclonal or polyclonal), antibody fragments, antigens, epitopes, carbohydrates, lipids, fatty acids, enzymes, natural products, nucleic acids (including DNA, RNA, nucleosides, nucleotides, structure analogs or combinations thereof), nutrients, receptors, and vitamins. In some cases, a molecule in an array is a mimotope, a molecule that mimics the structure of an epitope and is able to bind an epitope-elicited antibody. In some cases, a molecule in the array is a paratope or a paratope mimetic, comprising a site in the variable region of an antibody (or T cell receptor) that binds to an epitope an antigen. In some cases, an array of the invention is a peptide array comprising random peptide sequences.

An intra-amino acid distance in a peptide array is the distance between each peptide in a peptide microarray. An intra-amino acid distance can contribute to an off-target binding and/or to an avidity of binding of a molecule to an array. An intra-amino acid difference can be about 0.5 nm, about 1 nm, about 1 nm, 1.1 nm, about 1.2 nm, about 1.3 nm, about 1.4 nm, about 1.5 nm, about 1.6 nm, about 1.7 nm, about 1.8 nm, about 1.9 nm, about 2 nm, about 2.1 nm, about 2.2 nm, about 2.3 nm, about 2.4 nm, about 2.5 nm, about 2.6 nm, about 2.7 nm, about 2.8 nm, about 2.9 nm, about 3 nm, about 3.1 nm, about 3.2 nm, about 3.3 nm, about 3.4 nm, about 3.5 nm, about 3.6 nm, about 3.7 nm, about 3.8 nm, about 3.9 nm, about 4 nm, about 4.1 nm, about 4.2 nm, about 4.3 nm, about 4.4 nm, about 4.5 nm, about 4.6 nm, about 4.7 nm, about 4.8 nm, about 4.9 nm, about 5 nm, about 5.1 nm, about 5.2 nm, about 5.3 nm, about 5.4 nm, about 5.5 nm, about 5.6 nm, about 5.7 nm, about 5.8 nm, about 5.9 nm, and/or about 6 nm. In some cases, the intra-amino acid distance is less than 6 nanometers (nm).

An intra-amino acid difference can be at least 0.5 nm, at least 1 nm, at least 1 nm, at least 1.1 nm, at least 1.2 nm, at least 1.3 nm, at least 1.4 nm, at least 1.5 nm, at least 1.6 nm, at least 1.7 nm, at least 1.8 nm, at least 1.9 nm, at least 2 nm, at least 2.1 nm, at least 2.2 nm, at least 2.3 nm, at least 2.4 nm, at least 2.5 nm, at least 2.6 nm, at least 2.7 nm, at least 2.8 nm, at least 2.9 nm, at least 3 nm, at least 3.1 nm, at least 3.2 nm, at least 3.3 nm, at least 3.4 nm, at least 3.5 nm, at least 3.6 nm, at least 3.7 nm, at least 3.8 nm, at least 3.9 nm, at least 4 nm, at least 4.1 nm, at least 4.2 nm, at least 4.3 nm, at least 4.4 nm, at least 4.5 nm, at least 4.6 nm, at least 4.7 nm, at least 4.8 nm, at least 4.9 nm, at least 5 nm, at least 5.1 nm, at least 5.2 nm, at least 5.3 nm, at least 5.4 nm, at least 5.5 nm, at least 5.6 nm, at least 5.7 nm, at least 5.8 nm, or at least 5.9 nm.

An intra-amino acid difference can be not more than 0.5 nm, not more than 1 nm, not more than 1 nm, not more than 1.1 nm, not more than 1.2 nm, not more than 1.3 nm, not more than 1.4 nm, not more than 1.5 nm, not more than 1.6 nm, not more than 1.7 nm, not more than 1.8 nm, not more than 1.9 nm, not more than 2 nm, not more than 2.1 nm, not more than 2.2 nm, not more than 2.3 nm, not more than 2.4 nm, not more than 2.5 nm, not more than 2.6 nm, not more than 2.7 nm, not more than 2.8 nm, not more than 2.9 nm, not more than 3 nm, not more than 3.1 nm, not more than 3.2 nm, not more than 3.3 nm, not more than 3.4 nm, not more than 3.5 nm, not more than 3.6 nm, not more than 3.7 nm, not more than 3.8 nm, not more than 3.9 nm, not more than 4 nm, not more than 4.1 nm, not more than 4.2 nm, not more than 4.3 nm, not more than 4.4 nm, not more than 4.5 nm, not more than 4.6 nm, not more than 4.7 nm, not more than 4.8 nm, not more than 4.9 nm, not more than 5 nm, not more than 5.1 nm, not more than 5.2 nm, not more than 5.3 nm, not more than 5.4 nm, not more than 5.5 nm, not more than 5.6 nm, not more than 5.7 nm, not more than 5.8 nm, not more than 5.9 nm, and/or not more than 6 nm. In some cases, the intra-amino acid distance is not more than 6 nanometers (nm).

An intra-amino acid difference can range from 0.5 nm to 1 nm, 0.5 nm to 2 nm, 0.5 nm to 3 nm, 0.5 nm to 3 nm, 0.5 nm to 4 nm, 0.5 nm to 5 nm, 0.5 nm to 6 nm, 1 nm to 2 nm, 1 nm to 3 nm, 1 nm to 4 nm, 1 nm to 5 nm, 1 nm to 6 nm, 2 nm to 3 nm, 2 nm to 4 nm, 2 nm to 5 nm, 2 nm to 6 nm, 3 nm to 4 nm, 3 nm to 5 nm, 3 nm to 6 nm, 4 nm to 5 nm, 4 nm to 6 nm, and/or 5 nm to 6 nm.

A peptide array can comprise a plurality of different peptides patterns a surface. A peptide array can comprise, for example, a single, a duplicate, a triplicate, a quadruplicate, a quintuplicate, a sextuplicate, a septuplicate, an octuplicate, a nonuplicate, and/or a decuplicate replicate of the different pluralities of peptides and/or molecules. In some cases, pluralities of different peptides are spotted in replica on the surface of a peptide array. A peptide array can, for example, comprise a plurality of peptides homogenously distributed on the array. A peptide array can, for example, comprise a plurality of peptides heterogeneously distributed on the array.

A peptide can be "spotted" in a peptide array. A peptide spot can have various geometric shapes, for example, a peptide spot can be round, square, rectangular, and/or triangular. A peptide spot can have a plurality of diameters. Non-limiting examples of peptide spot diameters are about 3 $\mu$m to about 8 $\mu$m, about 3 to about 10 mm, about 5 to about 10 mm, about 10 $\mu$m to about 20 $\mu$m, about 30 $\mu$m, about 40 $\mu$m, about 50 $\mu$m, about 60 $\mu$m, about 70 $\mu$m, about 80 $\mu$m, about 90 $\mu$m, about 100 $\mu$m, about 110 $\mu$m, about 120 $\mu$m, about 130 $\mu$m, about 140 $\mu$m, about 150 $\mu$m, about 160 $\mu$m, about 170 $\mu$m, about 180 $\mu$m, about 190 $\mu$m, about 200 $\mu$m, about 210 $\mu$m, about 220 $\mu$m, about 230 $\mu$m, about 240 $\mu$m, and/or about 250 $\mu$m.

A peptide array can comprise a number of different peptides. In some cases, a peptide array comprises about 10 peptides, about 50 peptides, about 100 peptides, about 200 peptides, about 300 peptides, about 400 peptides, about 500 peptides, about 750 peptides, about 1000 peptides, about 1250 peptides, about 1500 peptides, about 1750 peptides, about 2,000 peptides; about 2,250 peptides; about 2,500 peptides; about 2,750 peptides; about 3,000 peptides; about 3,250 peptides; about 3,500 peptides; about 3,750 peptides; about 4,000 peptides; about 4,250 peptides; about 4,500 peptides; about 4,750 peptides; about 5,000 peptides; about 5,250 peptides; about 5,500 peptides; about 5,750 peptides; about 6,000 peptides; about 6,250 peptides; about 6,500 peptides; about 7,500 peptides; about 7,725 peptides 8,000 peptides; about 8,250 peptides; about 8,500 peptides; about 8,750 peptides; about 9,000 peptides; about 9,250 peptides; about 10,000 peptides; about 10,250 peptides; about 10,500 peptides; about 10,750 peptides; about 11,000 peptides; about 11,250 peptides; about 11,500 peptides; about 11,750 peptides; about 12,000 peptides; about 12,250 peptides; about 12,500 peptides; about 12,750 peptides; about 13,000 peptides; about 13,250 peptides; about 13,500 peptides; about 13,750 peptides; about 14,000 peptides; about 14,250 peptides; about 14,500 peptides; about 14,750 peptides; about 15,000 peptides; about 15,250 peptides; about 15,500 peptides; about 15,750 peptides; about 16,000 peptides; about 16,250 peptides; about 16,500 peptides; about 16,750 peptides; about 17,000 peptides; about 17,250 peptides; about 17,500 peptides; about 17,750 peptides; about 18,000 peptides; about 18,250 peptides; about 18,500 peptides; about 18,750 peptides; about 19,000 peptides; about 19,250 peptides; about 19,500 peptides; about 19,750 peptides; about 20,000 peptides; about 20,250 peptides; about 20,500 peptides; about 20,750 peptides; about 21,000 peptides; about 21,250 peptides; about 21,500 peptides; about 21,750 peptides; about 22,000 peptides; about 22,250 peptides; about 22,500 peptides; about 22,750 peptides; about 23,000 peptides; about 23,250 peptides; about 23,500 peptides; about 23,750 peptides; about 24,000 peptides; about 24,250 peptides; about 24,500 peptides; about 24,750 peptides; about 25,000 peptides; about 25,250 peptides; about 25,500 peptides; about 25,750 peptides; and/or about 30,000 peptides.

In some cases, a peptide array used in a method of health monitoring, a method of treatment, a method of diagnosis, and a method for preventing a condition comprises more than 30,000 peptides. In some cases, a peptide array used in a method of health monitoring comprises about 330,000 peptides. In some cases the array comprise about 30,000 peptides; about 35,000 peptides; about 40,000 peptides; about 45,000 peptides; about 50,000 peptides; about 55,000 peptides; about 60,000 peptides; about 65,000 peptides; about 70,000 peptides; about 75,000 peptides; about 80,000 peptides; about 85,000 peptides; about 90,000 peptides; about 95,000 peptides; about 100,000 peptides; about 105,000 peptides; about 110,000 peptides; about 115,000 peptides; about 120,000 peptides; about 125,000 peptides; about 130,000 peptides; about 135,000 peptides; about 140,000 peptides; about 145,000 peptides; about 150,000 peptides; about 155,000 peptides; about 160,000 peptides; about 165,000 peptides; about 170,000 peptides; about 175,000 peptides; about 180,000 peptides; about 185,000 peptides; about 190,000 peptides; about 195,000 peptides; about 200,000 peptides; about 210,000 peptides; about 215,000 peptides; about 220,000 peptides; about 225,000 peptides; about 230,000 peptides; about 240,000 peptides; about 245,000 peptides; about 250,000 peptides; about 255,000 peptides; about 260,000 peptides; about 265,000 peptides; about 270,000 peptides; about 275,000 peptides; about 280,000 peptides; about 285,000 peptides; about 290,000 peptides; about 295,000 peptides; about 300,000 peptides; about 305,000 peptides; about 310,000 peptides; about 315,000peptides; about 320,000 peptides; about 325,000 peptides; about 330,000 peptides; about 335,000 peptides; about 340,000 peptides; about 345,000 peptides; and/or about 350,000 peptides. In some cases, a peptide array used in a method of health monitoring comprises more than 330,000 peptides.

A peptide array can comprise a number of different peptides. In some cases, a peptide array comprises at least 2,000 peptides; at least 3,000 peptides; at least 4,000 peptides; at least 5,000 peptides; at least 6,000 peptides; at least 7,000 peptides; at least 8,000 peptides; at least 9,000 peptides; at least 10,000 peptides; at least 11,000 peptides; at least 12,000 peptides; at least 13,000 peptides; at least 14,000 peptides; at least 15,000 peptides; at least 16,000 peptides; at least 17,000 peptides; at least 18,000 peptides; at least 19,000 peptides; at least 20,000 peptides; at least 21,000 peptides; at least 22,000 peptides; at least 23,000 peptides; at least 24,000 peptides; at least 25,000 peptides; at least 30,000 peptides; at least 40,000 peptides; at least 50,000 peptides; at least 60,000 peptides; at least 70,000 peptides; at least 80,000 peptides; at least 90,000 peptides; at least 100,000 peptides; at least 110,000 peptides; at least 120,000 peptides; at least 130,000 peptides; at least 140,000 peptides; at least 150,000 peptides; at least 160,000 peptides; at least about 170,000 at least 180,000 peptides; at least 190,000 peptides; at least 200,000 peptides; at least 210,000 peptides; at least 220,000 peptides; at least 230,000 peptides; at least 240,000 peptides; at least 250,000 peptides; at least 260,000 peptides; at least 270,000 peptides; at least 280,000 peptides; at least 290,000 peptides; at least 300,000 peptides; at least 310,000 peptides; at least 320,000 peptides; at least 330,000 peptides; at least 340,000 peptides; at least 350,000 peptides. In some cases, a peptide array used in a method of health monitoring comprises at least 330,000 peptides.

A peptide can be physically tethered to a peptide array by a linker molecule. The N- or the C-terminus of the peptide can be attached to a linker molecule. A linker molecule can be, for example, a functional plurality or molecule present on the surface of an array, such as an imide functional group, an amine functional group, a hydroxyl functional group, a carboxyl functional group, an aldehyde functional group, and/or a sulfhydryl functional group. A linker molecule can be, for example, a polymer. In some cases the linker is maleimide. In some cases the linker is a glycine-serine-cysteine (GSC) or glycine-glycine-cysteine (GGC) linker. In some cases, the linker consists of a polypeptide of various lengths or compositions. In some cases the linker is polyethylene glycol of different lengths. In yet other cases, the linker is hydroxymethyl benzoic acid, 4-hydroxy-2-methoxy benzaldehyde, 4-sulfamoyl benzoic acid, or other suitable for attaching a peptide to the solid substrate.

A surface of a peptide array can comprise a plurality of different materials. A surface of a peptide array can be, for example, glass. Non-limiting examples of materials that can comprise a surface of a peptide array include glass, functionalized glass, silicon, germanium, gallium arsenide, gallium phosphide, silicon dioxide, sodium oxide, silicon nitrade, nitrocellulose, nylon, polytetraflouroethylene, polyvinylidendiflouride, polystyrene, polycarbonate, methacrylates, or combinations thereof.

A surface of a peptide array can be flat, concave, or convex. A surface of a peptide array can be homogeneous and a surface of an array can be heterogeneous. In some cases, the surface of a peptide array is flat.

A surface of a peptide array can be coated with a coating. A coating can, for example, improve the adhesion capacity of an array described herein. A coating can, for example, reduce background adhesion of a biological sample to an array described herein. In some cases, a peptide array described herein comprises a glass slide with an aminosilane-coating.

A peptide array can have a plurality of dimensions. A peptide array can be a peptide microarray.

Manufacturing Arrays.

Also disclosed herein are methods to facilitate patterning techniques for manufacturing complex bioarrays, such as the peptide arrays above. Existing methods have shown the feasibilty of using lithography or other patterning techniques to make a library of heteropolymers with defined positions on a surface. The methods have been applied extensively to DNA and peptide arrays. The simplest approach is to make the library of heteropolymers in layers. Consider a heteropolymer of length N consisting of a sequence of M monomers. In general, there are M steps of patterning per layer, one step for each of the monomers. For a sequence length of N, there would be N layers of patterning. The total number of patterning steps thus is NxM.

Another aspect of pattering is that it is a binary event. In other words, any region of the surface in each paterning step is either "exposed" or "unexposed," where exposure is to whatever radiation, chemical, effector or force being used in the patterning. Patterning an assay in this way involves projecting a sequence space of $M^N$ possibilities onto a binary space of $2^R$ possibilities, where R is the total number of patterning steps. In principle, the minimum value of R is given by setting the two expressions equal, and solving the quation leads to:

$$R = N \frac{\ln M}{\ln 2}$$

This represents the theoretical minimum number of patterning steps if one wishes to be able to represent any heteropolymer in $M^N$ space by a series of patterning steps in $2^R$ space. One can compare the number of patterning steps in a layer by layer algorithm (M x N) to the minimum number given by R above, shown in the following **TABLE 1**.

| TABLE 1, part 1 | | | | | | | | | | | | |
|---|---|---|---|---|---|---|---|---|---|---|---|---|
| N\M | **10** | | **11** | | **12** | | **13** | | **14** | | **15** | |
| | MxN | R | MxN | R | MxN | R | MxN | R | MxN | R | MxN | R |
| **8** | 80 | 27 | 88 | 28 | 96 | 29 | 104 | 30 | 112 | 30 | 120 | 31 |
| **9** | 90 | 30 | 99 | 31 | 108 | 32 | 117 | 33 | 126 | 34 | 135 | 35 |
| **10** | 100 | 33 | 110 | 35 | 120 | 36 | 130 | 37 | 140 | 38 | 150 | 39 |
| **11** | 110 | 37 | 121 | 38 | 132 | 39 | 143 | 41 | 154 | 42 | 165 | 43 |
| **12** | 120 | 40 | 132 | 42 | 144 | 43 | 156 | 44 | 168 | 46 | 180 | 47 |
| **13** | 130 | 43 | 143 | 45 | 156 | 47 | 169 | 48 | 182 | 49 | 195 | 51 |
| **14** | 140 | 47 | 154 | 48 | 168 | 50 | 182 | 52 | 196 | 53 | 210 | 55 |
| **15** | 150 | 50 | 165 | 52 | 180 | 54 | 195 | 56 | 210 | 57 | 225 | 59 |
| **16** | 160 | 53 | 176 | 55 | 192 | 57 | 208 | 59 | 224 | 61 | 240 | 63 |
| **17** | 170 | 56 | 187 | 59 | 204 | 61 | 221 | 63 | 238 | 65 | 255 | 66 |
| **18** | 180 | 60 | 198 | 62 | 216 | 65 | 234 | 67 | 252 | 69 | 270 | 70 |
| **19** | 190 | 63 | 209 | 66 | 228 | 68 | 247 | 70 | 266 | 72 | 285 | 74 |
| **20** | 200 | 66 | 220 | 69 | 240 | 72 | 260 | 74 | 280 | 76 | 300 | 78 |

| TABLE 1, part 2 | | | | | | | | | | |
|---|---|---|---|---|---|---|---|---|---|---|
| N\M | **16** | | **17** | | **18** | | **19** | | **20** | |
| | MxN | R | MxN | R | MxN | R | MxN | R | MxN | R |
| **8** | 128 | 32 | 136 | 33 | 144 | 33 | 152 | 34 | 160 | 35 |
| **9** | 144 | 36 | 153 | 37 | 162 | 38 | 171 | 38 | 180 | 39 |
| **10** | 160 | 40 | 170 | 41 | 180 | 42 | 190 | 42 | 200 | 43 |
| **11** | 176 | 44 | 187 | 45 | 198 | 46 | 209 | 47 | 220 | 48 |
| **12** | 192 | 48 | 204 | 49 | 216 | 50 | 228 | 51 | 240 | 52 |
| **13** | 208 | 52 | 221 | 53 | 234 | 54 | 247 | 55 | 260 | 56 |
| **14** | 224 | 56 | 238 | 57 | 252 | 58 | 266 | 59 | 280 | 61 |

| | | | | | | | | | | |
|---|---|---|---|---|---|---|---|---|---|---|
| **15** | 240 | 60 | 255 | 61 | 270 | 63 | 285 | 64 | 300 | 65 |
| **16** | 256 | 64 | 272 | 65 | 288 | 67 | 304 | 68 | 320 | 69 |
| **17** | 272 | 68 | 289 | 69 | 306 | 71 | 323 | 72 | 340 | 73 |
| **18** | 288 | 72 | 306 | 74 | 324 | 75 | 342 | 76 | 360 | 78 |
| **19** | 304 | 76 | 323 | 78 | 342 | 79 | 361 | 81 | 380 | 82 |
| **20** | 320 | 80 | 340 | 82 | 360 | 83 | 380 | 85 | 400 | 86 |

The number of steps involved in the layer by layer approach is very large compared to the theoretical minimum from binary representation. The problem is that under normal photolithography processing, each amino acid is added separately and thus there is no way to directly imprint a binary code that would sort out the different amino acids using this approach. However, it is also unnecessary to add amino acids to only one layer at a time, leading to a significant change in the number of cycles needed.

The new patterning process of this invention is described in the following way. In an example case, an array of heteropolymers formed by using 10 different kinds of monomers is used, and the percentages of monomers for forming the heteropolymers are equal, *i.e.,* 10% for each monomer. The first patterning step adds the monomer A; namely 10% of the heteropolymers will have an A in the first layer. The second step considers monomer B. In this case, 10% of the monomers assigned to the first layer will have B; but in addition, 10% of the currently available second layer (i.e., the 10% that received A in the first layer) will also be ready to receive a B monomer. Thus B will actually be coupled to 11% of the total amine sites. In the third step where monomer C is added, there are 10% of heteropolymers receiving C as the first layer, and then 10% of the sites in the second layer and 10% of the sites now open for the third layer that already have both A and B added. This process continues and eventually stabilizes at a level where each monomer placed on the surface represents close to 20% of the available amines, even though there are only 10% with any particular monomer added per layer. This results in a nearly two fold increase in the average length of polymers made for a particular number of steps, compared to a layer by layer synthesis.

The process can be described in an algorithmic form. In short, the process is to recursively add the next monomer in series to every layer available that the sequences dictate. The algorithm has the greatest effect when one cycles in the monomers in a particular order over and over again. In general, the algorithm works in the following way:

- Select a set of monomers for making the heteropolymers.
- Assign a fraction of the addition sites (e.g., amines in peptide synthesis) covered per layer (per residue) to each monomer

    ◦ In one case, choose a fraction to be 1/(# of monomers).
    ◦ In another case, the monomers have different fractions whose sum is 100%.
    ◦ In another case when generating pseudo random peptide sequences, the fractions associated with the monomers may equal to a value greater than 100%.

- Create a set of desired heteropolymer sequences, which includes the use of pseudo-random or random sequences.
- Use patterned chemical methods: add one monomer at a time to all positions that will properly extend the peptides according to the desired sequences, irrespective of which residue position in the heteropolymer is available for addition.

    ◦ In one case, the step comprises cycling through the monomers in a predetermined order. This gives the longest peptides for the smallest number of patterning steps.

The order or addition in each cycle may also be changed or randomized completely, but the random ordered patterning will increase the number of patterning steps required to achieve a particular average length.

Given the fraction assignment above, even though any particular layer has only the fraction of a monomer, the actual fraction that is added in a patterning step using this algorithm is considerably higher. The quantity of added monomers in a patterning step can be evaluated as follows. Let $f_j$ denote the fraction of a layer that a particular monomer is added to. Summing up all the fractions of monomers added in all layers leads to

$$\left( f_i \sum_{j=i-Z}^{i-1} f_j \right) + f_i$$

where the subscript i designates the current patterning step number; Z is the number of different monomers that have been added since the last time that the current monomer was added; the sum is thus over the fractions per layer associated with all the monomers that have been added since the last time the current monomer was added.

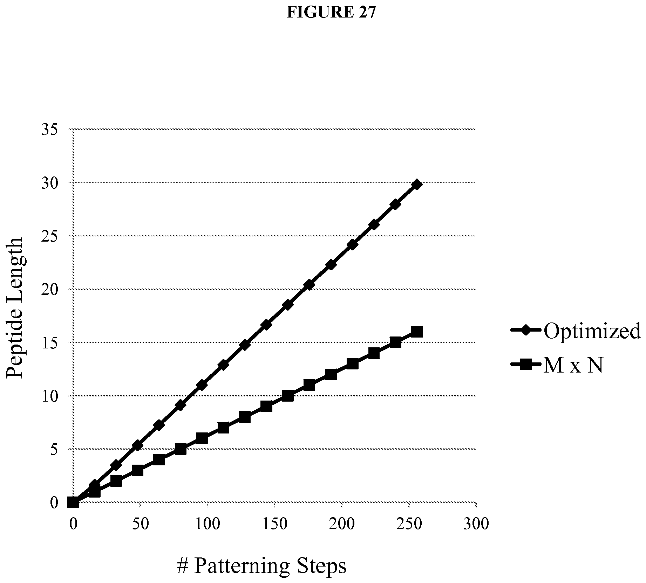

An example case is shown herein. In this case 16 amino acids are being used to build 10,000 predefined peptide sequences. **FIGURE 27** shows the average length of peptide synthesized as a function of the number of patterning steps. The Y axis is the average peptide length and the X axis is the number of patterning cycles. Note that for nearly any number of patterning cycles, the optimized model improves manufacturing efficiency by almost a factor of two.

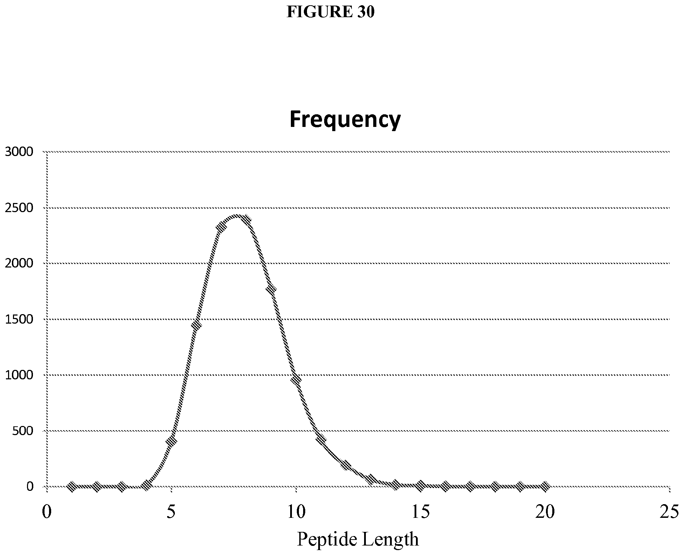

The other approach to generating pseudo random peptides using this invention is to generate a very large number of peptide sequences computationally using this method, but then only include the longest ones in the production of the array. This approach results in a bias towards sequences that have an order similar to the order that amino acids are added in (though generally not sequential). The resulting sequences still cover a large amount of space, and the degree of randomness depends on what fraction of the distribution the practitioner selects. With reference to **FIGURE 30,** an case of a distribution resulting from 70 steps of the optimized algorithm using 16 different amino acids is described below. The top 5% of these sequences average about 12 residues in length and could be selected for actual synthesis in an array and the other sequences discarded. If one drops the number of patterning steps down to 60, one could get about the same average peptide length by selecting the longest 0.5% of peptides. Once again, the smaller the number of patterning steps used, the more sequence bias is imposed in the library of peptides, but to the extent that some bias can be tolerated, the number of patterning steps can be greatly reduced.

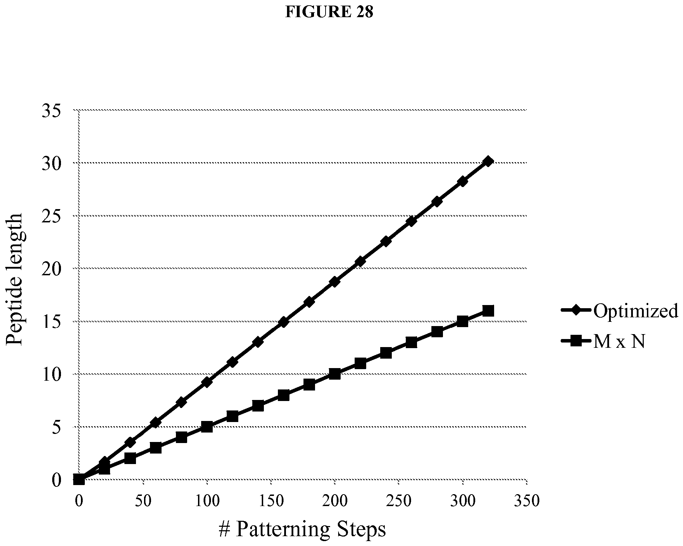

In some cases, using this invention could create an array of peptides of defined sequences that has an average length of 12 residues using 16 different acids in just over 100 patterning steps. However, when an case attempts to make a particular set of heteropolymers with a particular set of sequences, we will not get all the way to the end of each sequence until essentially M x N patterning steps. In the cases where a fraction of the sequences end one or two monomers short of what is predefined, we can make the sequences in many fewer steps than M x N. **FIGURE 28** shows the results of using all 20 amino acids for the standard layer by layer approach vs. the optimized algorithm.

Another case considers generating overlapping peptide sequences that between them cover an entire proteome, such as the human proteome. One might use such an array for epitope discovery or to identify binding sites of proteins or small molecules. Linear epitopes could be identified using an array of peptides 12-15 residues long with a 3-5 amino acid overlap, for example. It would take a couple million peptides on a surface to generate such an array. This could be accomplished by making an array with an average length of 13 residues which would require approximately 140 steps using the optimized algorithm vs. 260 steps using the layer by layer approach.

The arrays disclosed herein can be used in conjunction with immunosignaturing as described above. Variable lengths of peptides on the array are acceptable, and sometimes desirable, when used in conjunction with immunosignaturing. Peptides with an average of 12 residues and using 16 different amino acids have been shown to work well for immunosignaturing and a random array of such peptides could be made in just over 100 patterning steps, as shown in **FIGURE 27**. In contrast, using a layer by layer synthesis will take 192 steps.

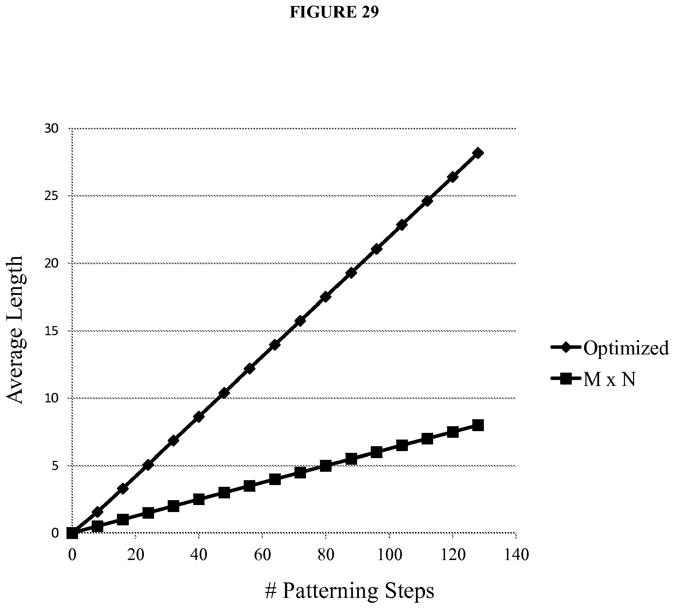

Immunosignaturing can also be accomplished efficiently with peptides that are not completely random. There are two ways to use this algorithm to create pseudo random peptides in fewer steps than purely random ones. Consider the example of an array using 16 types of monomers, say amino acids. We can simply run the cycles of amino acids as thought there were only 8 amino acids instead of 16, but then alternate between the sets of 8 being used. This way of adding monomers means that the initial few amino acids in the series will be biased towards the first set of 8. Eventually, the bias will damp out, though not completely disappear. **FIGURE 29** shows the results of this case; we can achieve an average of 12 residues in length after less than 60 steps.

Detection.

Binding interactions between components of a sample and an array can be detected in a variety of formats. In some formats, components of the samples are labeled. The label can be a radioisotope or dye among others. The label can be supplied either by administering the label to a patient before obtaining a sample or by linking the label to the sample or selective component(s) thereof.

Binding interactions can also be detected using a secondary detection reagent, such as an antibody. For example, binding of antibodies in a sample to an array can be detected using a secondary antibody specific for the isotype of an antibody (e.g., IgG (including any of the subtypes, such as IgG1, IgG2, IgG3 and IgG4), IgA, IgM). The secondary antibody is usually labeled and can bind to all antibodies in the sample being analyzed of a particular isotype. Different secondary antibodies can be used having different isotype specificities. Although there is often substantial overlap in compounds bound by antibodies of different isotypes in the same sample, there are also differences in profile.

Binding interactions can also be detected using label-free methods, such as surface plasmon resonance (SPR) and mass spectrometry. SPR can provide a measure of dissociation constants, and dissociation rates. The A-100 Biocore/GE instrument, for example, is suitable for this type of analysis. FLEXchips can be used to analyze up to 400 binding reactions on the same support.

Optionally, binding interactions between component(s) of a sample and the array can be detected in a competition format. A difference in the binding profile of an array to a sample in the presence versus absence of a competitive inhibitor of binding can be useful in characterizing the sample. The competitive inhibitor can be for example, a known protein associated with a disease condition, such as pathogen or antibody to a pathogen. A reduction in binding of member(s) of the array to a sample in the presence of such a competitor provides an indication that the pathogen is present. The stringency can be adjusted by varying the salts, ionic strength, organic solvent content and temperature at which library members are contacted with the target.

An antibody based method of detection, such as an enzyme-linked immunosorbent assay (ELISA) method can be used to detect a pattern of binding to an array of the invention. For example, a secondary antibody that detects a particular isotype of an immunoglobulin, for example the IgM isotype, can be used to detect a binding pattern of a plurality of IgM antibodies from a complex biological sample of a subject to an array. The secondary antibody can be, for example conjugated to a detectable label, such as a fluorescent moiety or a radioactive label.

The invention provides arrays and methods for the detection of an off-target binding of a plurality of different antibodies to an array of the invention. A plurality of antibodies in a complex biological sample are capable of off-target binding of a plurality of peptides in a peptide microarray. In some cases, detecting an off-target binding of at least one antibody to a plurality of peptides in the peptide array can form an immunosignature. A plurality of classes or isotypes of antibodies can provide an off-target pattern of binding to an array. An antibody, or immunoglobulin, can be an IgA, IgD, IgE, IgG, and/or an IgM antibody.

A pattern of binding of at least one IgM antibody from a complex biological sample to a peptide array can form an immunosignature. An IgM antibody can form polymers where multiple immunoglobulins are covalently linked together with disulfide bonds. An IgM polymer can be a pentamer. The polymeric nature of an antibody with the IgM isotype can increase off-target binding of a sample to an array. A polymeric nature of an antibody can increase an avidity of binding of a sample to an array. For example, a pattern of binding of antibodies of a polymeric IgM isotype antibodies to a peptide microarray can form a unique pentameric driven immunosignature.

An IgA antibody can be an IgA1 or an IgA2 antibody. An antibody of the IgA isotype can form a dimer. An IgG antibody can be an IgG1, IgG2, IgG3, or an IgG4 antibody. An antibody of the IgG isotype can exist as a monomer. An IgD and/or an IgE antibody can form a monomer. In some cases, the invention can detect an off-target binding of at least one IgM antibody from a complex biological sample of a subject to a peptide array.

Monitoring a Subject through the Lifespan of the Subject.

The methods, devices, kits, arrays, and systems described herein can be used to monitor a subject through the lifespan of the subject. A subject's lifespan can refer to what has happened to the subject since birth. The monitoring of the health of a subject with the methods, arrays, kits, and systems of the invention can be incorporated in a medical record or Electronic Medical Records of a subject (EMRs) of a subject.

Electronic Medical Records (EMRs) can relate to records obtained and stored by a subject's doctor, clinician, insurance company, hospital and/or other facilities where a subject is a patient. In some cases, the doctor can include a medical doctor, a dentist, an optometrist, a therapist, a chiropractor, and anyone who provides healthcare services to the subject. Electronic medical records (EMR) can comprise, for example, CAT scans, MRIs, ultrasounds, blood glucose levels, diagnoses, allergies, lab test results, EKGs, medications, daily charting, medication administration, physical assessments, admission nursing notes, nursing care plans, referrals, present and past symptoms, medical history, life style, physical examination results, tests, procedures, treatments, medications, discharges, history, diaries, problems, findings, immunizations, admission notes, on-service notes, progress notes, preoperative notes, operative notes, postoperative notes, procedure notes, delivery notes, postpartum notes, and discharge notes.

Treatments and Conditions.

The array and methods described herein can be used, for example, to diagnose, monitor, characterize, and guide treatment of a plurality of different conditions of a subject. A subject can be a human, a guinea pig, a dog, a cat, a horse, a mouse, a rabbit, and various other animals. A subject can be of any age, for example, a subject can be an infant, a toddler, a child, a pre-adolescent, an adolescent, an adult, or an elderly individual.

A condition of a subject can correspond to a disease or a healthy condition. In some cases, a condition of a subject is a healthy condition, and a method of the invention monitors the healthy condition. In some cases, a condition of a subject is a disease condition, and a method described herein is used to diagnose/monitor a state and/or the progression of the condition. A method described herein can also be used in the prevention of a condition. In some cases, a method described herein is used in conjunction with a prophylactic treatment.

The array devices and methods disclosed herein importantly detect and monitor a variety of diseases and/or conditions simultaneously. For example, the array devices and methods disclosed herein are capable of simultaneously detecting inflammatory conditions, cancer diseases and pathogenic infection on the same array. Accordingly, only one array, *i.e.* one immunosignature assay, is necessary to detect a wide spectra of diseases and conditions. Thus, the monitoring of a subject through its lifespan will provide, with every immunosignature performed, a snapshot through time of the subject's health status. This provides a powerful means of detecting global and specific changes in the subject's health status, and together with the high sensitivity of the immunosignature assay, provides a system capable of detecting at very early stages any change in the individual's health status.

Accordingly, the methods, systems and array devices disclosed herein are capable of detecting, diagnosing, monitoring, preventing and/or treating a disease and/or condition at an early stage of the disease and/or condition. For example, the methods, systems and array devices disclosed herein are capable of detecting, diagnosing and monitoring a disease and/or condition days or weeks before traditional biomarker-based assays. Moreover, only one array, *i.e.,* one immunosignature assay, is needed to detect, diagnose and monitor a side spectra of diseases and conditions, including inflammatory conditions, cancer and pathogenic infections.

An array and a method described herein can also be used to, for example, diagnose, monitor, prevent and/or treat a cancer. Non-limiting examples of cancers that can be diagnosed, monitored, prevented, and/or treated with an array and a method of the invention can include: acute lymphoblastic leukemia, acute myeloid leukemia, adrenocortical carcinoma, AIDS-related cancers, AIDS-related lymphoma, anal cancer, appendix cancer, astrocytomas, basal cell carcinoma, bile duct cancer, bladder cancer, bone cancers, brain tumors, such as cerebellar astrocytoma, cerebral astrocytoma/malignant glioma, ependymoma, medulloblastoma, supratentorial primitive neuroectodermal tumors, visual pathway and hypothalamic glioma, breast cancer, bronchial adenomas, Burkitt lymphoma, carcinoma of unknown primary origin, central nervous system lymphoma, cerebellar astrocytoma, cervical cancer, childhood cancers, chronic lymphocytic leukemia, chronic myelogenous leukemia, chronic myeloproliferative disorders, colon cancer, cutaneous T-cell lymphoma, desmoplastic small round cell tumor, endometrial cancer, ependymoma, esophageal cancer, Ewing's sarcoma, germ cell tumors, gallbladder cancer, gastric cancer, gastrointestinal carcinoid tumor, gastrointestinal stromal tumor, gliomas, hairy cell leukemia, head and neck cancer, heart cancer, hepatocellular (liver) cancer, Hodgkin lymphoma, Hypopharyngeal cancer, intraocular melanoma, islet cell carcinoma, Kaposi sarcoma, kidney cancer, laryngeal cancer, lip and oral cavity cancer, liposarcoma, liver cancer, lung cancers, such as non-small cell and small cell lung cancer, lymphomas, leukemias, macroglobulinemia, malignant fibrous histiocytoma of bone/osteosarcoma, medulloblastoma, melanomas, mesothelioma, metastatic squamous neck cancer with occult primary, mouth cancer, multiple endocrine neoplasia syndrome, myelodysplastic syndromes, myeloid leukemia, nasal cavity and paranasal sinus cancer, nasopharyngeal carcinoma, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer, ovarian germ cell tumor, pancreatic cancer, pancreatic cancer islet cell, paranasal sinus and nasal cavity cancer, parathyroid cancer, penile cancer, pharyngeal cancer, pheochromocytoma, pineal astrocytoma, pineal germinoma, pituitary adenoma, pleuropulmonary blastoma, plasma cell neoplasia, primary central nervous system lymphoma, prostate cancer, rectal cancer, renal cell carcinoma, renal pelvis and ureter transitional cell cancer, retinoblastoma, rhabdomyosarcoma, salivary gland cancer, sarcomas, skin cancers, skin carcinoma merkel cell, small intestine cancer, soft tissue sarcoma, squamous cell carcinoma, stomach cancer, T-cell lymphoma, throat cancer, thymoma, thymic carcinoma, thyroid cancer, trophoblastic tumor (gestational), cancers of unkown primary site, urethral cancer, uterine sarcoma, vaginal cancer, vulvar cancer, Waldenstrom macroglobulinemia, and Wilms tumor.

In some cases, a method of the invention can be used to diagnose, monitor, prevent and/or treat a condition associated with the immune system. Non-limiting examples of disorders associated with the immune system can include: auto-immune disorders, inflammatory diseases, HIV, rheumatoid arthritis, diabetes mellitus type 1, systemic lupus erythematosus, scleroderma, multiple sclerosis, severe combined immunodeficiency (SCID), DiGeorge syndrome, ataxia-telangiectasia, seasonal allergies, perennial allergies, food allergies, anaphylaxis, mastocytosis, allergic rhinitis, atopic dermatitis, Parkinson's, Alzheimer's, hypersplenism, leukocyte adhesion deficiency, X-linked lymphoproliferative disease, X-linked agammaglobulinemia, selective immunoglobulin A deficiency, hyper IgM syndrome, autoimmune lymphoproliferative syndrome, Wiskott-Aldrich syndrome, chronic granulomatous disease, common variable immunodeficiency (CVID), hyperimmunoglobulin E syndrome, and Hashimoto's thyroiditis.

Described herein is a method of preventing a condition, the method comprising: a) providing a complex biological sample from a subject; b) contacting the complex biological sample to a peptide array, wherein the peptide array comprises different peptides capable of off-target binding of at least one antibody in the complex biological sample; c) measuring an off-target binding of the complex biological sample to a plurality of the different peptides to form an immunosignature; d) associating the immunosignature with a condition; and e) receiving a treatment for the condition.

In some cases, a method described herein can be used in conjunction with a prophylactic treatment. Vaccines, for example, can be prophylactic treatments. Non-limiting examples of vaccines that function as prophylactic treatments include polio vaccines, smallpox vaccines, measles vaccines, mumps vaccines, human papillomavirus (HPV) vaccines, and influenza vaccines. In some cases, a method described herein is used to monitor, for example, a subject's response to a prophylactic vaccine.

In some cases described herein a method of providing a treatment, the method comprising: a) receiving a complex biological sample from a subject; b) contacting the complex biological sample to a peptide array, wherein the peptide array comprises different peptides capable of off-target binding of at least one antibody in the biological sample; c) measuring the off-target binding of the antibody to a plurality of the different peptides to form an immunosignature; d) associating the immunosignature with a condition; and e) providing the treatment for the condition.

In some cases, described herein is a method of diagnosis, the method comprising: a) receiving a complex biological sample from a subject; b) contacting the complex biological sample to a peptide array, wherein the peptide array comprises different peptides capable of off-target binding of at least one antibody in the biological sample; c) measuring the off-target binding of the antibody to a group of different peptides in the peptide array to form an immunosignature; and d) diagnosing a condition based on the immunosignature.

In some cases, a method described herein can be used as a method of diagnosing, monitoring, and treating a condition. A method of treating a condition can require the prescription of a therapeutic agent targeted to treat the subject's condition or disease. In some cases, a therapeutic agent can be prescribed in a range of from about 1 mg to about 2000 mg; from about 5 mg to about 1000 mg, from about 10 mg to about 500 mg, from about 50 mg to about 250 mg, from about 100 mg to about 200 mg, from about 1 mg to about 50 mg, from about 50 mg to about 100 mg, from about 100 mg to about 150 mg, from about 150 mg to about 200 mg, from about 200 mg to about 250 mg, from about 250 mg to about 300 mg, from about 300 mg to about 350 mg, from about 350 mg to about 400 mg, from about 400 mg to about 450 mg, from about 450 mg to about 500 mg, from about 500 mg to about 550 mg, from about 550 mg to about 600 mg, from about 600 mg to about 650 mg, from about 650 mg to about 700 mg, from about 700 mg to about 750 mg, from about 750 mg to about 800 mg, from about 800 mg to about 850 mg, from about 850 mg to about 900 mg, from about 900 mg to about 950 mg, or from about 950 mg to about 1000 mg.

In some cases, at least 1 mg, at least 5 mg, at least 15 mg, at least 15 mg, at least 20 mg, at least 25 mg, at least 30 mg, at least 35 mg, at least 40 mg, at least 45 mg, at least 50 mg, at least 55 mg, at least 60 mg, at least 65 mg, at least 70 mg, at least 80 mg, at least 85 mg, at least 90 mg, at least 100 mg, at least 150 mg, at least 200 mg, at least 250 mg, at least 300 mg, at least 350 mg, at least 400 mg, at least 450 mg, at least 500 mg, at least 550 mg, at least 600 mg, at least 650 mg, at least 700 mg, at least 750 mg, at least 800 mg, at least 850 mg, at least 900 mg, at least 950 mg, or at least 1000 mg of the therapeutic agent is prescribed.

The arrays and methods described herein can be used by a user. A plurality of users can use a method of the invention to monitor, diagnose, treat or prevent the onset of a condition. A user can be, for example, a human who wishes to monitor one's own health. A user can be, for example, a health care provider. A health care provider can be, for example, a physician. In some cases, the user is a health care provider attending the subject. Non-limiting examples of physicians and health care providers that can be users of the invention can include, an anesthesiologist, a bariatric surgery specialist, a blood banking transfusion medicine specialist, a cardiac electrophysiologist, a cardiac surgeon, a cardiologist, a certified nursing assistant, a clinical cardiac electrophysiology specialist, a clinical neurophysiology specialist, a clinical nurse specialist, a colorectal surgeon, a critical care medicine specialist, a critical care surgery specialist, a dental hygienist, a dentist, a dermatologist, an emergency medical technician, an emergency medicine physician, a gastrointestinal surgeon, a hematologist, a hospice care and palliative medicine specialist, a homeopathic specialist, an infectious disease specialist, an internist, a maxillofacial surgeon, a medical assistant, a medical examiner, a medical geneticist, a medical oncologist, a midwife, a neonatal-perinatal specialist, a nephrologist, a neurologist, a neurosurgeon, a nuclear medicine specialist, a nurse, a nurse practioner, an obstetrician, an oncologist, an oral surgeon, an orthodontist, an orthopedic specialist, a pain management specialist, a pathologist, a pediatrician, a perfusionist, a periodontist, a plastic surgeon, a podiatrist, a proctologist, a prosthetic specialist, a psychiatrist, a pulmonologist, a radiologist, a surgeon, a thoracic specialist, a transplant specialist, a vascular specialist, a vascular surgeon, and a veterinarian. A diagnosis identified with an array and a method of the invention can be incorporated into a subject's medical record.

Kits.

Devices described herein can be packaged as a kit. In some cases, a kit includes written instructions on the use of the device. The written material can be, for example, a label. The written material can suggest conditions methods of administration. The instructions provide the subject and the supervising physician with the best guidance for achieving the optimal clinical outcome from the administration of the therapy.

**EXAMPLES**

**EXAMPLE 1:** <u>Immunosignaturing as a Method of Diagnosing Cancer.</u>

The following example demonstrates a method of diagnosing cancer with exemplary arrays of the invention. The example describes two trials, Trial #1 and Trial #2, which tested methods of the invention on biological samples collected from a plurality of subjects, at a plurality of different sites.

<u>Peptide Array.</u>

Two different libraries of 10,000 non-natural sequence peptides comprising different sequences were printed on two distinct peptide arrays. Peptide array #1 comprises 10,420 peptides and was experimentally tested on Trial #1. Peptide array #2 comprises 10,286 peptides and was experimentally tested on Trial #2.

Library 1 was printed such that two complete assays are available on one slide but only a single peptide per sequence is available per assay. Library 1 slides are compartmentalized into two physically separate chambers with a flexible gasket (Agilent, Santa Clara, CA) separating each chamber. Library 2 was printed with duplicate peptides but only one assay is available per slide.

Peptides for Trial #1 were synthesized by Sigma Genosys (St. Louis, MO) and for Trial #2 by Alta Biosciences (Birmingham, UK) with a common GSC linker on the amine terminus (Trial #1) or the carboxy-terminus (Trial #2) followed by 17 fully randomized amino acids.

Arrays were printed onto aminosilane-coated glass slides (Schott, Jena, Germany) by Applied arrays (Tempe, AZ) using non-contact piezo printing. Arrays are pre-incubated with blocking buffer (BB = 10nM Phosphate Buffered Saline, pH 7.3 and 05% BSA [Sigma Aldrich], 0.5% Tween) for 1 hour prior to addition of a 1:500 dilution of serum into sample buffer (SB = BB less 0.5% Tween) for one hour at 25°C. Slides are exposed to 5nM of AlexaFluor 647-conjugated anti-human secondary (Rockland Antibodies, Gilbertsville, PA) for 1 hour in SB at 25°C and washed 3x in SB, then 5x in 18MOhm water followed by centrifugation at 1800g for 5' to dry. Arrays are scanned in an Agilent 'C' scanner at 647nm using high laser power and 70% PMT at 10 um resolution. TIFF images are aligned with the corresponding gal file that connects peptide name with intensity.

<u>Study Design and Biological Samples.</u>

Controlled experiments were designed to test an Immunosignature system for the diagnosis of cancer. Trial #1 examines a small number of samples collected from 2-3 different cohorts per disease using a classic train/blinded test paradigm. Trial #2 is a cross-validation of a large number of disease samples processed over multiple years, composed of an unbalanced and diverse cohort from a large number of collection sites.

Study Design and Biological Sample for Trial #1: a blinded test-train trial was created using three technical replicates of 20 unblinded training samples for each of five different cancers plus 20 otherwise healthy controls. An equivalent sized test cohort was created using the same random selection process but only selecting samples that remained blinded. Collection site, collection date, age, and sex were randomized. Samples were serum or plasma from venous draws of 2 to 10 mls each, stored at -20°C for different lengths of time. Samples are described further in **TABLE 2**. Samples were collected from a plurality of different sites, which are abbreviated as follows: ASU: Arizona State University collection, Tempe, AZ; BNI: Barrow Neurological Institute, St. Joseph's Hospital and Medical Center, Phoenix, AZ; CC: Cleveland Clinic, Cleveland, OH; FHCRC: Fred Hutchison Cancer Research Center, Seattle, WA; MSKCC: Memorial Sloan-Kettering Cancer Center, New York, NY; MMRF: Multiple Myeloma Research Foundation, Norwalk, CT; MS: Mt. Sinai Hospital, New York, NY; PCRT: Pancreatic Cancer Research Team, Phoenix, AZ; UTSW: University of Texas Southwestern Medical Center, Dallas, TX; UCI: University of California Irvine, Irvine, CA; UPitt: University of Pittsburg Dept. of Immunology, Pittsburgh, PA; and UW: University of Washington Medical Center, Seattle, WA. In Table 2 a collaborator made the collections, often at various sites. The abbreviation is for where the collaborator was from.

| TABLE 2 | | | |
|---|---|---|---|
| **Disease, health state** | Training | Test | Collection Site |
| **Healthy controls** | 20 | 20 | ASU, PCRT, FHCRC, UTSW |
| ***Glioblastoma multiformae*** | 20 | 20 | BNI |
| **Pancreatic cancer** | 20 | 20 | CC, PCRT, UW |
| **Lung cancer** | 20 | 20 | FHCRC |

(continued)

| TABLE 2 | | | |
|---|---|---|---|
| **Disease, health state** | Training | Test | Collection Site |
| **Multiple myeloma** | 20 | 20 | MMRC |
| **Breast cancer** | 20 | 20 | ASU, FHCRC, UTSW |

Twenty randomly selected sera samples from patients with advanced pancreatic cancer (PC), therapy-naive *Glioblastoma multiformae* (an aggressive form of astrocytoma), (GBM), esophageal adenocarcinoma (EC), multiple myeloma (MM), and stage IV breast cancer (BC) were tested in Trial #1 as well as twenty mixed 'non-disease' controls (**TABLE 2**). **TABLE 2** describes a primary disease status noted at the time of diagnosis and used for classification. Any reported co-morbidities were ignored for the purpose of the classification.

Study Design and Biological Samples for Trial #2: 2118 samples from 10 different collaborators were Immunosignatured between September 2007 and January 2011 in Trial #2. The sera bank analyzed in this trial is inherently unbalanced in terms of number of patients per disease, age, sex, ethnicity, reported co-morbidity, and the number of controls that contributed to the "non-disease" cohort. Independent arrays whose technical replicates had a Pearson's correlation coefficient < 0.85 were not analyzed. The remaining arrays were analyzed for array batch bias using ComBat. 1516 samples were considered useful for this test.

**TABLE 3** is a description of the 1516 samples used in Trial #2. For each disease state listed in column 1, the number of available samples is listed in column 2, disease cohort. A 100-fold re-sampling method selected approximately ¼ of the samples for each disease to use for training. The average and standard deviation of the training cohorts is listed in column 3, training size. The institutional affiliation of collaborators who donated the samples are listed in column 4, collaborators.

| TABLE 3 | | | |
|---|---|---|---|
| **Disease, state** | Disease cohort | Training size | Collaborator(s) |
| **Healthy control** | 249 | $62 \pm 4$ | UCI |
| **2nd Breast Cancer** | 61 | $15 \pm 1$ | BNI |
| **Breast cancer stages II, III** | 141 | $35 \pm 3$ | ASU, FHCRC, UTSW, UCI |
| **Breast cancer stage IV** | 42 | $11 \pm 1$ | UTSW |
| **Astrocytoma** | 166 | $42 \pm 3$ | Barrow Neurological Institute |
| **Glioblastoma multiformae** | 27 | $7 \pm 1$ | ASU, BNI, CC, FHCRC, MSKCC, PCRT, UTSW, UCI, UPitt, UW |
| **Lung cancer** | 107 | $25 \pm 2$ | FHCRC |
| **Multiple myeloma** | 112 | $28 \pm 2$ | MMRC |
| **Oligodendroglioma** | 48 | $12 \pm 1$ | BNI |
| **Mixed Oligo/Astro** | 97 | $25 \pm 2$ | BNI |
| **Ovarian** | 86 | $22 \pm 2$ | MS, MSKCC |
| **Pancreatitis** | 82 | $20 \pm 1$ | CC, UW |
| **Pancreatic cancer** | 136 | $34 \pm 3$ | CC, UW |
| **Ewing's sarcoma** | 20 | $5 \pm 0$ | ASU |
| **Valley Fever** | 142 | $36 \pm 3$ | UA |

Trial #1.

Trial #1 demonstrates the simultaneous, high accuracy classification of multiple cancers with a method of the invention. Trial #1 describes a controlled experiment with equal numbers of training and test samples derived from multiple collection sites (**TABLE 2**). Twenty sera samples from patients with advanced pancreatic cancer (PC), therapy-naive Glioblastoma multiformae (GMB), esophageal adenocarcinoma (EC), multiple myeloma (MM), and stage IV breast cancer (BC) were tested as well as twenty mixed "non-disease" controls, which were collected at different sites.

The average Pearson's correlation coefficient across the two technical replicates for all 120 samples in the training set was $0.92 \pm 0.05$. Breast cancer demonstrated the lowest average replicate correlation (0.87) and esophageal cancer the highest (0.96). In order to gauge the magnitude and consistency of the difference between each disease and healthy, we performed a T-test between each of the N = 20 cancer and the N = 20 control groups one by one. The number of peptides either $p < 9.6 \times 10^{-5}$ (one FP allowed) is listed in **TABLE 4** with the absolute minimum p-value.

**TABLE 4** summarizes the results of a T-test statistical analysis of Trial #1 peptides. A T-test was used to compare the 20 training samples for each disease against 20 controls. Column 1 lists the disease cohort. Column 2 lists the number of peptides with a p-value $<9.6 \times 10^{-5}$ (corresponding to 1FP/10,480 peptides). Column 3 is the minimum p-value for that comparison. Column 4 is the number of peptides out of the top 100 most significant that overlap peptides from at least one other disease. Breast cancer had no overlap with any other disease while GMB overlapped with peptides from 3 other diseases.

| TABLE 4 | | | |
|---|---|---|---|
| **Disease** | Number of peptides with $p<9.6 \times 10^{-5}$ | Min p-value for comparison | Common peptides/100 |
| **Healthy** | NA | NA | NA |
| **Breast** | 608 | $1.54 \times 10^{-14}$ | 0 |
| **Esophageal** | 3103 | $4.8 \times 10^{-25}$ | 14 |
| **GBM** | 3596 | $9.05 \times 10^{-30}$ | 26 |
| **Myeloma** | 4478 | $3.52 \times 10^{-34}$ | 19 |
| **Pancreatic** | 1126 | $3.67 \times 10^{-11}$ | 12 |

When using only peptides from a T-test with FWER = 5%, perfect binary classification into disease versus healthy was possible using Support Vector Machines (SVM) as the classifier. This, however, does not address the issue of multiple disease classification performance. The rightmost column of **TABLE 4** shows the number of peptides that overlap at least one other disease when 100 of the most significant T-test peptides for each disease are compared. Some diseases had greater peptide overlap than others.

To improve the ability to classify multiple diseases, a filter was applied to peptides with overlapping specificity. First, ANOVA/FWER = 0.05% was applied to the training set to identify 4,620 peptides significantly different from the grand mean for each of the six classes. Second, pattern matching in GeneSpring 7.3.1 was used to remove peptides with high signal in more than one disease. Twenty-four peptides per disease were thus selected for a total of 120 peptides as the final feature set. Pancreatic and breast cancer had relatively low overall signal, esophageal and brain cancer cancer had much higher signals, but the selection method prevented the classifier from being overwhelmed by diseases with stronger signals and many significant peptides. A leave-one-out cross-validation of the training set produced two miscalls when using Support Vector Machines (SVM). The test dataset was then classified using these 120 peptides resulting in the scores shown in **TABLE 5.**

| TABLE 5 | | | | | | |
|---|---|---|---|---|---|---|
| **Disease (SVM)** | Breast Cancer (BC) | Brain Cancer (BC) | Esophageal Cancer (EC) | Multiple Myeloma | Non-Disease | Pancreatic Cancer |
| **Breast Cancer** | 20 | 0 | 0 | 0 | 0 | 2 |
| **Brain Cancer** | 0 | 19 | 1 | 0 | 0 | 0 |
| **Esophageal Cancer** | 0 | 0 | 19 | 0 | 0 | 0 |
| **Multiple Myeloma** | 0 | 1 | 0 | 20 | 0 | 0 |
| **Non-Disease** | 0 | 0 | 0 | 0 | 20 | 2 |
| **Pancreatic Cancer** | 0 | 0 | 0 | 0 | 0 | 16 |

(continued)

| TABLE 5 | | | | | | |
|---|---|---|---|---|---|---|
| Disease (SVM) | Breast Cancer (BC) | Brain Cancer (BC) | Esophageal Cancer (EC) | Multiple Myeloma | Non-Disease | Pancreatic Cancer |
| Sensitivity | 1 | 0.95 | 0.95 | 1 | 1 | 0.80 |
| Specificity | 0.98 | 0.99 | 1 | 0.99 | 0.98 | 1 |
| PPV | 0.91 | 0.95 | 1 | 0.95 | 0.91 | 1 |
| NPV | 1 | 0.99 | 0.99 | 1 | 1 | 0.96 |
| Prevalence | 0.17 | 0.17 | 0.17 | 0.17 | 0.17 | 0.17 |
| Detection Rate | 0.17 | 0.16 | 0.16 | 0.17 | 0.17 | 0.13 |
| Detection Prevalence | 0.18 | 0.17 | 0.15 | 0.18 | 0.18 | 0.13 |

Array Data Analysis.

For Trial #1, three technical replicates were averaged, biological replicates were left unaveraged. Any technical replicate that failed to achieve a Pearson's Correlation coefficient >0.85 was reprocessed. Data was median-normalized and $log_{10}$ transformed for visualization of line graphs. Initial selection of peptides for classification was performed using ANOVA and T-tests were corrected for multiple-testing using Family Wise Error Rate (FWER) set to 5%. Further filtering of the peptides was done using "Expression Profile" in GeneSpring with Euclidean Distance/Average Linkage as the similarity measure. For this filter, each disease group (Disease) was compared to all other disease groups (cumulatively referred to as Non-Disease). Peptides with consistently high signal in Disease and consistently low signal in Non-Disease were chosen, ensuring >3-fold average difference between Disease and Non-Disease signals. For multi-disease classification, equal number of peptides (features) per disease prevents a high average signal from biasing feature selection; however no further data preprocessing was done to ensure that classification performance relies on near-raw values.

Classification was done in R version 2.6.2 using Support Vector Machines (SMV) as the classifier. Misclassification scores for Trial #1 using Support Vector Machine (SVM) are shown in **TABLE 6.** True and false calls are listed in the gray area, performance statistics are listed in the white area. Average accuracy is 0.95 with a 95th percentile CI = 0.8943, 0.9981, kappa = 0.94. Correct calls are in the eigenvector, any miscalls for a given class yield a false positive in another class.

| TABLE 6 | | | | | | |
|---|---|---|---|---|---|---|
| Disease (SVM) | Breast Cancer (BC) | Brain Cancer (BC) | Esophageal Cancer (EC) | Multiple Myeloma | Non-Disease | Pancreatic Cancer |
| Breast Cancer | 20 | 0 | 0 | 0 | 0 | 2 |
| Brain Cancer | 0 | 19 | 1 | 0 | 0 | 0 |
| Esophageal Cancer | 0 | 0 | 19 | 0 | 0 | 0 |
| Multiple Myeloma | 0 | 1 | 0 | 20 | 0 | 0 |
| Non-Disease | 0 | 0 | 0 | 0 | 20 | 2 |
| Pancreatic Cancer | 0 | 0 | 0 | 0 | 0 | 16 |
| Sensitivity | 1 | 0.95 | 0.95 | 1 | 1 | 0.80 |
| Specificity | 0.98 | 0.99 | 1 | 0.99 | 0.98 | 1 |
| PPV | 0.91 | 0.95 | 1 | 0.95 | 0.91 | 1 |

(continued)

| TABLE 6 | | | | | | |
|---|---|---|---|---|---|---|
| Disease (SVM) | Breast Cancer (BC) | Brain Cancer (BC) | Esophageal Cancer (EC) | Multiple Myeloma | Non-Disease | Pancreatic Cancer |
| NPV | 1 | 0.99 | 0.99 | 1 | 1 | 0.96 |
| Prevalence | 0.17 | 0.17 | 0.17 | 0.17 | 0.17 | 0.17 |
| Detection Rate | 0.17 | 0.16 | 0.16 | 0.17 | 0.17 | 0.13 |
| Detection Prevalence | 0.18 | 0.17 | 0.15 | 0.18 | 0.18 | 0.13 |

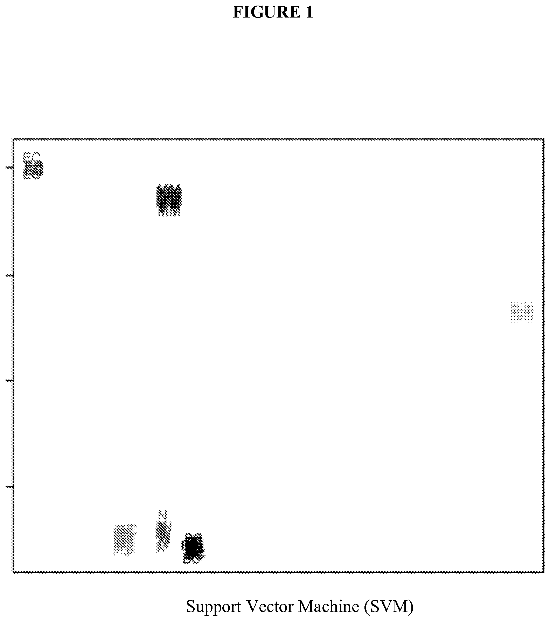

**FIGURE** 1 shows a visual representation of the relative inter- and intra-group differences as determined by the clustering and classification methods described herein. The quantitative differences illustrated in **FIGURE 1** are described in **TABLE 6**. Upper left: The first two principal components from PCA (not used to classify, only to display) are plotted on the X and Y axes. The 20 samples from the test dataset are labeled by disease: BC = breast cancer; EC esophageal cancer; N = normal donors; PC = pancreatic cancer; MM = multiple myeloma; and BrC = GMB brain cancer. Upper right: the first two linear discriminants from LDA are plotted on the X and Y axes with the disease abbreviation as noted above. Lower left: two of the support vectors that survived selection are plotted on the X and Y axes. Lower right: two Naive Bayes predictor variables are plotted on the X and Y axes.

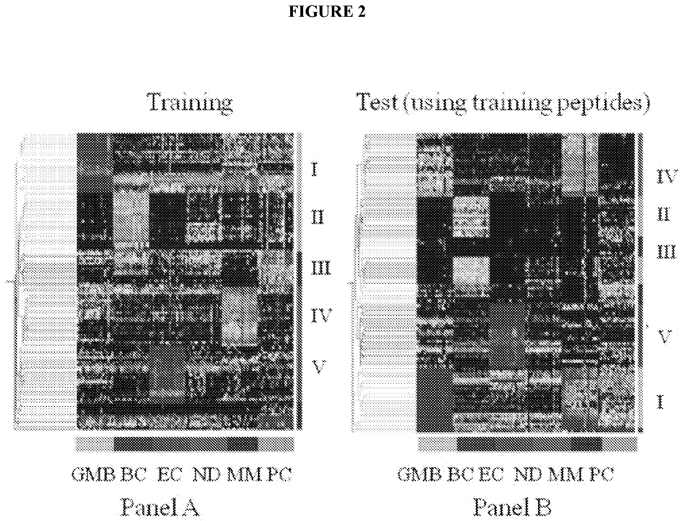

**FIGURE 2** shows a heatmap of 120 peptides (Y axis) and 120 patients (X axis) using divisive hierarchical clustering using Euclidean Distance with average linkage to estimate nodes.

This hierarchy is explicitly depicted in the colored dendrogram to the left. The result from a k-means clustering of the peptides where k = 5 classes numbered 1 to V, is shown to the right of each heatmap. The non-cancer controls were not used to select Non-Disease peptides, thus there were five groups of peptides and six groups of patients. Panel A illustrates the heatmap of the training dataset using the 120 selected features. Panel B illustrates the unblended test data clustered using the same 120 peptides. Note that the peptide class numbers follow the k-means coloring, but the peptides were re-clustered.

Trial #2.

Trial # 2 tested if Immunosignatures could classify fourteen different diseases including three subtypes of breast cancer. 1536 samples were used to create a set of 255 discriminatory peptides. In cross-validation testes the Immunosignature was 98% accurate. **TABLE 3** describes the samples used in a 1516-sample cohort analyzed in Trial #2. As described in **TABLE 4, 100** T-test peptides were chosen for each disease versus control group.

For Trial #2 a re-sampling method to provide an unbiased estimate of classification performance was used. The following procedure was repeated 100 times; results are the average of the 100 different training/testing iterations. First, 25% $\pm$ 7% of the samples for each disease were removed without replacement and used as training for feature selection. Feature selection picked exactly 255 total peptides each time. The 7% variation in cohort size simulates natural variation in disease prevalence and/or sample availability. Cross-validation was performed by classifying the remaining ~75% of the samples using the 255 features selected from training. The 95th percentile confidence interval was calculated for all statistical evaluations. Trial #2 used Support Vector Machine (SVM) as implemented in Trial #1.

**TABLE** 7 displays the results of LDA, NB and SVM classification with the 95th percentile confidence interval from re-sampling and re-analyzing 100 times. The predictions are scored as a false positive if the predicted disease appears as a prediction in any other disease category and a false negative if missed for the correct category. Given the high accuracy for Trial #1, even small cohorts with high inherent patient variability allow accurate Immunosignaturing using linear hyperplanes that optimize the distance from any training point to that plane.

| TABLE 7 | | | | | |
|---|---|---|---|---|---|
| Disease / (LDA) | Accuracy | Sensitivity | Specificity | PPV | NPV |
| 2nd BC | 97.8 $\pm$ 0.14 | 69.1 $\pm$ 2.82 | 99.21 $\pm$ 0.1 | 81.05 $\pm$ 3.46 | 98.48 $\pm$ 0.11 |
| Astro | 96.93 $\pm$ 0.17 | 90.1 $\pm$ 1.3 | 97.82 $\pm$ 0.17 | 83.79 $\pm$ 3.46 | 98.73 $\pm$ 0.18 |
| BC | 99.51 $\pm$ 0.05 | 99.71 $\pm$ 0.2 | 99.49 $\pm$ 0.08 | 95.45 $\pm$ 0.68 | 99.97 $\pm$ 0.18 |

(continued)

| TABLE 7 | | | | | |
|---|---|---|---|---|---|
| Disease / (LDA) | Accuracy | Sensitivity | Specificity | PPV | NPV |
| BCIVa | 99.62 ± 0.06 | 89.85 ± 1.49 | 100 ± 0 | 100 ± 0 | 99.6 ± 0.06 |
| GBM | 99.18 ± 0.1 | 94.33 ± 2 | 99.25 ± 0.09 | 62.1 ± 4.24 | 99.92 ± 0.03 |
| Lung | 99.02 ± 0.12 | 92.37 ± 0.58 | 99.59 ± 0.09 | 94.79 ± 1.27 | 99.35 ± 0.05 |
| MM | 98.72 ± 0.11 | 100 ± 0 | 98.62 ± 0.12 | 85.13 ± 1.13 | 100 ± 0 |
| ND | 96.62 ± 0.17 | 85.45 ± 0.77 | 99.31 ± 0.1 | 96.66 ± 0.47 | 96.6 ± 0.23 |
| Oligo | 99.65 ± 0.17 | 92.57 ± 1.95 | 99.86 ± 0.03 | 95.21 ± 1.19 | 99.78 ± 0.06 |
| OligoAstro | 98.94 ± 0.15 | 98.45 ± 0.82 | 98.95 ± 0.12 | 86.41 ± 1.78 | 99.91 ± 0.04 |
| Ovarian | 99.92 ± 0.03 | 100 ± 0 | 99.91 ± 0.03 | 98.67 ± 0.47 | 100 ± 0 |
| Pancreatitis | 99.67 ± 0.05 | 95.42 ± 1 | 99.91 ± 0.03 | 98.5 ± 0.54 | 99.74 ± 0.05 |
| PC | 97.69 ± 0.11 | 86.61 ± 1.39 | 98.79 ± 0.08 | 87.22 ± 1.19 | 98.67 ± 0.12 |
| Sarcoma | 98.81 ± 0.11 | 54.15 ± 5.48 | 99.67 ± 0.07 | 71.55 ± 5.65 | 99.12 ± 0.12 |
| VF | 99.67 ± 0.08 | 100 ± 0 | 99.64 ± 0.09 | 96.87 ± 0.74 | 100 ± 0 |
| Total | 98.77 ± 0.04 | 89.87 ± 1.32 | 99.33 ± 0.08 | 88.89 ± 1.59 | 99.33 ± 0.07 |
| Disease / (NB) | Accuracy | Sensitivity | Specificity | PPV | NPV |
| 2nd BC | 96 ± 0.16 | 56.07 ± 1.46 | 99.46 ± 0.07 | 90.37 ± 11.68 | 96.31 ± 0.15 |
| Astro | 91.92 ± 0.23 | 91.96 ± 1.25 | 91.91 ± 0.25 | 31.39 ± 10.61 | 99.66 ± 0.06 |
| BC | 98.78 ± 0.07 | 97.75 ± 0.46 | 98.91 ± 0.12 | 90.55 ± 9.81 | 99.73 ± 0.06 |
| BCIVa | 99.4 ±0.09 | 84.48 ± 2.05 | 100 ± 0 | 100 ± 0 | 99.38 ± 0.09 |
| GBM | 96.08 ± 0.1 | 43.19 ± 2.17 | 99.72 ± 0.05 | 88.81 ± 16.61 | 97.04 ± 0.19 |
| Lung | 99.08 ± 0.1 | 92.4 ± 0.89 | 99.74 ± 0.06 | 97.32 ± 6.18 | 99.25 ± 0.08 |
| MM | 96.45 ± 0.15 | 81.51 ± 2.07 | 97.76 ± 0.14 | 75.72 ± 11.16 | 98.38 ± 0.2 |
| ND | 95.84 ± 0.17 | 93.18 ± 0.62 | 96.41 ± 0.18 | 83.88 ± 7.21 | 98.54 ± 0.14 |
| Oligo | 98.54 ± 0.14 | 74.38 ± 2.24 | 99.94 ± 0.03 | 98.56 ± 5.95 | 98.85 ± 0.09 |
| OligoAstro | 97.75 ± 0.15 | 86.11 ± 0.86 | 98.72 ± 0.13 | 84.75 ± 13.01 | 99.87 ± 0.04 |
| Ovarian | 99.79 ± 0.05 | 98.48 ± 0.43 | 99.9 ±0.03 | 98.81 ± 3.75 | 99.45 ± 0.11 |
| Pancreatitis | 99.3 ± 0.11 | 92.27 ± 1.49 | 99.8 ± 0.05 | 97.4 ± 5.82 | 97.13 ± 0.17 |
| PC | 95.91 ± 0.2 | 78.67 ± 0.96 | 98.26 ± 0.09 | 85.62 ± 7.73 | 96.69 ± 0.21 |
| Sarcoma | 96.73 ± 0.2 | 25.21 ± 1.44 | 100 ± 0 | 100 ± 0 | 99.73 ± 0.07 |
| VF | 97.96 ± 0.22 | 97.48 ± 0.6 | 97.99 ± 0.22 | 84.63 ± 12.45 | 98.57 ± 0.12 |
| Total | 97.35 ± 0.15 | 79.52 ± 1.27 | 98.57 ± 0.1 | 87.19 ± 8.13 | 98.57 ± 0.12 |
| Disease / (SVM) | Accuracy | Sensitivity | Specificity | PPV | NPV |
| 2nd BC | 98.89 ± 0.03 | 91.04 ± 0.59 | 99.19 ± 0.04 | 81.16 ± 8.55 | 99.65 ± 0.03 |
| Astro | 97.12 ± 0.06 | 84.11 ± 0.31 | 98.93 ± 0.03 | 91.96 ± 2.18 | 97.82 ± 0.06 |
| BC | 99.78 ± 0.02 | 99.39 ± 0.13 | 99.82 ± 0.02 | 98.4 ± 1.34 | 99.93 ± 0.01 |
| BCIVa | 99.89 ± 0.02 | 96.26 ± 0.75 | 100 ± 0 | 100 ± 0 | 99.88 ± 0.02 |
| GBM | 99.08 ± 0.03 | 100 ± 0 | 99.07 ± 0.03 | 46.42 ± 21.1 | 100 ± 0 |
| Lung | 99.73 ± 0.02 | 96.82 ± 0.18 | 99.97 ± 0.01 | 99.65 ± 1.12 | 99.73 ± 0.02 |

(continued)

| Disease / (SVM) | Accuracy | Sensitivity | Specificity | PPV | NPV |
|---|---|---|---|---|---|
| MM | 99.58 ± 0.01 | 99.89 ± 0.08 | 99.55 ± 0.01 | 94.7 ± 1.19 | 99.99 ± 0.01 |
| ND | 98.13 ± 0.07 | 91.33 ± 0.35 | 99.7 ± 0.02 | 98.6 ± 0.81 | 98.03 ± 0.09 |
| Oligo | 99.82 ± 0.01 | 94.76 ± 0.3 | 99.96 ± 0.01 | 98.67 ± 3.38 | 99.85 ± 0.01 |
| OligoAstro | 99.29 ± 0.03 | 100 ± 0 | 99.24 ± 0.03 | 89.66 ± 4.09 | 100 ± 0 |
| Ovarian | 99.92 ± 0.01 | 98.7 ± 0.1 | 100 ± 0 | 100 ± 0 | 99.92 ± 0.01 |
| Pancreatitis | 99.73 ± 0.02 | 96.27 ± 0.27 | 99.94 ± 0.01 | 99.07 ± 1.58 | 99.77 ± 0.02 |
| PC | 98.62 ± 0.03 | 90.98 ± 0.21 | 99.45 ± 0.02 | 94.74 ± 2.16 | 99.02 ± 0.02 |
| Sarcoma | 99.19 ± 0.04 | 100 ± 0 | 99.18 ± 0.03 | 38.81 ± 31.06 | 100 ± 0 |

The low confidence intervals suggest that neither linear nor probabilistic classifiers are particularly biased for large numbers of unbalanced classes or large numbers of peptide features. As in Trial #1, a separation of each class versus normal samples was observed with SVM. The overlap from the T-test peptides produced at least one and an average of fifteen peptides that overlapped at least one other disease. There was no set of T-test peptides that did not contain at least one peptide that overlapped with at least one other disease.

**TABLE 8** The values in Table 8 contain the actual calls made for each classifier (PCA, NB and LDA and k-NN). Calls are listed by prediction (column header) vs. true disease (row) such that column 1, row 1 contains the number of calls correctly identified by the PCA classifier. Column 1, row 2 contains the number of times the classifier identified Breast Cancer (BC) as Brain Cancer (BrC). Multiple classifiers were included in this table to ensure that no classification algorithm produced severely discrepant calls.

| TABLE 8 | | | | | | |
|---|---|---|---|---|---|---|
| Disease / (PCA) | BC | BrC | EC | MM | ND | PC |
| Breast Cancer | 15 | 0 | 1 | 1 | 8 | 2 |
| Brain Cancer | 0 | 7 | 0 | 5 | 1 | 3 |
| Esophageal Cancer | 0 | 0 | 14 | 2 | 5 | 0 |
| Multiple Myeloma | 2 | 11 | 3 | 11 | 0 | 5 |
| Non-Disease | 3 | 0 | 1 | 1 | 5 | 3 |
| Pancreatic Cancer | 0 | 2 | 1 | 0 | 1 | 7 |
| Sensitivity | 0.75 | 0.35 | 0.70 | 0.55 | 0.25 | 0.35 |
| Specificity | 0.88 | 0.91 | 0.93 | 0.79 | 0.92 | 0.96 |
| PPV | 0.56 | 0.44 | 0.67 | 0.34 | 0.38 | 0.64 |
| NPV | 0.95 | 0.88 | 0.94 | 0.90 | 0.86 | 0.88 |
| Prevalence | 0.17 | 0.17 | 0.17 | 0.17 | 0.17 | 0.17 |
| Detection Rate | 0.13 | 0.06 | 0.12 | 0.09 | 0.04 | 0.06 |
| Detection Prevalence | 0.23 | 0.13 | 0.18 | 0.27 | 0.11 | 0.09 |
| Disease / (NB) | BC | BrC | EC | MM | ND | PC |
| Breast Cancer | 13 | 0 | 0 | 0 | 0 | 0 |
| Brain Cancer | 0 | 19 | 0 | 4 | 0 | 0 |
| Esophageal Cancer | 0 | 0 | 20 | 0 | 9 | 0 |
| Multiple Myeloma | 0 | 1 | 0 | 16 | 0 | 0 |
| Non-Disease | 0 | 0 | 0 | 0 | 10 | 1 |

(continued)

| Disease / (NB) | BC | BrC | EC | MM | ND | PC |
|---|---|---|---|---|---|---|
| Pancreatic Cancer | 7 | 0 | 0 | 0 | 1 | 19 |
| Sensitivity | 0.65 | 0.95 | 1 | 0.80 | 0.50 | 0.95 |
| Specificity | 1 | 0.96 | 0.91 | 0.99 | 0.99 | 0.92 |
| PPV | 1 | 0.83 | 0.69 | 0.94 | 0.91 | 0.70 |
| NPV | 0.93 | 0.99 | 1 | 0.96 | 0.91 | 0.99 |
| Prevalence | 0.17 | 0.17 | 0.17 | 0.17 | 0.17 | 0.17 |
| Detection Rate | 0.11 | 0.16 | 0.17 | 0.13 | 0.08 | 0.16 |
| Detection Prevalence | 0.11 | 0.19 | 0.24 | 0.14 | 0.09 | 0.23 |
| Disease / (LDA) | BC | BrC | EC | MM | ND | PC |
| Breast Cancer | 20 | 0 | 0 | 0 | 1 | 3 |
| Brain Cancer | 0 | 16 | 0 | 1 | 0 | 0 |
| Esophageal Cancer | 0 | 0 | 20 | 0 | 0 | 0 |
| Multiple Myeloma | 0 | 4 | 0 | 19 | 0 | 0 |
| Non-Disease | 0 | 0 | 0 | 0 | 19 | 2 |
| Pancreatic Cancer | 0 | 0 | 0 | 0 | 0 | 15 |
| Sensitivity | 1 | 0.80 | 1 | 0.95 | 0.95 | 0.75 |
| Specificity | 0.96 | 0.99 | 1 | 0.96 | 0.98 | 1 |
| PPV | 0.83 | 0.94 | 1 | 0.83 | 0.91 | 1 |
| NPV | 1 | 0.96 | 1 | 0.99 | 0.99 | 0.95 |
| Prevalence | 0.17 | 0.17 | 0.17 | 0.17 | 0.17 | 0.17 |
| Detection Rate | 0.17 | 0.13 | 0.17 | 0.16 | 0.16 | 0.13 |
| Detection Prevalence | 0.20 | 0.14 | 0.17 | 0.19 | 0.18 | 0.13 |
| Disease / (k-NN) | BC | BrC | EC | MM | ND | PC |
| Breast Cancer | 20 | 0 | 0 | 0 | 0 | 4 |
| Brain Cancer | 0 | 17 | 0 | 0 | 0 | 0 |
| Esophageal Cancer | 0 | 0 | 20 | 0 | 0 | 0 |
| Multiple Myeloma | 0 | 3 | 0 | 20 | 0 | 0 |
| Non-Disease | 0 | 0 | 0 | 0 | 20 | 3 |
| Pancreatic Cancer | 0 | 0 | 0 | 0 | 0 | 13 |
| Sensitivity | 1 | 0.85 | 1 | 1 | 1 | 0.65 |
| Specificity | 0.96 | 1 | 1 | 0.97 | 0.97 | 1 |
| PPV | 0.83 | 1 | 1 | 0.87 | 0.87 | 1 |
| NPV | 1 | 0.97 | 1 | 1 | 1 | 0.93 |
| Prevalence | 0.17 | 0.17 | 0.17 | 0.17 | 0.17 | 0.11 |
| Detection Rate | 0.17 | 0.14 | 0.17 | 0.17 | 0.17 | 0.11 |
| Detection Prevalence | 0.20 | 0.14 | 0.17 | 0.19 | 0.19 | 0.11 |

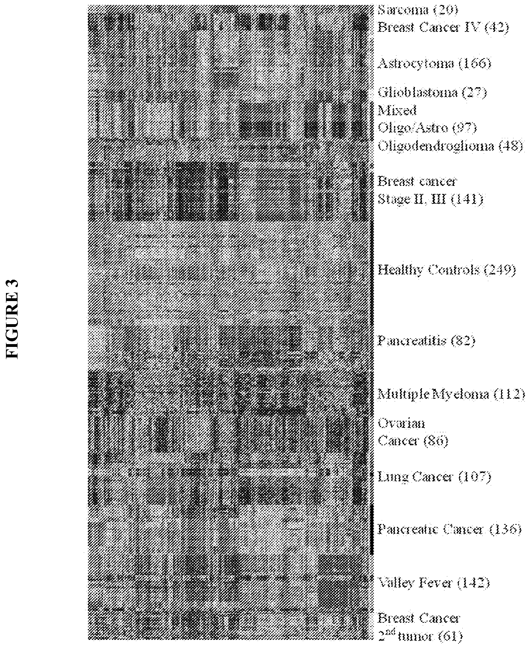

FIGURE 3 is a heatmap depicting the 255 classifier peptides across the 1516 patient samples, with cohort size listed in parenthesis. The colors distinguish high (red) from low (blue) intensity, and the patterns that remain after hierarchical clustering of both peptides (Y axis and patients (X axis) help visualize the relative difference within and across disease cohorts. Patients with known co-morbidities were not excluded, and the control samples exhibited highly diverse signals.

**FIGURE 4** shows the behavior of select peptides selected from the 255 classifier peptides. Some peptides are highly selective for a particular cancer, and contribute fully to the classification accuracy. Many peptides have imperfect consistency within a disease. Some other peptides are high for more than one disease. Separate Receiver Operator Characteristic (ROC) curves were drawn and the Area under Curve (AUC) values calculated for each disease for each classification algorithm. The AUC for SVM is show in gray. Panel A is a graphical representation of the ROC curve for Breast Cancer. Panel B is a graphical representation of the ROC curve for Brain Cancer. Panel C is a graphical representation of the ROC curve for Esophageal Cancer. Panel D is a graphical representation of the ROC curve for Multiple Myeloma. Panel E is a graphical representation of the ROC curve for Healthy controls. Panel F is a graphical representation of the ROC curve for Pancreatic Cancer.

**FIGURE 5** Is a graphical representation of Receiver Operator Characteristic (ROC) Curves for Trial #1. The Area Under Curve (AUC) for PCA is shown in gray.

**FIGURE 6** Is a graphical representation of Receiver Operator Characteristic (ROC) Curves for Trial #1. The Area Under Curve (AUC) for NB is shown in gray.

**FIGURE 7** Is a graphical representation of Receiver Operator Characteristic (ROC) Curves for Trial #1. The Area Under Curve (AUC) for LDA is shown in gray.

**FIGURE 8** Is a graphical representation of Receiver Operator Characteristic (ROC) Curves for Trial #1. The Area Under Curve (AUC) for k-NN is shown in gray.

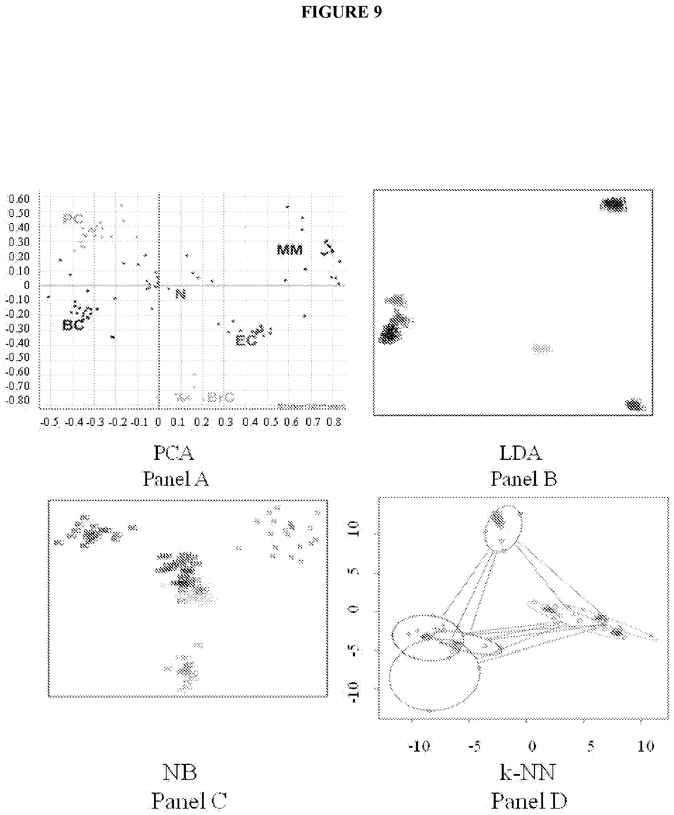

**FIGURE 9** summarizes four classifiers, PCA, LDA, NB, and k-NN, that can produce a graphical interpretation of the associated classification performance, as in **FIGURE 1** for SVM. Panel A is a graphical representation of PCA, the first two principal components are plotted. Panel B is a graphical representation of LDA, the X and Y axes depict the top two linear discriminants. Panel C is a graphical representation of NB, the predictor variable are plotted. Panel D is a graphical representation of k-NN, the groupwise distances are plotted.

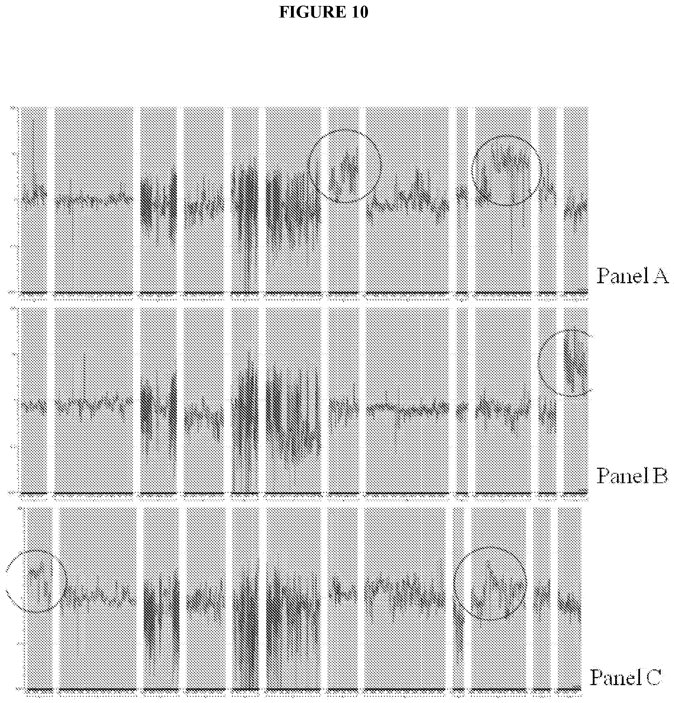

**FIGURE 10** Is a linegraph for 3 of the 255 classifier peptides from Trial #2. This intensity profile shows the individuals on the X axis, with the diseases separated by spaces, and the logic intensity for each peptide on the Y axis. Three examples of specificity are shown. Panel A illustrates a linegraph for a peptide high for disease 6 and 9 but low for all others. This enhances the specificity against the other 9 diseases, but creates possible misinterpretation between disease 6 and 9. Panel B illustrates a peptide high for disease 11 is on average 9-fold higher than any other diseases. Although diseases 3, 5, and 6 have high variation, disease 11 is highly consistent and enhances the specificity for disease 11. Panel C illustrates a peptide high for disease 1 and part of disease 9. Peptides that differ within a cohort but are disease-specific do not negatively impact the specificity for that disease, but can impact sensitivity. Given the relatively high signal within disease 1, this peptide is only moderately successful in distinguishing only disease 9, but is very successful at discriminating against diseases 2-8 and 10-11.

Immunosignaturing as a Method of Health Monitoring, a Method of Diagnosis, a Method of Treatment, and a Method Preventive Care.

A challenge faced in the diagnosis, health monitoring, treatment, and prevention of disease is the variability of sample cohorts, distinct methods of blood collection, and the submission of samples to freeze-thaw cycles. Trial #1 and Trial #2 demonstrated that the invention can overcome those challenges, and Trial #2 and Trial #2 demonstrated high condition classification specificity in a broad range of subjects with the methods of the invention. Trial #1 and Trial #2 also demonstrated that Immunosignaturing can be used in high volume sample processing, allowing more disease and control samples in the discovery phase. This feature of Immunosignaturing can overcome overfitting, a common problem with standard biomarker discovery.

Trial #1 and Trial #2 demonstrated Immunosignaturing as a method capable of high accuracy classification of different types of cancers in a standard training, blinded test assay. Variations in the number of peptides in the array, optimization of the proximity of the peptides in the array, and variation in the types of molecules in the array can make Immunosignaturing a powerful method for health monitoring, diagnosis, treatment, and prevention of a number of distinct states of health.

**EXAMPLE 2:** Computer Architectures for Use with an Immunosignature System.

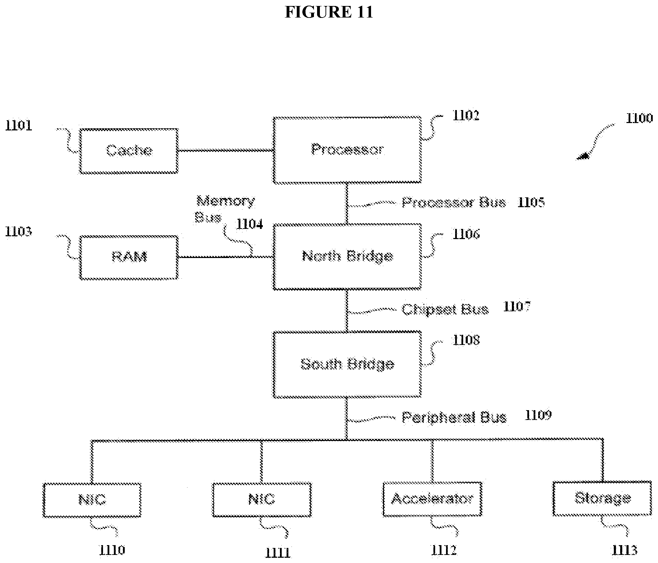

The data detected from an array of the invention can be analyzed by a plurality of computers, with various computer architectures. **FIGURE 11** is a block diagram illustrating a first example architecture of a computer system **1100** that can be used in connection with example cases of the present invention. As depicted in **FIGURE 11,** the example computer system can include a processor **1102** for processing instructions. Non-limiting examples of processors include: Intel Core i7TM processor, Intel Core i5TM processor, Intel Core i3TM processor, Intel XeonTM processor, AMD OpteronTM processor, Samsung 32-bit RISC ARM 1176JZ(F)-S v1.0TM processor, ARM Cortex-A8 Samsung S5PC100TM processor, ARM Cortex-A8 Apple A4TM processor, Marvell PXA 930TM processor, or a functionally-equivalent processor. Multiple threads of execution can be used for parallel processing. In some cases, multiple processors or processors with multiple cores can be used, whether in a single computer system, in a cluster, or distributed across systems over a network comprising a plurality of computers, cell phones, and/or personal data assistant devices.

Data acquisition, processing and storage.

As illustrated in **FIGURE 11**, a high speed cache **1101** can be connected to, or incorporated in, the processor **1102** to provide a high speed memory for instructions or data that have been recently, or are frequently, used by processor **1102**. The processor **1102** is connected to a north bridge **1106** by a processor bus **1105**. The north bridge **1106** is connected to random access memory (RAM) **1103** by a memory bus **1104** and manages access to the RAM **1103** by the processor **1102**. The north bridge **1106** is also connected to a south bridge **1108** by a chipset bus **1107**. The south bridge **1108** is, in turn, connected to a peripheral bus **1109**. The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **1109.** In some architectures, the functionality of the north bridge can be incorporated into the processor instead of using a separate north bridge chip.

In some cases, system **1100** can include an accelerator card **1112** attached to the peripheral bus **1109.** The accelerator can include field programmable gate arrays (FPGAs) or other hardware for accelerating certain processing.

Software interface(s).

Software and data are stored in external storage **1113** and can be loaded into RAM 1103 and/or cache **1101** for use by the processor. The system **1100** includes an operating system for managing system resources; non-limiting examples of operating systems include: Linux, WindowsTM, MACOSTM, BlackBerry OSTM, iOSTM, and other functionally-equivalent operating systems, as well as application software running on top of the operating system.

In this example, system **1100** also includes network interface cards (NICs) **1110** and **1111** connected to the peripheral bus for providing network interfaces to external storage, such as Network Attached Storage (NAS) and other computer systems that can be used for distributed parallel processing.

Computer systems.

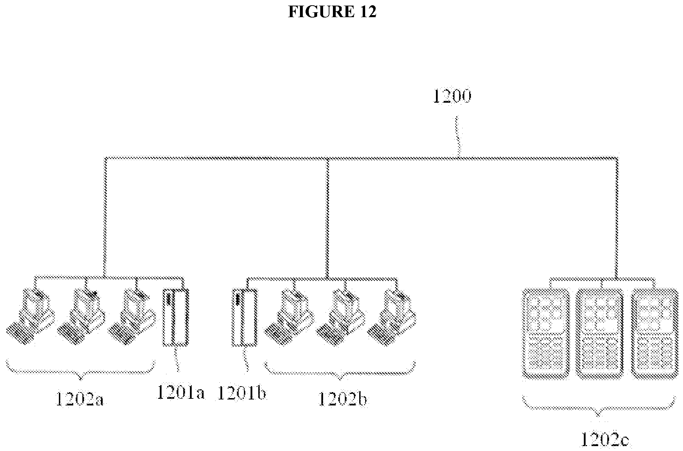

**FIGURE 12** is a diagram showing a network **1200** with a plurality of computer systems **1202a,** and **1202b,** a plurality of cell phones and personal data assistants **1202c,** and Network Attached Storage (NAS) **1201a,** and **1201b.** In some cases, systems **1202a, 1202b,** and **1202c** can manage data storage and optimize data access for data stored in Network Attached Storage (NAS) **1201a** and **1202b.** A mathematical model can be used for the data and be evaluated using distributed parallel processing across computer systems **1202a,** and **1202b,** and cell phone and personal data assistant systems **1202c.** Computer systems **1202a,** and **1202b,** and cell phone and personal data assistant systems **1202c** can also provide parallel processing for adaptive data restructuring of the data stored in Network Attached Storage (NAS) **1201a** and **1201b. FIGURE 12** illustrates an example only, and a wide variety of other computer architectures and systems can be used in conjunction with the various cases of the present invention. For example, a blade server can be used to provide parallel processing. Processor blades can be connected through a back plane to provide parallel processing. Storage can also be connected to the back plane or as Network Attached Storage (NAS) through a separate network interface.

In some cases, processors can maintain separate memory spaces and transmit data through network interfaces, back plane, or other connectors for parallel processing by other processors. In some cases, some or all of the processors can use a shared virtual address memory space.

Virtual systems.

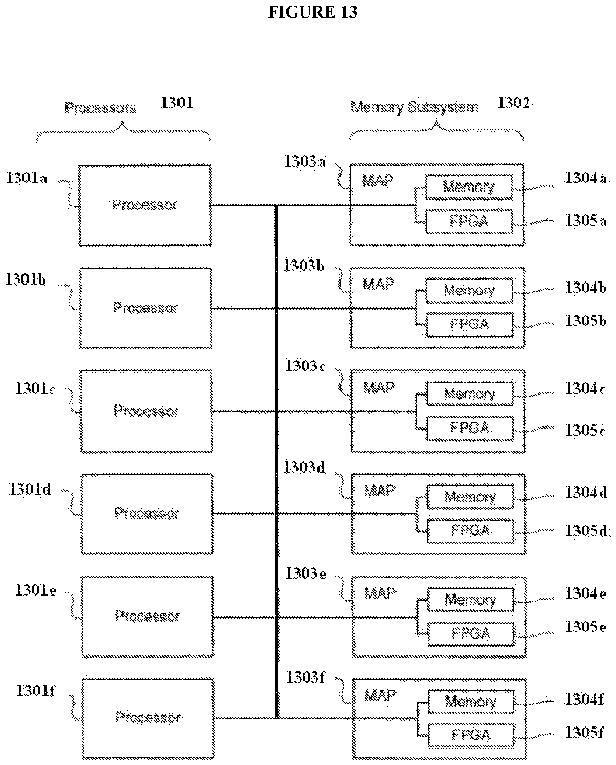

**FIGURE 13** is a block diagram of a multiprocessor computer system using a shared virtual address memory space. The system includes a plurality of processors **1301a-f** that can access a shared memory subsystem **1302.** The system incorporates a plurality of programmable hardware memory algorithm processors (MAPs) **1303a-f** in the memory subsystem **1302.** Each MAP **1303a-f** can comprise a memory **1304a-f** and one or more field programmable gate arrays (FPGAs) **1305a-f.** The MAP provides a configurable functional unit and particular algorithms or portions of algorithms can be provided to the FPGAs **1305a-f** for processing in close coordination with a respective processor. In this example, each MAP is globally accessible by all of the processors for these purposes. In one configuration, each MAP can use Direct Memory Access (DMA) to access an associated memory **1304a-f,** allowing it to execute tasks independently of, and asynchronously from, the respective microprocessor **1301a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example cases, including systems using any combination of general processors, co-processors, FPGAs and other programmable logic devices, system on chips (SOCs), application specific integrated circuits (ASICs), and other processing and logic elements. Any variety of data storage media can be used in connection with example cases, including random access memory, hard drives, flash memory, tape drives, disk arrays, Network Attached Storage (NAS) and other local or distributed data storage devices and systems.

In example cases, the computer system can be implemented using software modules executing on any of the above or other computer architectures and systems. In other cases, the functions of the system can be implemented partially or completely in firmware, programmable logic devices such as field programmable gate arrays (FPGAs) as referenced in **FIGURE 13,** system on chips (SOCs), application specific integrated circuits (ASICs), or other processing and logic elements. For example, the Set Processor and Optimizer can be implemented with hardware acceleration through the use of a hardware accelerator card, such as accelerator card **1112** illustrated in **FIGURE 11.**

**FIGURE 14** illustrates exemplary arrays of the invention with distinct peptide densities. Any of the computer architectures described above can be used in detecting, processing, and analyzing an Immunosignature.

**EXAMPLE 3:** Methods of Health Monitoring, Methods of Diagnosis, Methods of Treatment, and Methods of Preventing a Condition.

The health of a subject can be monitored at a plurality of time points in the life of the subject, including prior- and post-administration of a treatment. The following example illustrates an application of the methods and exemplary arrays of the invention in monitoring the health of six subjects. In the example described herein, methods for diagnosing, treating, monitoring, and preventing a condition of one or more of the six subjects were tested with exemplary peptide array. The experiments described in this example were conducted with a particular microarray of about 10,000 peptides. Any microarray of the invention can be used in conjunction with a method of the invention.

Health Monitoring.

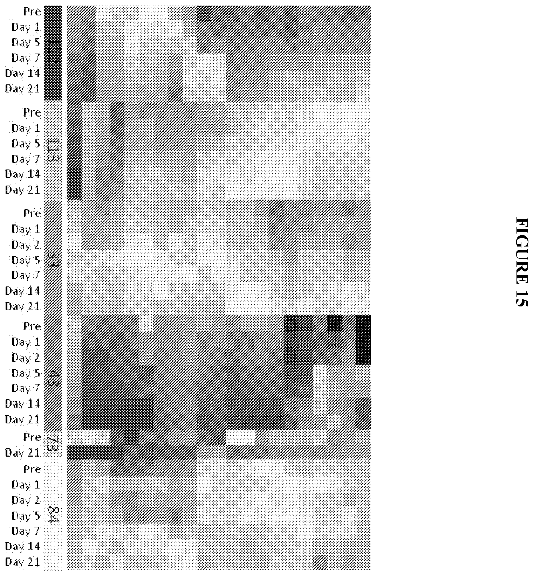

The health of multiple subjects was tracked "before" and "after" the treatment of the subjects with a dosage of the flu vaccine. **FIGURE 15** is a heatmap illustrating an Immunosignature profile of six subjects over a period of time after receiving the flu vaccine. In **FIGURE 15,** "before" refers to 1-2 weeks prior to vaccination, and after can refer to one of six distinct time-points in a period of 21 days post-vaccination. In **FIGURE 15,** an Immunosignaturing binding pattern for six subjects is illustrated as follows: 1) six de-identified subjects are represented by the numbers: 112, 113, 33, 43, 73, and 84. 2) Immunosignaturing binding patterns are clustered as: a) subject 112, "red tab", pre-vaccination, day 1, day 5, day 7, day 14, day 21; b) subject 113, "green tab", pre-vaccination, day 1, day 5, day 7, day 14, day 21; c) subject 33, "blue tab", pre-vaccination, day 1, day 5, day 7, day 14, day 21; d) subject 43, "orange tab", pre-vaccination, day 1, day 5, day 7, day 14, day 21; e) subject 73, "light pink tab", pre-vaccination, day 21; and f) subject 84, "yellow tab", pre-vaccination, day 1, day 5, day 7, day 14, day 21.

Types of Biological Samples.

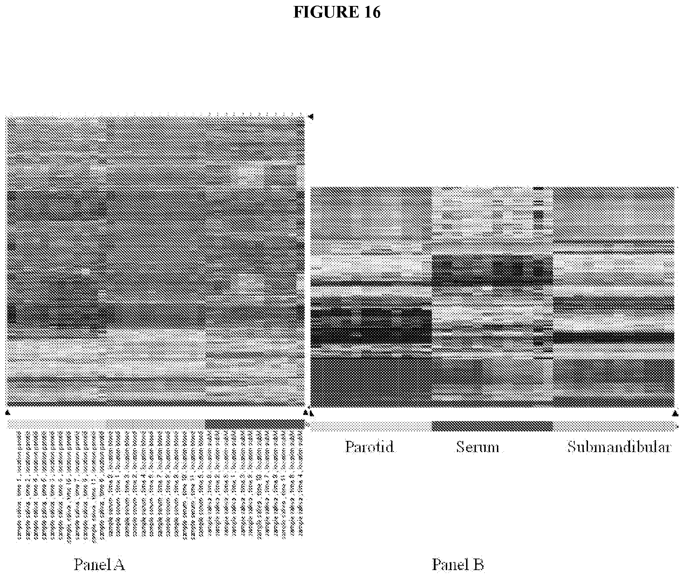

Biological samples were collected from different sources within the body of one of the six subject's described this example. The health of one of the subject's was monitored every hour for 1 day. **FIGURE 16** Panel A is a heatmap illustrating an Immunosignaturing binding pattern of the different biological samples from the same subject over the course of the day. Biological samples were collected from three places, two distinct sources of saliva and from venous blood. The two saliva collection sites are: a) parotid gland, clustered in the "yellow tab"; and b) mandibular samples, clustered above the "blue tab." The biological samples from blood are derived from a venous blood of the subject. Panel A is a heatmap illustrating the clustering of the different biological samples over 11 different time points. Panel B is a higher resolution analysis of a region of the heatmap shown in Panel A. Panel B illustrates differences in the clustering of the different biological samples in a 10,000 peptide array.

Additional sources of biological samples can be used and tested with arrays and methods of the invention.

Preventive Care.

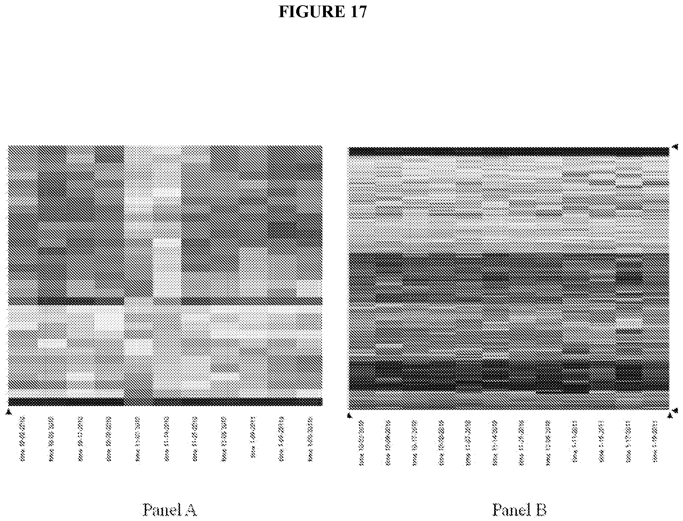

The health of one of the subject's was tracked periodically over several months. During this time the subject reported feeling ill prior to November 25, 2010. **FIGURE 17** is a heatmap illustrating an Immunosignaturing binding pattern of the subject monitored over several months. Panel A illustrates a peak in the Immunosignaturing binding pattern of the subject around November 07, 2010. The Immunosignaturing binding pattern in Panel A indicates a peak prior to the reporting of symptoms by the subject, followed by a subsequent decline. Panel B shows the consistency across all 10,000 peptides with the disease signature buried among the normal variation in antibodies. This demonstrates that a method of the invention can identify an Immunosignaturing binding pattern associated with a condition prior to the appearance of a symptom.

A binding pattern associated with a condition prior to the appearance of a symptom can be used to prevent a condition, including an onset or a progression of a condition. A physician could, for example, prescribe a medication to treat the condition identified prior to the appearance of symptoms.

Detecting and Clustering Distinct Pattern's of Binding to an Array.

More than one method can be applied for the detection of a pattern of binding a biological sample to an array. We demonstrate here the application of detecting a pattern of binding of IgM and IgG antibodies to an array of the invention.

The health of 3 of the subjects was monitored with arrays and methods of the invention. The detection and clustering of patterns of binding of IgM antibodies and IgG antibodies from the three subject's was analyzed in the peptide array. **FIGURE 18** is a heatmap illustrating an Immunosignaturing binding pattern of 3 subjects over a time course of 21 days, at day 0, day 1, day 2, day 5, day 7, and day 21. Panel A illustrates the clustering of a peptide array with about 10,000 peptides when the binding of an IgM immunoglobulin is detected. Panel B illustrates the clustering of a peptide array with 50 personal peptides when the binding of an IgM immunoglobulin is detected. Panel C illustrates the clustering of a peptide array with about 10,000 peptides when the binding of an IgG immunoglobulin is detected. Panel D illustrates the clustering of a peptide array with 50 personal peptides when the binding of an IgG immunoglobulin is detected. When a pattern of binding by IgM immunoglobulin's to a peptide array is detected and clustered using hierarchical distance, the array with groups of 10,000 peptides failed to organize individual subjects into the correct groups corresponding to the dates their blood were drawn (Panel B). When a pattern of binding by IgG immunoglobulins to the array is detected and clustered using hierarchical distance, the subject's identity and dates of blood draw cluster correctly. For Panel B, the top 50 peptides from a 2-way ANOVA analysis are shown. For Panel C, the top 50 peptides from a 2-way ANOVA analysis are shown. Each class corresponds to a subject.

Health Monitoring.

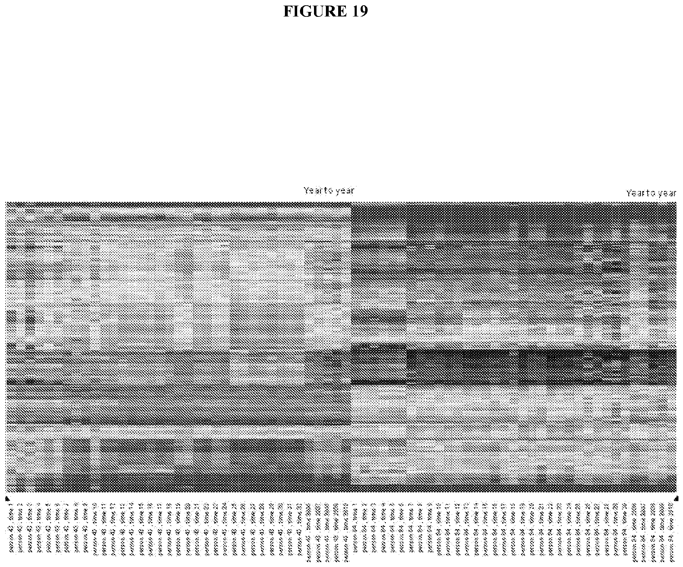

The health of one of the subject's was tracked periodically over several months. **FIGURE 19** is a heatmap illustrating a 30 day time course analyses of two subjects with Immunosignaturing binding pattern analysis. The time course includes a year-to-year clustering of an Immunosignaturing binding profile of the two subjects.

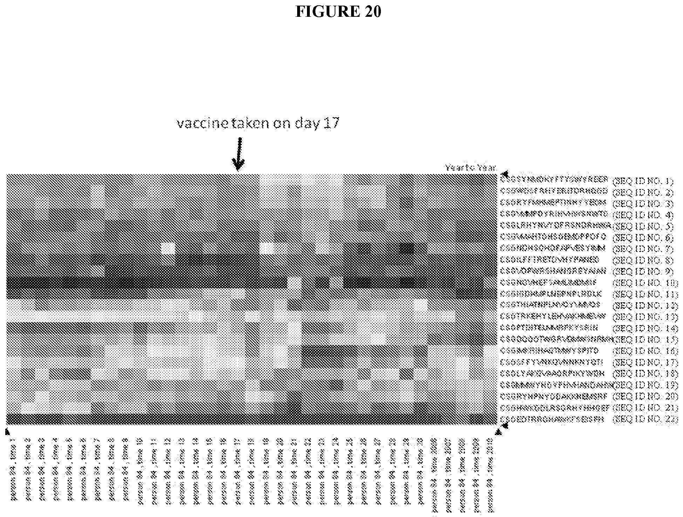

One of the subjects, subject 84, received a dosage of a flu vaccine on day 17 of the described time course. **FIGURE 20** is a heatmap illustrating the Immunosignaturing binding profile of subject 84 to twenty-two specific peptide sequences. **FIGURE 20** includes a year-to-year clustering of an Immunosignature binding profile of subject 84. The sequences of the twenty-two peptides are: SEQ ID NO. 1: CSGSYNMDKYFTYSWYREER; SEQ ID NO. 2: CSGWDSFRHYERITDRHQGD; SEQ ID NO. 3: CSGRYFMHMEPTINHYYEGM; SEQ ID NO. 4: CSCVMMPDYRIHVHWSNWTG; SEQ ID NO. 5: CSGLRHYNVYDFRSNDRHWA; SEQ ID NO. 6: CSGVMAHTGHSGRMGPPDFQ; SEQ ID NO. 7: CSGNDHSQHDFAPVESYIMM; SEQ ID NO. 8: CSGILFFTRETDVHYPANEG; SEQ ID NO. 9: CSGVDPWRSHANQREYAJAN; SEQ ID NO. 10: CSGNGVHEFSAMLIMDMIIF; SEQ ID NO. 11: CSGIGDHMPLNEPNPLRDLK; SEQ ID NO. 12: CSGTHIATNPLNVQYVMVQS; SEQ ID NO. 13: CSGTRKEHYLEHVAKHMEVW; SEQ ID NO 14: CSGPTDITELMMRPKYSRIN; SEQ ID NO. 15: CSGDQQGTWGRVDMWSNRMH; SEQ ID NO. 16: CSGIMKRIHAQTMWYSPITD; SEQ ID NO. 17: CSGSFFYVNKQVNNKNYQTI; SEQ ID NO. 18: CSGLYAKQVAAQRPIKYWDH; SEQ ID NO. 19: CSGMMWYHGYPHVHANDAHW; SEQ ID NO. 20: CSGRYHPNYGDAKKHBMSRF; SEQ ID NO. 21: CSGHWKGDLRSGRHYHHQEF; and SEQ ID NO. 22: CSGEDTRRGHAWKFSEISPH.

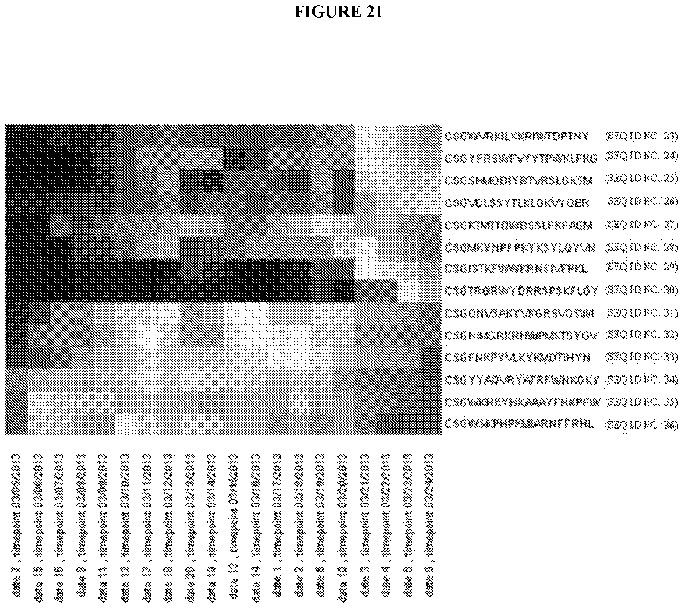

FIGURE 21 is a heatmap illustrating an Immunosignature binding profile of a blood sample of subject 84 for about 20 days following a diagnosis of bronchitis. **FIGURE 21** demonstrates a pattern of binding of a biological sample to fourteen select peptides of the invention. The sequences of the fourteen peptides are: SEQ ID NO. 23: CSGWVRKILKKRIWTDPTNY; SEQ ID NO. 24: CSGYPRSWFVYYTPWKLFKG; SEQ ID NO. 25: CSGSHMQDIYRTVRSLGKSM; SEQ ID NO. 26: CSGVQLSSYTLKLGKVYQER; SEQ ID NO. 27: CSGKTMTTQWRSSLFKFAGM; SEQ ID NO. 28: CSGMKYNPFPKYKSYLQYVN; SEQ ID NO. 29: CSGISTKFWWKRNSIVFPKL; SEQ ID NO. 30: CSGTRGRWYDRRSPSKFLGY; SEQ ID NO. 31: CSGQNVSAKYVKGRSVQSWI; SEQ ID NO. 32: CSGHIMGRKRHWPMSTSYGV; SEQ ID NO. 33: CSGFNKPYVLKYKMDTIHYN; SEQ ID NO. 34: CSGYYAQVRYATRFWNKGKY; SEQ ID NO. 35: CSGWKHKYHKAAAYFHKPFW; and SEQ ID NO. 36: CSGWSKPHPKMIARNFFRHL.

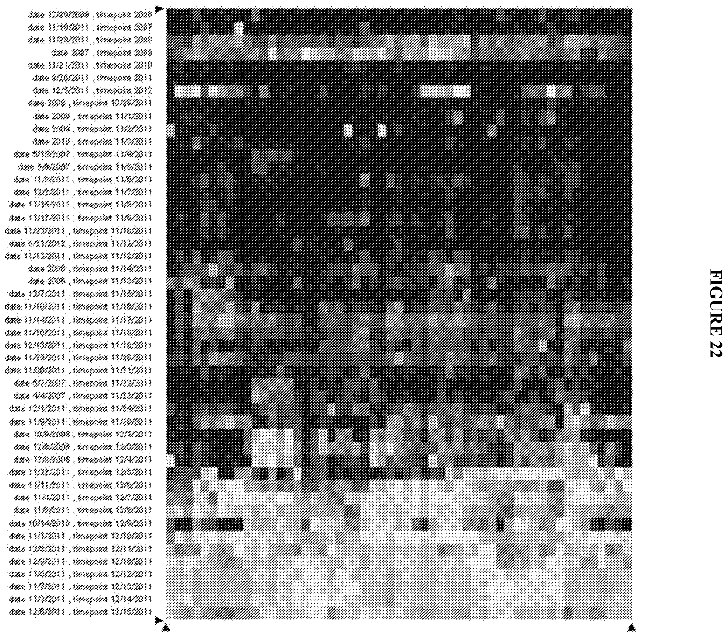

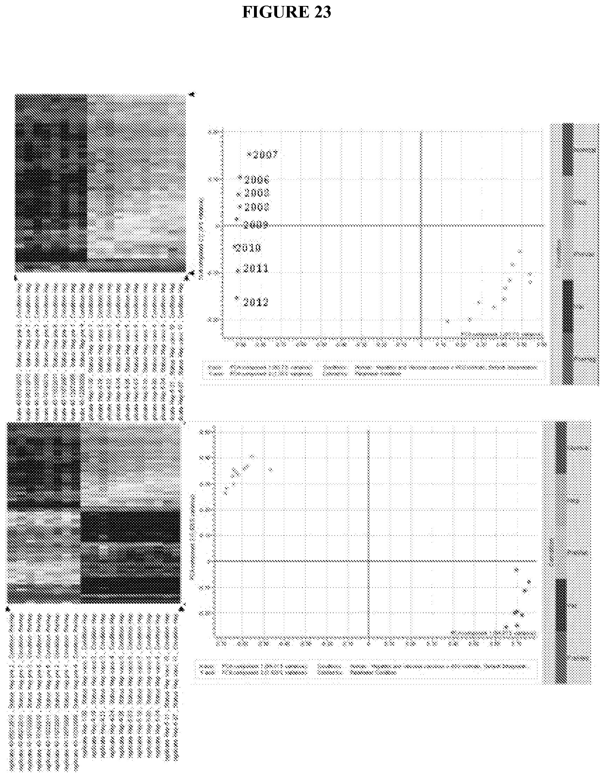

**FIGURE 22** is a heatmap illustrating a post-symptom diagnosis of the subject characterized in **FIGURE 20** with influenza on 12/11/2011. **FIGURE 23** is a heatmap illustrating an Immunosignaturing binding pattern of a subject receiving a treatment with a hepatitis vaccine, and a first booster treatment 3 months thereafter.

Simultaneous Identification of Multiple Infectious Diseases.

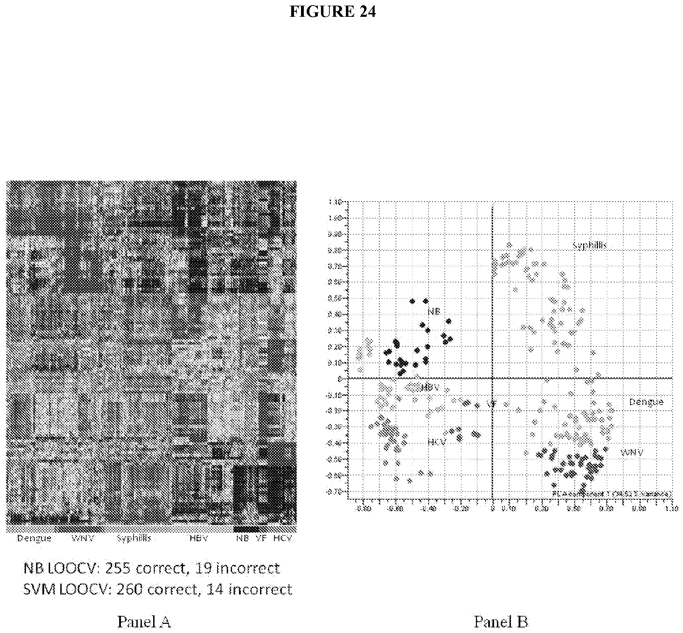

**FIGURE 24** demonstrates the identification of multiple infectious diseases with methods and arrays of the invention. **FIGURE 24** illustrates a summary of a classification of multiple infectious diseases. Panel A is a heatmap illustrating a clustered Immunosignaturing binding profile of Dengue, West Nile Virus (WNV), Syphilis, Hepatitis B Virus (HBV), Normal Blood, Valley Fever, and Hepatitis C Virus. Panel B is a graphical representation of a PCA classification.

**EXAMPLE 4:** Immunosignaturing System.

The following example describes an automated system for Immunosignaturing.

The automated system comprises several components: 1) an automated system to receive, log, and dilute a biological sample from a subject, such as a blood or a saliva sample. The automated system contacts the biological sample with a peptide microarray of the invention.

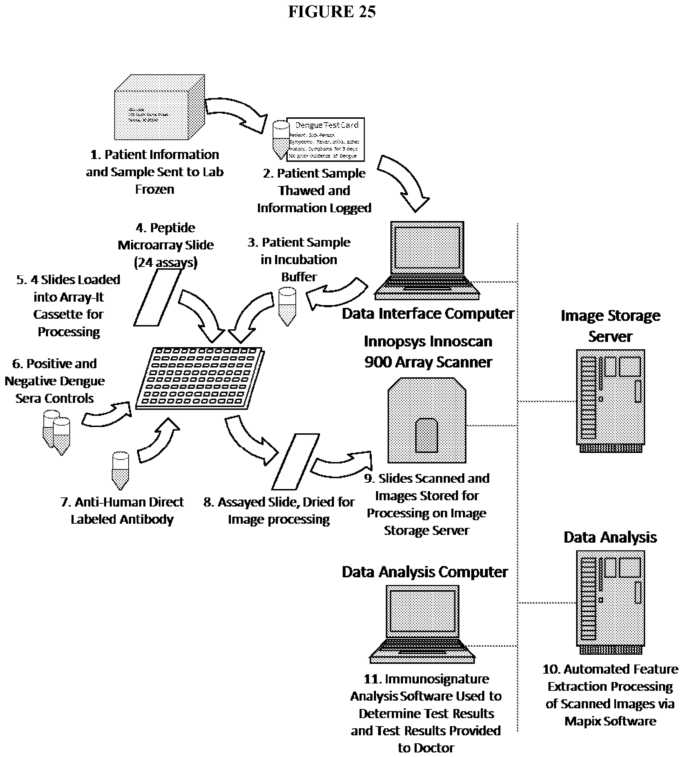

An Immunosignaturing of a subject can be obtained in an immunosignature assay of subjects consisting of the automated steps of: a) applying a diluted sample to a peptide array; b) incubating for a specific time; c) removing the sample and washing the array; d) applying a secondary antibody solution for a specific time; e) removing unbound and/or excess secondary antibody with a wash step; and f) drying and scanning the array to determine a fluorescence of an spot. **FIGURE 25** is a diagram of components of an Immunosignaturing system of the invention.

Data collection and analysis.

Arrays are aligned and signatures determined relative to standard signatures. A standard signature can be the signature of a health subject or a reference signal of an unbound peptide.

Based on the immunosignature obtained with a system of the invention, a diagnosis can be provided.

**FIGURE 26** Panel A illustrates a Phage Display library. Panel A illustrates the steps of a) a creation of phage libraries with combinatorial synthesis, b) a panning of serum against phage-displayed random antigens, and c) a selection and sequencing. Panel B illustrates a peptide microarray.

**EXAMPLE 5:** Peptide array design and manufacturing.

A set of masks for peptide array generation were designed to meet the following criteria:

- 18 different amino acids used
- 331,000 peptides in the array
- Each peptide between 10 and 16 amino acids in length
- The peptide sequences were optimized to maximize the total number of different pentamers represented (as many different 5-amino acid sequences as possible are represented within the peptide sequences on the array as a way of maximizing sequence diversity).
- No more that 6% of the peptides were allowed to have any one of the 18 amino acids at the N-terminus
- The library must be possible to generate using 90 masks (90 lithography steps).

The following steps were performed:
A large set ($\sim10^{10}$) of 16 residue peptide sequences were generated with a random number generator.

Using a computer simulation of the approach outlined previously (see "Manufacturing Arrays" above), as much of the sequence of each of the peptides in the $10^{10}$ peptide set was created as possible, using only 90 lithography steps.

From the peptide sequences resulting from the simulated synthesis, only those peptides with lengths between 10 and 16 amino acids were selected.

From the length-selected peptides, a subset of peptides optimized for inclusion of as many distinct pentamer sequences (amino acid sequences 5 long) as possible was selected.

From the pentamer-selected peptides, peptides in which the N-terminal amino acid composition contained no more than 6% of any particular amino acid was selected.

In total, the final group of peptide sequences selected to meet all the above criteria was 331,000.

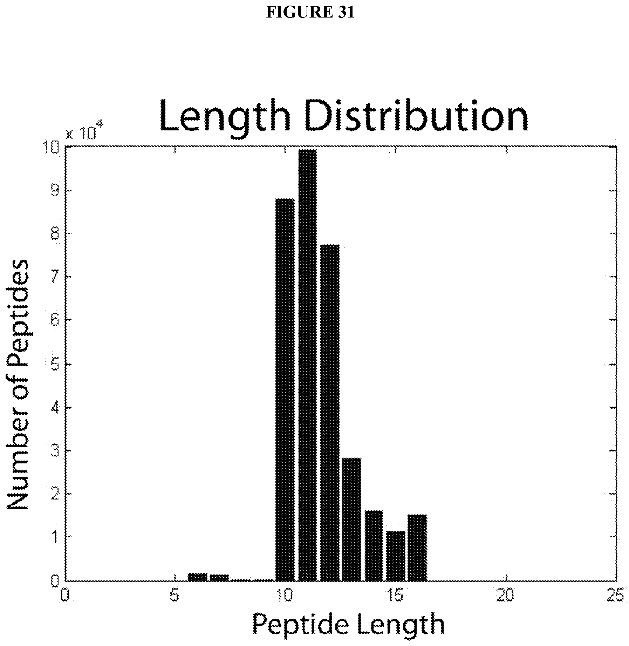

The graph in **FIGURE 31** shows a distribution of the lengths of the peptide sequences selected as described above. The Y-axis is the number of peptides with a particular length. This axis extends from 0 to 100,000. The X-axis shows the length of peptide in amino acids. As required by the criteria described above, all peptides were between 10 and 16 amino acids. The average length was approximately 11.5 amino acids.

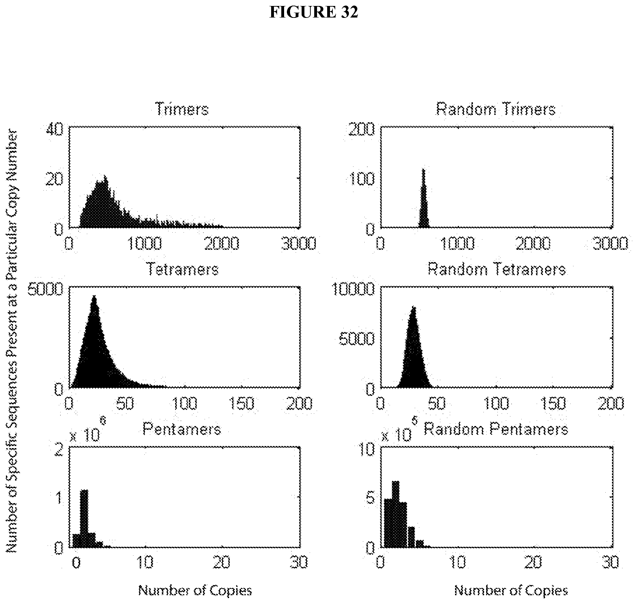

The graphs shown in **FIGURE 32** are distributions of the possible sequences that are 3, 4 or 5 amino acids long. The top two graphs show the distribution of trimer sequences (3 amino acid long peptides). There are 18x18x18 = 5832 different possible trimer sequences. The left side shows the population distribution of these trimer sequences for the peptides selected as described above. The right side shows the distribution for a library of peptide sequences that were created using a random number generator. For each graph, the X-axis depicts the number of times a particular trimer sequence is present in the library. The Y-axis depicts the number of trimer sequences that are present the number of times denoted on the X-axis. Thus one can see that for peptide sequences, generated using a random number generator, almost all of the 5832 trimer sequences are represented between 400 and 600 times in the library. For the selected peptides on the left, in contrast, the distribution of trimer sequences is broader, with some trimer sequences present only about 100 times and others present more than 1000 times. All possible trimer sequences are represented multiple times in the library.

The middle graphs are for tetramer sequences (4 amino acid sequences). The axes are similar to that described for the trimers. One can see that most tetramer sequences are present in a library of this size generated using a random number generator (right panel) about 30 times. In the peptide library selected as described above (left panel), the peak of the distribution is about 20 and the width is larger than seen from sequences generated with a random number generator. There are a total of 18^4 = 104976 possible tetramer sequences. 99.99% of all possible tetramer sequences are represented in the peptide library selected as described above.

The bottom graphs are for pentamer sequences (5 amino acid sequences). The axes are as described for trimer sequences. There are 1,889,568 possible pentamer sequences. Note that for the peptide sequences selected as described above (left panel), 14% of the all possible pentamer sequences are not represented (this is the first bar in the graph). Most of the pentamer sequences are represented once (the second bar) and a few more than once. In all, 86% of all possible pentamer sequences are represented in the selected library. In contrast, only about 75% of all pentamers are represented in a library of peptide sequences generated using a random number generator (right panel). One can see that the first bar in the graph on the right for the randomly generated sequences, representing sequences not represented in the library, is larger than the first bar in the graph on the left for the peptides selected as described above.

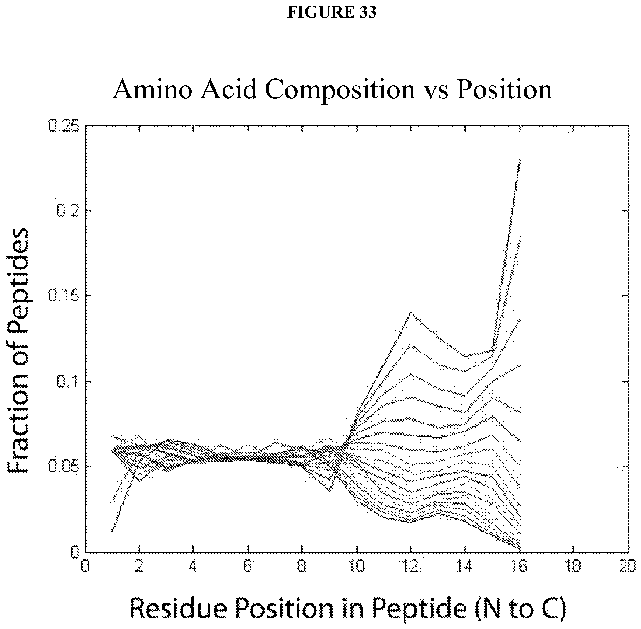

**FIGURE 33** shows the amino acid composition as a function of position in the peptide for the peptide library selected as described above. The N-terminus is at position 1 and the C-terminus is at a position between 10 and 16 (as described above, there is a distribution of peptide lengths in this library). This is shown on the X-axis. The Y-axis shows the fraction of the peptides that contain a particular amino acid at the position shown on the X-axis. Each line (each color) represents one of the 18 amino acids. One can see that at the N-terminus, two of the amino acids are somewhat underrepresented (less than 5% of the peptides contain these amino acids at their N-terminus). Through most of the sequence, the composition of amino acids is substantially constant and just above 5% on average. This is what one would expect for an even distribution of 18 amino acids (1/18 = ~0.056). The divergence near the C-terminus occurs in part because the number of peptides decreases with increasing length in this region.

## Claims

1. A method of health monitoring, performed on a dried blood sample obtained from a patient said method comprising:

   a) reconstituting the dried blood sample with a solution; contacting the reconstituted sample to a peptide array, wherein the array comprises a plurality of in-situ synthesized peptides immobilized to pre-determined different locations on a solid support, wherein the in-situ synthesis of the peptides comprises the steps of:
   b) contacting the reconstituted sample to a peptide array, wherein the array comprises a plurality of in-situ synthesized peptides immobilized to pre-determined different locations on a solid support, wherein the in-situ synthesis of the peptides comprises the steps of:

      a. adding a first monomer to a pre-determined fraction of locations on the solid support;
      b. adding a second monomer to a pre-determined fraction of locations on the solid support, wherein the pre-determined fraction of locations for the second monomer includes locations containing the first monomer and locations with no monomer;
      c. adding a third monomer to a pre-determined fraction of locations on the solid support, wherein the pre-determined fraction of locations for the second monomer includes locations containing the first and second monomer, locations containing the second monomer and locations containing no monomer; and

d. repeating steps a-c with a defined set of monomers until the monomers form at least one peptide of a desired average length and the sum of the fractions total is at least 100%; wherein the plurality of different peptides are capable of off-target binding of at least one antibody in the reconstituted dried blood sample;

c) measuring the off-target binding of the at least one antibody to the plurality of different peptides in the peptide array to form an immunosignature; and

d) identifying a state of health based on the immunosignature, wherein the immunosignature measures changes in binding of the antibody from the reconstituted dried blood sample to the peptide array as compared to a control.

2. The method of claim 1, wherein the reconstituted dried blood sample is diluted prior to contacting the peptide array.

3. The method of any one of claims 1 and 2, wherein a plurality of reconstituted antibodies in the reconstituted dried blood sample bind to the peptide array.

4. The method of any one of claims 1-3, wherein the state of health of the subject is healthy, afflicted with breast cancer, afflicted with ovarian cancer, afflicted with pancreatic cancer, afflicted with brain cancer, afflicted with esophageal cancer, afflicted with multiple myeloma afflicted with an autoimmune disorder or afflicted with rheumatoid arthritis.

5. The method of any one of claims 1-4, wherein the plurality of different peptides on the peptide array are between 8 and 35 residues in length.

6. The method of any one of claims 1-5, wherein the peptide array comprises greater than 3,000 different peptides.

7. The method of any one of claims 1-6, wherein different peptides on the peptide array have an average spacing ranging from 1 nm to 1.5 nm within a spot on the peptide array.

8. The method of any one of claims 1-7, wherein the plurality of different peptides bind to the at least two antibodies with an association constant in the range of $10^3$ to $10^6$ $M^{-1}$.

9. The method of any one of claims 1-8, wherein the plurality of different peptides bind to the at least two antibodies with an association constant in the range of $10^4$ to $10^6$ $M^{-1}$.

10. The method of any one of claims 1-9, wherein the array is a pseudo-random array or a random array.

11. The method of any one of claims 1-10, wherein the array further comprises monomers chosen from the group consisting of nucleic acids and peptide nucleic acids.

12. The method of any one of claims 1-11, wherein the monomers are attached to the array by a linker molecule.

13. The method of any one of claims 1-12, wherein at least 5% of the peptides have a length of at least 10 residues.

14. The method of any one of claims 1-13, wherein the method is performed on an immunosignaturing system, the immunosignaturing system comprising an automated device consisting of the following components:

1) an automated system to receive, and reconstitute a dried blood sample from a subject, thereby performing step a) of the method of claim 1;

2) a compartment for an automated immunosignaturing assay, the immunosignaturing assay comprising:

a) an application of a diluted sample to a peptide array,
b) an incubation for a specific time,
c) a wash and removal of unbound sample,
d) application of a secondary antibody solution for a specific time,
e) a wash and removal of excess secondary antibody, and
f) a drying and scanning of the array to determine a fluorescence of each spot, thereby performing step c) of the method of claim 1; and

3) using a computer system to measure changes in binding of the antibody from the reconstituted dried blood sample to the peptide array as compared to a control, thereby performing step d) of the method of claim 1.

**Patentansprüche**

1. Verfahren zur Gesundheitsüberwachung, das an einer getrockneten Blutprobe durchgeführt wird, die von einem Patienten erhalten wurde, wobei das Verfahren umfasst:

   a) Rekonstituieren der getrockneten Blutprobe mit einer Lösung; Inkontaktbringen der rekonstituierten Probe mit einem Peptid-Array, wobei das Array eine Vielzahl von *in situ* synthetisierten Peptiden umfasst, die an verschiedenen zuvor festgelegten Positionen auf einem festen Träger immobilisiert wurden, wobei die In-Situ-Synthese der Peptide die Schritte umfasst:
   b) Inkontaktbringen der rekonstituierten Probe mit einem Peptid-Array, wobei das Array eine Vielzahl von *in situ* synthetisierten Peptiden umfasst, die an verschiedenen zuvor festgelegten Positionen auf einem festen Träger immobilisiert wurden, wobei die *In-Situ*-Synthese der Peptide die Schritte umfasst:

   a. Hinzugeben eines ersten Monomers an einen zuvor festgelegten Teil der Positionen auf dem festen Träger;
   b. Hinzugeben eines zweiten Monomers an einen zuvor festgelegten Teil der Positionen auf dem festen Träger, wobei der zuvor festgelegte Teil der Positionen für das zweite Monomer Positionen, die das erste Monomer enthalten, und Positionen beinhaltet, die kein Monomer enthalten;
   c. Hinzugeben eines dritten Monomers an einen zuvor festgelegten Teil der Positionen auf dem festen Träger, wobei der zuvor festgelegte Teil der Positionen für das zweite Monomer Positionen, die das erste und zweite Monomer enthalten, Positionen, die das zweite Monomer enthalten, und Positionen beinhaltet, die kein Monomer enthalten; und
   d. Wiederholen der Schritte a-c mit einem definierten Satz von Monomeren bis die Monomere mindestens ein Peptid mit einer gewünschten durchschnittlichen Länge bilden und die Summe der Teile insgesamt mindestens 100 % beträgt; wobei die Vielzahl an verschiedenen Peptiden dazu fähig ist, sich off-target an mindestens einen Antikörper in der rekonstituierten getrockneten Blutprobe zu binden;

   c) Messen der Off-Target-Bindung des mindestens einen Antikörpers an die Vielzahl von verschiedenen Peptiden in dem Peptid-Array, um eine Immunsignatur zu bilden; und
   d) Identifizieren eines Gesundheitszustands basierend auf der Immunsignatur, wobei die Immunsignatur die Veränderungen in der Bindung des Antikörpers von der rekonstituierten getrockneten Blutprobe an das Peptid-Array im Vergleich zu einer Kontrolle misst.

2. Verfahren nach Anspruch 1, wobei die rekonstituierte getrocknete Blutprobe vor dem Inkontaktbringen mit dem Peptid-Array verdünnt wird.

3. Verfahren nach einem der Ansprüche 1 und 2, wobei sich eine Vielzahl von rekonstituierten Antikörpern in der rekonstituierten getrockneten Blutprobe an das Peptid-Array bindet.

4. Verfahren nach einem der Ansprüche 1-3, wobei der Gesundheitszustand des Subjekts gesund, an Brustkrebs erkrankt, an Eierstockkrebs erkrankt, an Bauchspeicheldrüsenkrebs erkrankt, an Hirntumor erkrankt, an Speiseröhrenkrebs erkrankt, an multiplem Myelom erkrankt, an einer Autoimmunerkrankung erkrankt oder an rheumatoider Arthritis erkrankt lautet.

5. Verfahren nach einem der Ansprüche 1-4, wobei die Vielzahl von verschiedenen Peptiden auf dem Peptid-Array zwischen einer Länge von 8 und 35 Resten liegt.

6. Verfahren nach einem der Ansprüche 1-5, wobei das Peptid-Array mehr als 3.000 verschiedene Peptide umfasst.

7. Verfahren nach einem der Ansprüche 1-6, wobei verschiedene Peptide auf dem Peptid-Array einen durchschnittlichen Abstand im Bereich von 1 nm bis 1,5 nm an einem Fleck auf dem Peptid-Array aufweisen.

8. Verfahren nach einem der Ansprüche 1-7, wobei die Vielzahl von verschiedenen Peptiden an die mindestens zwei Antikörper mit einer Assoziationskonstante in dem Bereich von $10^3$ bis $10^6$ M$^{-1}$ bindet.

9. Verfahren nach einem der Ansprüche 1-8, wobei die Vielzahl von verschiedenen Peptiden an die mindestens zwei Antikörper mit einer Assoziationskonstante in dem Bereich von $10^4$ bis $10^6$ M$^{-1}$ bindet.

10. Verfahren nach einem der Ansprüche 1-9, wobei das Array ein Pseudo-Random-Array oder ein Random-Array ist.

11. Verfahren nach einem der Ansprüche 1-10, wobei das Array ferner Monomere umfasst, ausgewählt aus der Gruppe bestehend aus Nukleinsäuren und Peptid-Nukleinsäuren.

12. Verfahren nach einem der Ansprüche 1-11, wobei die Monomere über ein Linker-Molekül mit dem Array verbunden sind.

13. Verfahren nach einem der Ansprüche 1-12, wobei mindestens 5 % der Peptide eine Länge von mindestens 10 Resten aufweisen.

14. Verfahren nach einem der Ansprüche 1-13, wobei das Verfahren an einem Immunsignatur-System durchgeführt wird, wobei das Immunsignatur-System eine automatisierte Vorrichtung umfasst, die aus den folgenden Komponenten besteht:

1) ein automatisiertes System, um eine getrocknete Blutprobe von einem Subjekt zu erhalten und diese zu rekonstituieren, wobei Schritt a) des Verfahrens nach Anspruch 1 durchgeführt wird;
2) ein Kompartiment für einen automatisierten Immunsignatur-Assay, wobei der Immunsignatur-Assay umfasst:

a) ein Auftragen einer verdünnten Probe auf ein Peptid-Array,
b) eine Inkubation für eine bestimmte Zeit,
c) ein Waschen und Entfernen einer ungebundenen Probe,
d) Aufbringen einer Lösung mit sekundärem Antikörper für eine bestimmte Zeit,
e) ein Waschen und Entfernen eines überschüssigen sekundären Antikörpers, und
f) ein Trocknen und Scannen des Arrays, um eine Fluoreszenz jedes Flecks zu bestimmen und dabei Durchführen des Schritts c) des Verfahrens nach Anspruch 1; und

3) Verwenden eines Computersystems, um die Veränderungen in der Bindung des Antikörpers von der rekonstituierten getrockneten Blutprobe an das Peptid-Array im Vergleich zu einer Kontrolle zu messen und dabei Durchführen des Schritts d) des Verfahrens nach Anspruch 1.

## Revendications

1. Procédé de surveillance de la santé, effectué sur un échantillon de sang séché obtenu d'un patient, ledit procédé comprenant les étapes consistant à :

a) reconstituer l'échantillon de sang séché avec une solution ; mettre en contact l'échantillon reconstitué avec une matrice peptidique, où la matrice comprend une pluralité de peptides synthétisés *in situ* immobilisés sur différents emplacements prédéterminés sur un support solide, où la synthèse *in situ* de peptides comprend les étapes consistant à :
b) mettre en contact l'échantillon reconstitué avec une matrice peptidique, où la matrice comprend une pluralité de peptides synthétisés *in situ* immobilisés sur différents emplacements prédéterminés sur un support solide, où la synthèse in situ des peptides comprend les étapes consistant à :

a. ajouter un premier monomère à une fraction prédéterminée d'emplacements sur le support solide ;
b. ajouter un deuxième monomère à une fraction prédéterminée d'emplacements sur le support solide, où la fraction prédéterminée d'emplacements pour le deuxième monomère inclut des emplacements contenant le premier monomère et des emplacements sans monomère ;
c. ajouter un troisième monomère à une fraction prédéterminée d'emplacements sur le support solide, où la fraction prédéterminée d'emplacements pour le deuxième monomère inclut des emplacements contenant le premier et le deuxième monomères, des emplacements contenant le deuxième monomère et des emplacements sans monomère ; et
d. répéter les étapes a à c avec un ensemble défini de monomères jusqu'à ce que les monomères forment au moins un peptide d'une longueur moyenne souhaitée et la somme du total des fractions soit d'au moins 100 % ; où la pluralité des différents peptides est capable de liaison non ciblée d'au moins un anticorps dans l'échantillon de sang séché reconstitué ;

c) mesurer la liaison non ciblée d'au moins un anticorps à la pluralité de différents peptides dans la matrice peptidique pour former une immuno-signature ; et

d) identifier un état de santé d'après l'immuno-signature, où l'immuno-signature mesure les changements de liaison de l'anticorps provenant de l'échantillon de sang séché reconstitué à la matrice peptidique par rapport à un contrôle.

2. Procédé selon la revendication 1, dans lequel l'échantillon de sang séché reconstitué est dilué avant d'être mis en contact avec la matrice peptidique.

3. Procédé selon l'une quelconque des revendications 1 et 2, dans lequel une pluralité d'anticorps reconstitués dans l'échantillon de sang séché reconstitué se lie à la matrice peptidique.

4. Procédé selon l'une quelconque des revendications 1 à 3, dans lequel le sujet est en bonne santé, atteint d'un cancer du sein, atteint d'un cancer de l'ovaire, atteint d'un cancer du pancréas, atteint d'un cancer du cerveau, atteint d'un cancer de l'oesophage, atteint d'un myélome multiple, atteint d'une maladie auto-immune ou atteint d'une polyarthrite rhumatoïde.

5. Procédé selon l'une quelconque des revendications 1 à 4, dans lequel la pluralité de différents peptides sur la matrice peptidique a une longueur comprise entre 8 et 35 résidus.

6. Procédé selon l'une quelconque des revendications 1 à 5, dans lequel la matrice peptidique comprend plus de 3 000 peptides différents.

7. Procédé selon l'une quelconque des revendications 1 à 6, dans lequel les différents peptides sur la matrice peptidique ont un espacement moyen compris entre 1 nm et 1,5 nm à l'intérieur d'un point sur la matrice peptidique.

8. Procédé selon l'une quelconque des revendications 1 à 7, dans lequel la pluralité de différents peptides se lie à au moins deux anticorps avec une constante d'association comprise entre $10^3$ et $10^6$ $M^{-1}$.

9. Procédé selon l'une quelconque des revendications 1 à 8, dans lequel la pluralité de différents peptides se lie à au moins les deux anticorps avec une constante d'association comprise entre $10^4$ et $10^6$ $M^{-1}$.

10. Procédé selon l'une quelconque des revendications 1 à 9, dans lequel la matrice est une matrice pseudo-aléatoire ou une matrice aléatoire.

11. Procédé selon l'une quelconque des revendications 1 à 10, dans lequel la matrice comprend en outre des monomères choisis dans le groupe constitué par les acides nucléiques et les acides nucléiques peptidiques.

12. Procédé selon l'une quelconque des revendications 1 à 11, dans lequel les monomères sont liés à la matrice par une molécule de liaison.

13. Procédé selon l'une quelconque des revendications 1 à 12, dans lequel au moins 5 % des peptides ont une longueur d'au moins 10 résidus.

14. Procédé selon l'une quelconque des revendications 1 à 13, lequel procédé est effectué sur un système d'immuno-signature, le système d'immuno-signature comprenant un dispositif automatisé constitué des composants suivants :

1) un système automatisé pour recevoir et reconstituer un échantillon de sang séché d'un sujet, en effectuant ainsi l'étape a) du procédé selon la revendication 1 ;

2) un compartiment pour une matrice d'immuno-signature automatisée, la matrice d'immuno-signature comprenant :

a) une application d'un échantillon dilué sur une matrice peptidique,
b) une incubation pendant une durée spécifique,
c) un lavage et une élimination de l'échantillon non lié,
d) l'application d'une solution d'anticorps secondaire pendant une durée spécifique,
e) un lavage et une élimination de l'anticorps secondaire en excès, et
f) un séchage et un balayage de la matrice pour déterminer une fluorescence de chaque point, en effectuant ainsi l'étape c) du procédé selon la revendication 1 ; et

3) l'utilisation d'un système informatique pour mesurer les changements de liaison de l'anticorps provenant de l'échantillon de sang séché reconstitué à la matrice peptidique par rapport à un contrôle, en effectuant ainsi l'étape d) du procédé selon la revendication 1.

**FIGURE 1**

Support Vector Machine (SVM)

# FIGURE 2

Training

Test (using training peptides)

GMB BC EC ND MM PC
Panel A

GMB BC EC ND MM PC
Panel B

**FIGURE 3**

FIGURE 4

Breast Cancer — Panel A

Brain Cancer — Panel B

Esophageal Cancer — Panel C

Multiple Myeloma — Panel D

Healthy Control — Panel E

Pancreatic Cancer — Panel F

**FIGURE 5**

Panel A — Breast Cancer; Panel B — Brain Cancer; Panel C — Esophageal Cancer; Panel D — Multiple Myeloma; Panel E — Healthy Control; Panel F — Pancreatic Cancer

**FIGURE 6**

**FIGURE 7**

Panel A — Breast Cancer

Panel B — Brain Cancer

Panel C — Esophageal Cancer

Panel D — Multiple Myeloma

Panel E — Healthy Control

Panel F — Pancreatic Cancer

# FIGURE 8

**FIGURE 9**

PCA
Panel A

LDA
Panel B

NB
Panel C

k-NN
Panel D

**FIGURE 10**

Panel A

Panel B

Panel C

**FIGURE 11**

**FIGURE 12**

**FIGURE 13**

**FIGURE 14**

EP 2 890 984 B1

FIGURE 15

FIGURE 16

Parotid   Serum   Submandibular

Panel A          Panel B

**FIGURE 17**

Panel A                                    Panel B

**FIGURE 18**

Panel A

Panel B

Panel C

Panel D

FIGURE 19

## FIGURE 20

vaccine taken on day 17

FIGURE 21

FIGURE 22

**FIGURE 23**

**FIGURE 24**

NB LOOCV: 255 correct, 19 incorrect
SVM LOOCV: 260 correct, 14 incorrect

Panel A

Panel B

**FIGURE 25**

1. Patient Information and Sample Sent to Lab Frozen

2. Patient Sample Thawed and Information Logged

4. Peptide Microarray Slide (24 assays)

3. Patient Sample in Incubation Buffer

5. 4 Slides Loaded into Array-It Cassette for Processing

6. Positive and Negative Dengue Sera Controls

7. Anti-Human Direct Labeled Antibody

8. Assayed Slide, Dried for Image processing

9. Slides Scanned and Images Stored for Processing on Image Storage Server

Data Interface Computer

Innopsys Innoscan 900 Array Scanner

Image Storage Server

Data Analysis Computer

11. Immunosignature Analysis Software Used to Determine Test Results and Test Results Provided to Doctor

Data Analysis

10. Automated Feature Extraction Processing of Scanned Images via Mapix Software

## FIGURE 26

Panel A

Panel B

**FIGURE 27**

FIGURE 28

**FIGURE 29**

FIGURE 30

**Frequency**

**FIGURE 31**

FIGURE 32

FIGURE 33

## Amino Acid Composition vs Position

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012190574 A

**Non-patent literature cited in the description**

- **B.A. CHASE et al.** *clinical and Vaccine Immunology,* 2012, vol. 19, 352-358
- **HANASH, S.** Disease proteomics. *Nature,* 2003, vol. 422, 226-232
- **REDDY, M.M. et al.** Identification of Candidate IgG Biomarkers for Alzheimer's Disease via Combinatorial Library Screening. *Cell,* vol. 144, 132-142
- **PANICKER, R.C. et al.** Recent advances in peptide-based microarray technologies. *Comb Chem High Throughput Screen,* 2004, vol. 7, 547-556
- **FU, J. et al.** Exploring peptide space for enzyme modulators. *J Am Chem Soc,* 2010, vol. 132, 6419-6424
- **FU, J. et al.** Peptide-modified surfaces for enzyme immobilization. *PLoS One,* 2011, vol. 6, e18692
- **DIEHNELT, C.W. et al.** Discovery of high-affinity protein binding ligands-backwards. *PLoS One,* vol. 5, e10728
- **GREVING, M.P. et al.** High-throughput screening in two dimensions: binding intensity and off-rate on a peptide microarray. *Anal Biochem,* vol. 402, 93-95
- **GREVING, M.P. et al.** Thermodynamic additivity of sequence variations: an algorithm for creating high affinity peptides without large libraries or structural information. *PLoS One,* vol. 5, e15432
- **GUPTA, N. et al.** Engineering a synthetic ligand for tumor necrosis factor-alpha. *Bioconjug Chem,* vol. 22, 1473-1478
- **BOLTZ, K.W. et al.** Peptide microarrays for carbohydrate recognition. *Analyst,* 2009, vol. 134, 650-652
- **FOONG, Y.M. et al.** Current advances in peptide and small molecule microarray technologies. *Curr Opin Chem Biol,* 2012, vol. 16, 234-242
- **MORALES BETANZOS, C. et al.** Bacterial glycoprofiling by using random sequence peptide microarrays. *Chembiochem,* 2009, vol. 10, 877-888
- **FALSEY, J.R. et al.** Peptide and small molecule microarray for high throughput cell adhesion and functional assays. *Bioconjug Chem,* 2001, vol. 12, 346-353
- **CERECEDO, I. et al.** Mapping of the IgE and IgG4 sequential epitopes of milk allergens with a peptide microarray-based immunoassay. *J Allergy Clin Immunol,* 2008, vol. 122, 589-594
- **CRETICH, M. et al.** Epitope mapping of human chromogranin A by peptide microarrays. *Methods Mol Biol,* 2009, vol. 570, 221-232
- **LIN, J. et al.** Development of a novel peptide microarray for large-scale epitope mapping of food allergens. *J Allergy Clin Immunol,* 2009, vol. 124, 315-322
- **LORENZ, P. et al.** Probing the epitope signatures of IgG antibodies in human serum from patients with autoimmune disease. *Methods Mol Biol,* 2009, vol. 524, 247-258
- **PEREZ-GORDO, M. et al.** Epitope mapping of Atlantic salmon major allergen by peptide microarray immunoassay. *Int. Arch Allergy Immunol,* 2012, vol. 157, 31-40
- **SHREFFLER, W.G. et al.** IgE and IgG4 epitope mapping by microarray immunoassay reveals the diversity of immune response to the peanut allergen, Ara h 2. *J Allergy Clin Immunol,* 2005, vol. 116, 893-899
- **MOHAN, S. et al.** Association energetics of cross-reactive and specific antibodies. *Biochemistry,* 2009, vol. 48, 1390-1398
- **THORPE, I.F. ; BROOKS, C.L., 3RD.** Molecular evolution of affinity and flexibility in the immune system. *Proceedings of the National Academy of Sciences of the United States of America,* 2007, vol. 104, 8821-8826
- **ZHOU, Z.H. et al.** Properties and function of polyreactive antibodies and polyreactive antigen-binding B cells. *J Autoimmun.,* 2007, vol. 29, 219-228
- **FOLGORI, A. et al.** A general strategy to identify mimotopes of pathological antigens using only random peptide libraries and human sera. *Embo J,* 1994, vol. 13, 2236-2243
- **CHRISTIAN, R.B. et al.** Simplified methods for construction, assessment and rapid screening of peptide libraries in bacteriophage. *Journal of Molecular Biology,* 1992, vol. 227, 711-718

- **LIU, R. et al.** Combinatorial peptide library methods for immunobiology research. *Experimental Hematology,* 2003, vol. 31, 11-30
- **WANG, Y. et al.** Detection of Mammary Tumor Virus ENV Gene-like Sequences in Human Breast Cancer. *Cancer Research,* 1995, vol. 55, 5173-5179
- **DERDA, R. et al.** Diversity of phage-displayed libraries of peptides during panning and amplification. *Molecules,* 2011, vol. 16, 1776-1803
- **SZARDENINGS, M.** Phage display of random peptide libraries: applications, limits, and potential. *J Recept Signal Transduct Res,* 2003, vol. 23, 307-34953, 54
- **JANEWAY, C. ; TRAVERS, J.** Immunobiology: The Immune System in Health and Disease. Current Biology Limited, 1997
- **NOBREGA, A. et al.** Functional diversity and clonal frequencies of reactivity in the available antibody repertoire. *European Journal of Immunology,* 1998, vol. 28, 1204-1215
- **BROWN, J.R. et al.** Statistical methods for analyzing immunosignatures. *BMC Bioinformatics,* 2011, vol. 12, 349
- **HUGHES, A.K. et al.** Immunosignaturing can detect products from molecular markers in brain cancer. *PLoS One,* 2012, vol. 7, e40201
- **KROENING, K. et al.** Autoreactive antibodies raised by self derived de novo peptides can identify unrelated antigens on protein microarrays. Are autoantibodies really autoantibodies?. *Exp Mol Pathol,* 2012, vol. 92, 304-311
- **KUKREJA, M. et al.** Comparative study of classification algorithms for immunosignaturing data. *BMC Bioinformatics,* 2012, vol. 13, 139
- **KUKREJA, M. et al.** Immunosignaturing Microarrays Distinguish Antibody Profiles of Related Pancreatic Diseases. *Journal of Proteomics and Bioinformatics,* 2012
- **LEGUTKI, J.B. et al.** A general method for characterization of humoral immunity induced by a vaccine or infection. *Vaccine,* 2010, vol. 28, 4529-4537
- **STAFFORD, P. ; JOHNSTON, S.** Microarray technology displays the complexities of the humoral immune response. *Expert Rev Mol Diagn,* 2011, vol. 11, 5-8
- **HALPERIN, R.F. et al.** Exploring Antibody Recognition of Sequence Space through Random-Sequence Peptide Microarrays. *Molecular & Cellular Proteomics,* 2011, 10
- **HALPERIN, R.F. et al.** Exploring Antibody Recognition of Sequence Space through Random-Sequence Peptide Microarrays. *Molecular & Cellular Proteomics,* 2011, vol. 10
- **STAFFORD, P. ; JOHNSTON, S.** Microarray technology displays the complexities of the humoral immune response. *Expert Rev Mol Diagn,* 2011, vol. 11
- **STAFFORD, P. et al.** Physical characterization of the "immunosignaturing effect. *Mol Cell Proteomics,* 2012, vol. 11 (M111), 011593
- **CHASE, B.A. et al.** Evaluation of biological sample preparation for immunosignature-based diagnostics. *Clin Vaccine Immunol,* 2012, vol. 19, 352-358
- **RESTREPO, L. et al.** Application of immunosignatures to the assessment of Alzheimer's disease. *Annals of Neurology,* 2011, vol. 70, 286-295
- **UHLEN, M. ; HOBER, S.** Generation and validation of affinity reagents on a proteome-wide level. *J Mol Recognit,* 2009, vol. 22, 57-64
- **DRAGHICI, S.** Statistics and Data Analysis for Microarrays Using R and Bioconductor. Chapman & Hall/CRC, 2012
- **YANG, Y. et al.** Segmentation and intensity estimation for microarray images with saturated pixels. *BMC Bioinformatics,* 2011, vol. 12, 462
- **HUGHES, A.K. et al.** Immunosignaturing can detect products from molecular markers in brain cancer. *PLoS One,* vol. 7, e40201